# EUROPEAN PATENT APPLICATION

(11) **EP 2 251 028 A1**
(43) Date of publication of application: **17.11.2010**
(21) Application number: 09006411.4
(22) Date of filing: 12.05.2009
(51) Int. Cl.: A61K 38/39, A61K 9/16, A61K 9/50, A61K 48/00, A61P 27/00, A61P 27/02, A61P 27/06

(54) **Treatment of eye diseases using encapsulated cells encoding and secreting an anti-angiogenic factor and/or a neuroprotective factor**

(71) Applicant: Biocompatibles Uk Ltd., Chapman House Farnham Business Park Weydon Lane Farnham Surrey GU9 8QL (GB)
(72) Inventor: Jonas, Jost B., 69469 Weinheim (DE); Wallrapp, Christine, 63762 Großostheim (DE); Thoenes, Eric, 63654 Büdingen (DE); Geigle, Peter, 63755 Alzenau (DE); Panda-Jonas, Songhomitra, 69469 Weinheim (DE)
(74) Representative: Graf von Stosch, Andreas

(57) **Abstract**

The present application refers to cells, e.g. mesenchymal stem cells or mesenchymal stromal cells, or any further suitable cell, encoding and secreting an anti-angiogenic factor, a neuroprotective factor and/or any other protein or protein-like substance suitable for (intraocular) treatment of eye diseases. Such eye diseases include glaucoma and other optic nerve disorders, retinal diseases, particularly retinitis pigmentosa (RP), age-related macular degeneration (AMD) and diabetic retinopathy. The cells used herein are encapsulated in a (spherical) microcapsule to prevent a response of the immune system of the patient to be treated. The present application also refers to the use of these (spherical) microcapsule(s) or such factors for (intraocular) treatment of eye diseases as defined herein (for the preparation of a (pharmaceutical) composition) for the treatment of such eye diseases.

SEQUENCE LISTING

<110>  Biocompatibles UK Limited

<120>  Treatment of eye diseases using encapsulated cells encoding and
       secreting an anti-angiogenic and/or a neuroprotective factor

<130>  BI07P006EP

<160>  48

<170>  PatentIn version 3.5

<210>  1
<211>  31
<212>  PRT
<213>  Artificial

<220>
<223>  Description of sequence:  synthetic peptide corresponding to
       GLP-1(7-37)

<400>  1

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  2
<211>  15
<212>  PRT
<213>  Artificial

<220>
<223>  Description of sequence: full-length IP-2 sequence having all 15
       amino acids of the naturally occurring IP-2 sequence, human;

<400>  2

Arg Arg Asp Phe Pro Glu Glu Val Ala Ile Val Glu Glu Leu Gly
1               5                   10                  15

<210>  3
<211>  15
<212>  PRT
<213>  Artificial

<220>
<223>  Description of sequence: full-length IP-2 sequence having all 15
       amino acids of the naturally occurring IP-2 sequence, murine;

<400>  3

Arg Arg Asp Phe Pro Glu Glu Val Ala Ile Ala Glu Glu Leu Gly
1               5                   10                  15

<210>  4
<211>  35
<212>  PRT
<213>  Artificial

<220>
<223>  Description of sequence: murine isoform of GLP-2

<400>  4

His Ala Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp Arg Lys
        35

<210>  5
<211>  35
<212>  PRT
<213>  Artificial

<220>
<223>  Description of sequence: human isoform of GLP-2

<400>  5

His Ala Asp Gly Ser Phe Ser Asp Glu Met Ser Thr Ile Leu Asp Asn
1               5                   10                  15

Leu Ala Thr Arg Asp Phe Ile Asn Trp Leu Ile Gln Thr Lys Ile Thr
            20                  25                  30

Asp Lys Lys
        35

<210>  6
<211>  79
<212>  PRT
<213>  Artificial

<220>
<223>  Description of sequence: SEQ ID No: 6 (ID6syn, CM1) corresponds
       to GLP-1(7-37)-IP2-RR-GLP1(7-37), 79 aa, 8,7 kD

<400>  6

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Glu Gly Thr Phe Thr Ser Asp Val Ser Ser Tyr Leu Glu Gly
    50                  55                  60

Gln Ala Ala Lys Glu Phe Ile Ala Trp Leu Val Lys Gly Arg Gly
65                  70                  75

<210>  7

<211>  83
<212>  PRT
<213>  Artificial

<220>
<223>  Description of sequence: SEQ ID No: 7 (ID7rec, CM2) corresponds
       to GLP-1(7-37)-IP2-RR-GLP2, 83 aa, 9,4 kD

<400>  7

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly Arg Arg
        35                  40                  45

His Ala Asp Gly Ser Phe Ser Asp Glu Met Ser Thr Ile Leu Asp Asn
    50                  55                  60

Leu Ala Thr Arg Asp Phe Ile Asn Trp Leu Ile Gln Thr Lys Ile Thr
65                  70                  75                  80

Asp Lys Lys

<210>  8
<211>  46
<212>  PRT
<213>  Artificial

<220>
<223>  Description of sequence: SEQ ID No:8 (ID8 syn, CM3) corresponds
       to GLP-1(7-37)-IP2, 46 aa, 5,1 kD;

<400>  8

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Ala Glu Glu Leu Gly
        35                  40                  45

<210>  9
<211>  97
<212>  PRT
<213>  Artificial

<220>
<223>  Description of sequence: artificial

<400>  9

Met Ala Pro Ala Ala Trp Leu Arg Ser Ala Ala Ala Arg Ala Leu Leu
1               5                   10                  15

Pro Pro Met Leu Leu Leu Leu Leu Gln Pro Pro Pro Leu Leu Ala Arg
            20                      25                      30

Ala Leu Pro Pro Asp Val His His Leu His Ala Glu Arg Arg Gly Pro
        35                      40                      45

Gln Pro Trp His Ala Ala Leu Pro Ser Ser Pro Ala Pro Ala Pro Ala
    50                      55                      60

Thr Gln Glu Ala Pro Arg Pro Ala Ser Ser Leu Arg Pro Pro Arg Cys
65                      70                      75                      80

Gly Val Pro Asp Pro Ser Asp Gly Leu Ser Ala Arg Asn Arg Gln Lys
                85                      90                      95

Arg

<210>  10
<211>  79
<212>  PRT
<213>  Artificial

<220>
<223>  Description of sequence: SEQ ID No: 10
       (N-GLP-1(7-37)-IP2(human)-RR-GLP-1(7-37)-C, also designated human
       CM1)

<400>  10

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Glu Gly Thr Phe Thr Ser Asp Val Ser Ser Tyr Leu Glu Gly
    50                  55                  60

Gln Ala Ala Lys Glu Phe Ile Ala Trp Leu Val Lys Gly Arg Gly
65                  70                  75

<210>  11
<211>  83
<212>  PRT
<213>  Artificial

<220>
<223>  Description of sequence: SEQ ID No: 11
       (N-GLP-1(7-37)-IP2(human)-RR-GLP-2-C), also designated human CM2
       herein)

<400>  11

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Asp Gly Ser Phe Ser Asp Glu Met Asn Thr Ile Leu Asp Asn
    50                  55                  60

Leu Ala Ala Arg Asp Phe Ile Asn Trp Leu Ile Gln Thr Lys Ile Thr
65                  70                  75                  80

Asp Arg Lys

<210>  12
<211>  46
<212>  PRT
<213>  Artificial

<220>
<223>  Description of sequence: SEQ ID No: 12, GLP-1(7-37) linked
       without any linker sequence via its C-terminus to human IP2

<400>  12

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly
        35                  40                  45

<210>  13
<211>  815
<212>  DNA
<213>  Artificial

<220>
<223>  Description of sequence: (SEQ ID No:13) represents the translated
       peptide sequence of the construct according to Fig. 1k (SEQ ID
       No: 14)

<220>
<221>  CDS
<222>  (11)..(118)

<220>
<221>  CDS
<222>  (387)..(809)

<400>  13
gatatccacc atg gcc ccc gcc gcc tgg ctg agg agc gcc gcc gcc agg          49

Met Ala Pro Ala Ala Trp Leu Arg Ser Ala Ala Ala Arg
1               5                   10

gcc ctg ctg cca ccc atg ctg ctg ctg ctg ctg cag ccc cca cct ctg      97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct      148
Leu Ala Arg Ala Leu Pro Pro
30                  35

cgctgagggg gcgccgggca cgcgggctgg gcccagcggc gtatccggac gccaagaaac      208

cagagagcca gccagatgcc aaagggccct gccatgtgcc ggtgcccttt ccctctccat      268

ttgccctgcc acacagtggg ctggggttgc acgtgtgttt gctgacaggc cacatctcta      328

actgtgggcc atgtggacct taggcctgac cagaccctca tgtcttcctc cttcccag      386

gac gtg cac cac ctg cac gcc gag agg cgc ggc cct cag ccc tgg cac      434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc      482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat      530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag      578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc      626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc      674
Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gag      722
Pro Glu Glu Val Ala Ile Val Glu Glu Leu Gly Arg Arg His Ala Glu
        135                 140                 145

ggc acc ttc acc tcc gac gtg agc agc tac ctg gag ggc cag gcc gcc      770
Gly Thr Phe Thr Ser Asp Val Ser Ser Tyr Leu Glu Gly Gln Ala Ala
    150                 155                 160

aag gag ttc atc gcc tgg ctg gtg aag ggc agg ggc tga gcgcgc      815
Lys Glu Phe Ile Ala Trp Leu Val Lys Gly Arg Gly
165                 170                 175

<210>  14
<211>  815
<212>  DNA
<213>  Artificial

<220>
<223>  Description of sequence: DNA sequence of the construct according
       to Fig. 1k

<400>  14
gatatccacc atggcccccg ccgcctggct gaggagcgcc gccgccaggg ccctgctgcc      60

acccatgctg ctgctgctgc tgcagccccc acctctgctg gcccgggccc tgcccccggt      120

gagtgcccgc cactcgccgt ccgctcctcg ctgagggggc gccgggcacg cgggctgggc 180

ccagcggcgt atccggacgc caagaaacca gagagccagc cagatgccaa agggccctgc 240

catgtgccgg tgccctttcc ctctccattt gccctgccac acagtgggct ggggttgcac 300

gtgtgtttgc tgacaggcca catctctaac tgtgggccat gtggacctta ggcctgacca 360

gaccctcatg tcttcctcct tcccaggacg tgcaccacct gcacgccgag aggcgcggcc 420

ctcagccctg gcacgccgcc ctgccaagca gccctgcccc tgccccagcc acccaggagg 480

cccccaggcc tgccagcagc ctgaggccac ccaggtgcgg cgtgcctgat ccctccgatg 540

gcctgagcgc tcggaatcgg cagaagaggc acgccgaggg caccttcacc tccgacgtga 600

gcagctacct ggagggccag gccgccaagg agttcatcgc ctggctggtg aagggcaggg 660

gccgcaggga cttccctgag gaggtggcca tcgtggagga gctgggccgg cgacacgccg 720

agggcacctt cacctccgac gtgagcagct acctggaggg ccaggccgcc aaggagttca 780

tcgcctggct ggtgaagggc aggggctgag cgcgc 815

<210> 15
<211> 834
<212> DNA
<213> Artificial

<220>
<223> Description of sequence: DNA sequence and sequence of the
      translated peptide according to the construct Fig. 1h

<220>
<221> CDS
<222> (11)..(118)

<220>
<221> CDS
<222> (387)..(821)

<400> 15
gatatccacc atg gcc ccc gcc gcc tgg ctg agg agc gcc gcc gcc agg 49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg 97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct 148
Leu Ala Arg Ala Leu Pro Pro
30                  35

cgctgagggg gcgccgggca cgcgggctgg gcccagcggc gtatccggac gccaagaaac 208

cagagagcca gccagatgcc aaagggccct gccatgtgcc ggtgcccttt ccctctccat 268

ttgccctgcc acacagtggg ctggggttgc acgtgtgttt gctgacaggc cacatctcta 328

actgtgggcc atgtggacct taggcctgac cagaccctca tgtcttcctc cttcccag 386

gac gtg cac cac ctg cac gcc gag agg cgc ggc cct cag ccc tgg cac 434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc     482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat     530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag     578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc     626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc     674
Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gac     722
Pro Glu Glu Val Ala Ile Val Glu Glu Leu Gly Arg Arg His Ala Asp
        135                 140                 145

ggc agc ttc agc gac gag atg aac acc atc ctg gac aac ctg gcc gcg     770
Gly Ser Phe Ser Asp Glu Met Asn Thr Ile Leu Asp Asn Leu Ala Ala
    150                 155                 160

cgc gac ttc atc aac tgg ctg atc cag acc aag atc acc gat cgg aag     818
Arg Asp Phe Ile Asn Trp Leu Ile Gln Thr Lys Ile Thr Asp Arg Lys
165                 170                 175                 180

tga gcgcgctgat atc                                                  834

<210>  16
<211>  834
<212>  DNA
<213>  Artificial

<220>
<223>  Description of sequence: DNA sequence of the construct according
       to Fig. 1h

<400>  16
gatatccacc atggcccccg ccgcctggct gaggagcgcc gccgccaggg ccctgctgcc      60

acccatgctg ctgctgctgc tgcagccccc acctctgctg gcccgggccc tgcccccggt     120

gagtgcccgc cactcgccgt ccgctcctcg ctgagggggc gccgggcacg cgggctgggc     180

ccagcggcgt atccggacgc caagaaacca gagagccagc cagatgccaa agggccctgc     240

catgtgccgg tgccctttcc ctctccattt gccctgccac acagtgggct ggggttgcac     300

gtgtgtttgc tgacaggcca catctctaac tgtgggccat gtggacctta ggcctgacca     360

gaccctcatg tcttcctcct tcccaggacg tgcaccacct gcacgccgag aggcgcggcc     420

ctcagccctg gcacgccgcc ctgccaagca gccctgcccc tgccccagcc acccaggagg     480

cccccaggcc tgccagcagc ctgaggccac ccaggtgcgg cgtgcctgat ccctccgatg     540

gcctgagcgc tcggaatcgg cagaagaggc acgccgaggg caccttcacc tccgacgtga     600

gcagctacct ggagggccag gccgccaagg agttcatcgc ctggctggtg aagggcaggg     660

gccgcaggga cttccctgag gaggtggcca tcgtggagga gctgggccgg cgacacgccg     720

acggcagctt cagcgacgag atgaacacca tcctggacaa cctggccgcg cgcgacttca 780

tcaactggct gatccagacc aagatcaccg atcggaagtg agcgcgctga tatc 834

<210> 17
<211> 780
<212> DNA
<213> Artificial

<220>
<223> Description of sequence: DNA sequence sequence and translated
      peptide sequence of the construct according to Fig. 1l,

<220>
<221> CDS
<222> (11)..(118)

<220>
<221> CDS
<222> (387)..(776)

<400> 17
gatatccacc atg gcc ccc gcc gcc tgg ctg agg agc gcc gcc gcc agg 49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg 97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct 148
Leu Ala Arg Ala Leu Pro Pro
30                  35

cgctgagggg gcgccgggca cgcgggctgg gcccagcggc gtatccggac gccaagaaac 208

cagagagcca gccagatgcc aaagggccct gccatgtgcc ggtgcccttt ccctctccat 268

ttgccctgcc acacagtggg ctggggttgc acgtgtgttt gctgacaggc cacatctcta 328

actgtgggcc atgtggacct taggcctgac cagaccctca tgtcttcctc cttcccag 386

gac gtg cac cac ctg cac gcc gag agg cgc ggc cct cag ccc tgg cac 434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc 482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat 530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag 578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc 626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc 674
Lys Glu Phe Ile Ala Trp Leu Val Lys Gly Arg Gly Arg Arg Asp Phe
            120                 125                 130

cct gag gag gtg gcc atc gtg gag gag ctg ggc cgg cga cac gcc gac 722

Pro Glu Glu Val Ala Ile Val Glu Glu Leu Gly Arg Arg His Ala Asp
      135                 140                 145

ggc agc ttc agc gac gag atg aac acc atc ctg gac aac ctg gcc gcg      770
Gly Ser Phe Ser Asp Glu Met Asn Thr Ile Leu Asp Asn Leu Ala Ala
    150                 155                 160

cgc tga tatc                                                         780
Arg
165

<210>  18
<211>  720
<212>  DNA
<213>  Artificial

<220>
<223>  Description of sequence: DNA sequence of the construct according
       to Fig. 1l

<400>  18
gatatccacc atggcccccg ccgcctggct gaggagcgcc gccgccaggg ccctgctgcc      60

acccatgctg ctgctgctgc tgcagccccc acctctgctg gcccgggccc tgcccccggt     120

gagtgcccgc cactcgccgt ccgctcctcg ctgagggggc gccgggcacg cgggctgggc     180

ccagcggcgt atccggacgc caagaaacca gagagccagc cagatgccaa agggccctgc     240

catgtgccgg tgccctttcc ctctccattt gccctgccac acagtgggct ggggttgcac     300

gtgtgtttgc tgacaggcca catctctaac tgtgggccat gtggacctta ggcctgacca     360

gaccctcatg tcttcctcct tcccaggacg tgcaccacct gcacgccgag aggcgcggcc     420

ctcagccctg gcacgccgcc ctgccaagca gccctgcccc tgccccagcc acccaggagg     480

cccccaggcc tgccagcagc ctgaggccac ccaggtgcgg cgtgcctgat ccctccgatg     540

gcctgagcgc tcggaatcgg cagaagaggc acgccgaggg caccttcacc tccgacgtga     600

gcagctacct ggagggccag gccgccaagg agttcatcgc ctggctggtg aagggcaggg     660

acggcagctt cagcgacgag atgaacacca tcctggacaa cctggccgcg cgctgatatc     720

<210>  19
<211>  716
<212>  DNA
<213>  Artificial

<220>
<223>  Description of sequence: DNA sequence and sequence of the
       translated peptide according to Fig. 1m

<220>
<221>  CDS
<222>  (11)..(118)

<220>
<221>  CDS
<222>  (387)..(710)

<400>  19
gatatccacc atg gcc ccc gcc gcc tgg ctg agg agc gcc gcc gcc agg        49
           Met Ala Pro Ala Ala Trp Leu Arg Ser Ala Ala Ala Arg
           1               5                   10

gcc ctg ctg cca ccc atg ctg ctg ctg ctg ctg cag ccc cca cct ctg 97
Ala Leu Leu Pro Pro Met Leu Leu Leu Leu Leu Gln Pro Pro Pro Leu
    15                  20                  25

ctg gcc cgg gcc ctg ccc ccg gtgagtgccc gccactcgcc gtccgctcct 148
Leu Ala Arg Ala Leu Pro Pro
30                  35

cgctgagggg gcgccgggca cgcgggctgg gcccagcggc gtatccggac gccaagaaac 208

cagagagcca gccagatgcc aaagggccct gccatgtgcc ggtgcccttt ccctctccat 268

ttgccctgcc acacagtggg ctggggttgc acgtgtgttt gctgacaggc cacatctcta 328

actgtgggcc atgtggacct taggcctgac cagaccctca tgtcttcctc cttcccag 386

gac gtg cac cac ctg cac gcc gag agg cgc ggc cct cag ccc tgg cac 434
Asp Val His His Leu His Ala Glu Arg Arg Gly Pro Gln Pro Trp His
            40                  45                  50

gcc gcc ctg cca agc agc cct gcc cct gcc cca gcc acc cag gag gcc 482
Ala Ala Leu Pro Ser Ser Pro Ala Pro Ala Pro Ala Thr Gln Glu Ala
        55                  60                  65

ccc agg cct gcc agc agc ctg agg cca ccc agg tgc ggc gtg cct gat 530
Pro Arg Pro Ala Ser Ser Leu Arg Pro Pro Arg Cys Gly Val Pro Asp
    70                  75                  80

ccc tcc gat ggc ctg agc gct cgg aat cgg cag aag agg cac gcc gag 578
Pro Ser Asp Gly Leu Ser Ala Arg Asn Arg Gln Lys Arg His Ala Glu
85                  90                  95                  100

ggc acc ttc acc tcc gac gtg agc agc tac ctg gag ggc cag gcc gcc 626
Gly Thr Phe Thr Ser Asp Val Ser Ser Tyr Leu Glu Gly Gln Ala Ala
                105                 110                 115

aag gag ttc atc gcc tgg ctg gtg aag ggc agg ggc cgc agg gac ttc 674
Lys Glu Phe Ile Ala Trp Leu Val Lys Gly Arg Gly Arg Arg Asp Phe
            120                 125                 130

cct gag gag gtg gcc atc gtg gag gag ctg ggc tga gcgcgc 716
Pro Glu Glu Val Ala Ile Val Glu Glu Leu Gly
        135                 140

<210> 20
<211> 716
<212> DNA
<213> Artificial

<220>
<223> Description of sequence: DNA sequence of the construct according
to Fig. 1m

<400> 20
gatatccacc atggcccccg ccgcctggct gaggagcgcc gccgccaggg ccctgctgcc 60

acccatgctg ctgctgctgc tgcagccccc acctctgctg gcccgggccc tgcccccggt 120

gagtgcccgc cactcgccgt ccgctcctcg ctgagggggc gccgggcacg cgggctgggc 180

ccagcggcgt atccggacgc caagaaacca gagagccagc cagatgccaa agggccctgc 240

catgtgccgg tgccctttcc ctctccattt gccctgccac acagtgggct ggggttgcac 300

gtgtgtttgc tgacaggcca catctctaac tgtgggccat gtggacctta ggcctgacca 360

gaccctcatg tcttcctcct tcccaggacg tgcaccacct gcacgccgag aggcgcggcc 420

ctcagccctg gcacgccgcc ctgccaagca gccctgcccc tgccccagcc acccaggagg 480

cccccaggcc tgccagcagc ctgaggccac ccaggtgcgg cgtgcctgat ccctccgatg 540

gcctgagcgc tcggaatcgg cagaagaggc acgccgaggg caccttcacc tccgacgtga 600

gcagctacct ggagggccag gccgccaagg agttcatcgc ctggctggtg aagggcaggg 660

gccgcaggga cttccctgag gaggtggcca tcgtggagga gctgggctga gcgcgc 716

<210> 21
<211> 31
<212> PRT
<213> Artificial

<220>
<223> Description of sequence: artificial

<400> 21

His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210> 22
<211> 10
<212> PRT
<213> Artificial

<220>
<223> Description of sequence: sequence contained in component (II) of
a GLP-1 fusion peptide

<400> 22

Arg Arg Asp Phe Pro Glu Glu Val Ala Ile
1               5                   10

<210> 23
<211> 14
<212> PRT
<213> Artificial

<220>
<223> Description of sequence: sequence contained in component (II) of
a GLP-1 fusion peptide

<400> 23

Arg Arg Asp Phe Pro Glu Glu Val Ala Ile Val Glu Glu Leu
1               5                   10

<210> 24
<211> 14
<212> PRT
<213> Artificial

<220>
<223> Description of sequence: sequence contained in component (II) of
a GLP-1 fusion peptide

<400>  24

Arg Arg Asp Phe Pro Glu Glu Val Ala Ile Ala Glu Glu Leu
1               5                   10


<210>  25
<211>  31
<212>  PRT
<213>  Artificial

<220>
<223>  Description of sequence: sequence according to formula (I)


<220>
<221>  misc_feature
<222>  (31)..(31)
<223>  Xaa = NH2, when sequence is GLP-1(7-36) amide, or Xaa = Gly-OH,
       when sequence is GLP-1(7-37) amide,

<400>  25

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg Xaa
            20                  25                  30


<210>  26
<211>  584
<212>  DNA
<213>  Artificial

<220>
<223>  Description of sequence: GLP-1 fusion peptide corresponding to
       Figure 1 e #217


<220>
<221>  CDS
<222>  (31)..(561)

<400>  26
aattcagata attcgatagc cccgggcacc atg gct ccc gct gca tgg ctg aga        54
                                 Met Ala Pro Ala Ala Trp Leu Arg
                                 1               5

tct gcg gcc gcg cgc gcc ctc ctg ccc ccg atg ctg ctg ctg ctg ctc        102
Ser Ala Ala Ala Arg Ala Leu Leu Pro Pro Met Leu Leu Leu Leu Leu
    10                  15                  20

cag ccg ccg ccg ctg ctg gcc cgg gct ctg ccg ccg gac gtc cac cac        150
Gln Pro Pro Pro Leu Leu Ala Arg Ala Leu Pro Pro Asp Val His His
25                  30                  35                  40

ctc cat gcc gag agg agg ggg cca cag ccc tgg cat gca gcc ctg ccc        198
Leu His Ala Glu Arg Arg Gly Pro Gln Pro Trp His Ala Ala Leu Pro
                45                  50                  55

agt agc ccg gca cct gcc cct gcc acg cag gaa gcc ccc cgg cct gcc        246
Ser Ser Pro Ala Pro Ala Pro Ala Thr Gln Glu Ala Pro Arg Pro Ala
            60                  65                  70

agc agc ctc agg cct ccc cgc tgt ggc gtg ccc gac cca tct gat ggg        294
Ser Ser Leu Arg Pro Pro Arg Cys Gly Val Pro Asp Pro Ser Asp Gly
        75                  80                  85

ctg agt gcc cgc aac cga cag aag agg cat gcc gaa ggg acc ttt acc          342
Leu Ser Ala Arg Asn Arg Gln Lys Arg His Ala Glu Gly Thr Phe Thr
    90                  95                  100

agc gat gtg agc tct tat ctg gaa ggc cag gct gcc aag gag ttc att          390
Ser Asp Val Ser Ser Tyr Leu Glu Gly Gln Ala Ala Lys Glu Phe Ile
105                 110                 115                 120

gct tgg ctg gtg aaa ggc cgg gga agg cgg gat ttc cca gag gag gtg          438
Ala Trp Leu Val Lys Gly Arg Gly Arg Arg Asp Phe Pro Glu Glu Val
                125                 130                 135

gcc atc gtg gag gag ctg ggc cgg cga cat gcc gaa ggg acc ttt acc          486
Ala Ile Val Glu Glu Leu Gly Arg Arg His Ala Glu Gly Thr Phe Thr
            140                 145                 150

agc gat gtg agc tct tat ctg gaa ggc cag gct gcc aag gag ttc att          534
Ser Asp Val Ser Ser Tyr Leu Glu Gly Gln Ala Ala Lys Glu Phe Ile
        155                 160                 165

gct tgg ctg gtg aaa ggc cgg gga tga attgccaagg gcgaattatc agg            584
Ala Trp Leu Val Lys Gly Arg Gly
    170                 175


<210>  27
<211>  8
<212>  PRT
<213>  Artificial Sequence

<220>
<223>  Description of sequence: sequence contained in component (II) of
       a GLP-1 fusion peptide

<400>  27

Asp Phe Pro Glu Glu Val Ala Ile
1               5


<210>  28
<211>  8
<212>  PRT
<213>  Artificial Sequence

<220>
<223>  Description of sequence: sequence contained in component (II) of
       a GLP-1 fusion peptide

<400>  28

Arg Asp Phe Pro Glu Glu Val Ala
1               5


<210>  29
<211>  8
<212>  PRT
<213>  Artificial Sequence

<220>
<223>  Description of sequence: sequence contained in component (II) of
       a GLP-1 fusion peptide

<400>  29

Arg Arg Asp Phe Pro Glu Glu Val
1               5

<210>  30
<211>  10
<212>  PRT
<213>  Artificial Sequence

<220>
<223>  Description of sequence: sequence contained in component (II) of
       a GLP-1 fusion peptide

<400>  30

Ala Ala Asp Phe Pro Glu Glu Val Ala Ile
1               5                   10


<210>  31
<211>  8
<212>  PRT
<213>  Artificial Sequence

<220>
<223>  Description of sequence: sequence contained in component (II) of
       a GLP-1 fusion peptide

<400>  31

Ala Asp Phe Pro Glu Glu Val Ala
1               5


<210>  32
<211>  8
<212>  PRT
<213>  Artificial Sequence

<220>
<223>  Description of sequence: sequence contained in component (II) of
       a GLP-1 fusion peptide

<400>  32

Ala Ala Asp Phe Pro Glu Glu Val
1               5


<210>  33
<211>  14
<212>  PRT
<213>  Artificial Sequence

<220>
<223>  Description of sequence: sequence contained in component (II) of
       a GLP-1 fusion peptide

<400>  33

Ala Ala Asp Phe Pro Glu Glu Val Ala Ile Val Glu Glu Leu
1               5                   10


<210>  34
<211>  14
<212>  PRT
<213>  Artificial Sequence

<220>
<223>  Description of sequence: sequence contained in component (II) of

a GLP-1 fusion peptide

<400> 34

Ala Ala Asp Phe Pro Glu Glu Val Ala Ile Ala Glu Glu Leu
1               5                   10


<210> 35
<211> 15
<212> PRT
<213> Artificial Sequence

<220>
<223> Description of sequence: sequence contained in component (II) of
a GLP-1 fusion peptide

<400> 35

Ala Ala Asp Phe Pro Glu Glu Val Ala Ile Val Glu Glu Leu Gly
1               5                   10                  15


<210> 36
<211> 15
<212> PRT
<213> Artificial Sequence

<220>
<223> Description of sequence: sequence contained in component (II) of
a GLP-1 fusion peptide

<400> 36

Ala Ala Asp Phe Pro Glu Glu Val Ala Ile Ala Glu Glu Leu Gly
1               5                   10                  15


<210> 37
<211> 46
<212> PRT
<213> Artificial Sequence

<220>
<223> Description of sequence: sequence of a GLP-1 fusion peptide

<400> 37

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg Gly Ala
            20                  25                  30


Ala Asp Phe Pro Glu Glu Val Ala Ile Ala Glu Glu Leu Gly
        35                  40                  45


<210> 38
<211> 46
<212> PRT
<213> Artificial Sequence

<220>
<223> Description of sequence: sequence of a GLP-1 fusion peptide

<400> 38

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Phe Pro Glu Glu Val Ala Ile Val Glu Glu Leu Gly
        35                  40                  45

<210>  39
<211>  46
<212>  PRT
<213>  Artificial Sequence

<220>
<223>  Description of sequence: sequence of a GLP-1 fusion peptide

<400>  39

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Ala Glu Glu Val Ala Ile Ala Glu Glu Leu Gly
        35                  40                  45

<210>  40
<211>  46
<212>  PRT
<213>  Artificial Sequence

<220>
<223>  Description of sequence: sequence of a GLP-1 fusion peptide

<400>  40

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Ala Ala Ala Ala Val Ala Ile Ala Glu Glu Leu Gly
        35                  40                  45

<210>  41
<211>  46
<212>  PRT
<213>  Artificial Sequence

<220>
<223>  Description of sequence: sequence of a GLP-1 fusion peptide

<400>  41

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Ala Ala Ala Ala Val Ala Ile Ala Ala Ala Leu Gly
        35                  40                  45

<210>  42
<211>  36
<212>  PRT
<213>  Artificial Sequence

<220>
<223>  Description of sequence: sequence of a GLP-1 fusion peptide

<400>  42

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro
        35

<210>  43
<211>  40
<212>  PRT
<213>  Artificial Sequence

<220>
<223>  Description of sequence: sequence of a GLP-1 fusion peptide

<400>  43

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala
        35                  40

<210>  44
<211>  51
<212>  PRT
<213>  Artificial Sequence

<220>
<223>  Description of sequence: sequence of a GLP-1 fusion peptide

<400>  44

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg

20                  25                  30

Arg Asp Phe Pro Glu Glu Val Ala Ile Ala Glu Glu Leu Gly Arg Arg
        35                  40                  45

His Ala Cys
    50

<210>  45
<211>  46
<212>  PRT
<213>  Artificial Sequence

<220>
<223>  Description of sequence: sequence of a GLP-1 fusion peptide

<400>  45

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Ala Glu Glu Val Ala Ile Val Glu Glu Leu Gly
        35                  40                  45

<210>  46
<211>  46
<212>  PRT
<213>  Artificial Sequence

<220>
<223>  Description of sequence: sequence of a GLP-1 fusion peptide

<400>  46

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Ala Ala Ala Ala Val Ala Ile Val Glu Glu Leu Gly
        35                  40                  45

<210>  47
<211>  46
<212>  PRT
<213>  Artificial Sequence

<220>
<223>  Description of sequence: sequence of a GLP-1 fusion peptide

<400>  47

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Asp Ala Ala Ala Ala Val Ala Ile Val Ala Ala Leu Gly
        35                  40                  45

<210>  48
<211>  51
<212>  PRT
<213>  Artificial Sequence

<220>
<223>  Description of sequence: sequence of a GLP-1 fusion peptide

<400>  48

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Arg Asp Phe Pro Glu Glu Val Ala Ile Val Glu Glu Leu Gly Arg Arg
        35                  40                  45

His Ala Cys
    50

## Description

The present application refers to cells, e.g. mesenchymal stem cells or mesenchymal stromal cells, or any further suitable cell, encoding and secreting an anti-angiogenic factor, a neuroprotective factor and/or any other protein or protein-like substance suitable for (intraocular) treatment of eye diseases. Such eye diseases include glaucoma and other optic nerve disorders, retinal diseases, particularly retinitis pigmentosa (RP), age-related macular degeneration (AMD) and diabetic retinopathy. The cells used herein are encapsulated in a (spherical) microcapsule to prevent a response of the immune system of the patient to be treated. The present application also refers to the use of these (spherical) microcapsule(s) or such factors for (intraocular) treatment of eye diseases as defined herein (for the preparation of a (pharmaceutical) composition) for the treatment of such eye diseases.

Eye diseases or disorders are of particular interest as these diseases or disorders may lead in some patients to severe impairment of visual perception and even blindness. Of particular interest among those eye diseases or disorders are glaucomas and other optic nerve diseases, and retinal disorders. Such retinal disorders may include among others retinitis pigmentosa (RP), macular degeneration (MD), age-related macular degeneration (AMD or ARMD), macula edema, macula edema particularly due to other reasons, retinal vessel occlusions, hypertensive retinopathy, and diabetic retinopathy, etc.

In the above context, retinitis pigmentosa (RP) is classified as a group of genetic eye conditions. In the progression of symptoms for retinitis pigmentosa, night blindness generally precedes tunnel vision by years or even decades. Many people with retinitis pigmentosa do not become legally blind until their 40s or 50s and retain some sight all their life. Others go completely blind from retinitis pigmentosa, in some patients as early as childhood. Retinitis pigmentosa is one of the most common forms of hereditary retinal dystrophies, a group of inherited disorders, which is characterized by the progressive loss of photoreceptor cells, abnormalities of the photoreceptors (rods and cones) and/or the retinal pigment epithelium (RPE) of the retina, which may lead to progressive visual loss. Affected individuals first experience defective dark adaptation or nyctalopia (night blindness), followed by reduction of the peripheral visual field (known as tunnel vision) and, depending on the specific disease, loss of central vision late in the course of the disease. Retinitis pigmentosa combined with progressive deafness is called Usher syndrome. As a form of hereditary retinal dystrophy multiple genes are supposed, when mutated, to cause the retinitis pigmentosa phenotype. In 1989, a mutation of the gene for rhodopsin, a pigment that plays an essential part in the visual transduction cascade enabling vision in low-light conditions, was identified. Since then, more than 100 mutations have been found in this gene, accounting for 15% of all types of retinal degeneration. Most of those mutations are missense mutations and are typically inherited. Furthermore, the rhodopsin gene encodes a principal protein of photoreceptor outer segments. Studies show that mutations in this gene are responsible for approximately 25% of autosomal forms of retinitis pigmentosa. Additionally, up to 150 mutations have been reported to date in the opsin gene associated with the retinitis pigmentosa since the Pro23His mutation in the intradiscal domain of the protein was first reported in 1990. These mutations are found throughout the opsin gene and are distributed along the three domains of the protein (the intradiscal, transmembrane, and cytoplasmic domains). Apart from heriditary causes, mutations causing retinitis pigmentosa may also be due to biochemical events such as protein misfolding or the involvement of molecular chaperones. At present, there is currently no medical treatment that can completely cure retinitis pigmentosa. Even though treatment with vitamin A represents the most common therapy, it may questionably only reduce the progression of the disease, e.g. by the daily intake of e.g. 15000 IU of vitamin A palmitate. Further treatments may involve retinal transplants, artificial retinal implants, gene therapy, stem cell therapy, nutritional supplements, etc.

A further form of retinal disorders may include macular degeneration (MD). Macular degeneration is a medical condition which represents a major cause of blindness in older adults (>50 years) and in highly myopic subjects. The symptoms of macular degeneration (MD) typically comprise a loss of vision in the center of the visual field (the macula) due to damage to the retina in this area. Macular degeneration can make it difficult or impossible to read or recognize faces, although enough peripheral vision remains to allow other activities of daily life. Diagnosis of MD may be carried out e.g. via fluorescein angiography, which allows for the identification and localization of abnormal vascular processes. Additionally, optical coherence tomography and other newly developed imaging techniques may be used in the diagnosis and evaluation of the response to treatment of MD.

One form of MD is the so called age-related macular degeneration (AMD or ARMD). Besides the glaucomas and diabetic retinopathy, it is one of the most common causes of visual impairment and blindness in Western countries. Onset of age-related macular degeneration is typically associated with characteristic yellowish deposits in the macula (central area of the retina which provides detailed central vision, termed fovea) called drusen between the retinal pigment epithelium and the underlying choroid. Most people with these early changes (referred to as age-related maculopathy) typically have no impaired vision. However, such early changes may relatively often develop to advanced AMD. The risk is considerably higher when the drusen are large and numerous and associated with disturbance in the pigmented cell layer under the macula. Advanced AMD, which is responsible for profound loss of vision, typically occurs in two forms: dry and wet AMD. The "dry" form of advanced AMD, also known as central geographic atrophy, typically results from atrophy of the retinal pigment epithelial layer (and presumably the choriocapillaris) below the retina and causes vision loss through loss of photoreceptors (rods and cones) in the central part of the eye. There can additionally be cellular debris (called *drusen)* accumulating between the retina and the choroid. While no treatment is available for this condition, vitamin supplements with high doses of antioxidants, lutein and zeaxanthin, have been demonstrated by the National Eye Institute and others to slow down the progression of dry macular degeneration and, in some patients, improve visual acuity. The "wet" form of advanced AMD, also known as neovascular or exudative AMD, represents the more severe form of AMD. The exudative age-related Macular Degeneration (AMD) (wet form of AMD) is typically characterized by abnormal blood vessel growth in the eye, wherein the faulty blood vessels leak fluids and blood. It may cause vision loss due to abnormal blood vessel growth from the choriocapillaries through Bruch's membrane into the subretinal space, ultimately leading to blood and protein leakage below the macula. Bleeding, leaking, and scarring from these blood vessels eventually causes irreversible damage to the photoreceptors, scar formation in the macula and relatively rapid vision loss if left untreated.

Until today, there are only few treatments available for AMD, particularly for the wet form of AMD. Exudative age-related Macular Degeneration (AMD) is nowadays typically treated with intraocular medication, and only relatively rarely with (additonal) laser coagulation. The intraocular medication includes the use of anti-angiogenic agents that may lead to regression of the abnormal blood vessels and improvement of vision when injected directly into the vitreous humor of the eye. Such anti-angiogenic agents, including agents effective against VEGF (anti-vascular endothelial growth factor), are used to prevent formation of abnormal, new blood vessels in the eye, and are capable to dry up vessels which have already begun to leak. Unfortunately, these injections have to be repeated frequently on a bi-monthly or monthly basis and harbour injection-related risks, particularly the risk of an intraocular infection. Examples of these agents include Lucentis™, Avastin™ (structrally closely related to Lucentis™) and Macugen™. Of these anti-angiogenic agents only Lucentis and Macugen are FDA-approved as of January 2009. Macugen has been found to have relatively low benefits in neovascular AMD, so that the use of Lucentis™ by far outweighs the use of Macugen™.

Oher relatively common retinal disorders include retinal vascular occlusions. According to recent population-based studies, about 1% of the general population aged 40+ years suffer from a retinal vascular occlusion, mainly retinal vein occlusion or retinal artery occlusions. For retinal artery occlusions, no evidenced-based therapy has been available so far. The occlusion of the artery usually lasts only for a limited duration, after which the artery reopens. It is therefore desirable to a therapy which helps the damaged retinal cells to survive until the artery re-canalizes. In the case of retinal vein occlusions, either retinal laser coagulation and/or intraocular application of drugs have been performed, to reduce the macular edema and to decrease the risk of an intraocular unwanted neovascularization. Also for the retinal vein occlusions, a neuroprotective therapy is warranted to help the cells survive until the surrounding conditions improve.

The glaucomas are a heterogenous group of diseases which are characterized by a loss of retinal ganglion cells and their axons, leading to the ophthalmoscopally typical appearance of the optic nerve head. The intraocular pressure can be elevated (as in so called high-pressure glaucoma" or it can be statistically normal (as in the so called normal-pressure glaucoma). According to relatively recent studies, the orbital cerebrospinal fluid pressure as trans-lamian cribrosa counter-pressure may be abnormally low in some patients with normal-pressure glaucoma. The glaucomas can be chronic (as in most of the open-angle glaucomas) or they can have an acute onset with marked pain (as in the some of the angle-closure glaucomas). The heterogenitiy of the glaucomas explains why different treatment modalities exist. All of them have in common to lower the intraocular pressure. Additonally, a neuroprotective or even neuro-regenerative therapy is necessary, to support damaged cells to survive or even to regenerate.

Quite a number of further agents may be used in general for the treatment of AMD and other retinal disorders, and optic nerve disorders. There are numerous phase 1, 2 and 3 studies going on to examine the clinical efficacy of such new drugs for the therapy of AMD and the other diseases mentioned, in addition to numerous experimental investigations to further develop new treatment strategies. However, there is at present no efficient therapy available which allows for protection or more preferably regeneration of retinal tissue including the optic nerve, the trabecular meshwork and/or the vascular cells as long term treatment as described above. Even though treatment with medicaments (such as vitamin A and vitamin A palmitate) for retinitis pigmentosa (RP) and treatment with drugs (such as Lucentis^{™} and Macugen^{™}) for AMD have been shown to slow down progression of these diseases, no efficient treatment is currently available, since all of these therapeutic strategies have not been able to show a protective effect against cell loss. Surgical interventions such as retinal transplants or artificial retinal implants are still in the early experimental stages. Likewise, efficient therapies such as gene therapy and stem cell therapy, nutritional supplements, etc. have not yet been developed up to date to clinical stage.

Therefore, it is an objective of the present invention to provide an efficient therapy for eye diseases and disorders as mentioned above, particularly of eye diseases and disorders caused by a degeneration of the retina, which avoid these problems and which allow to provide an efficient long term treatment without the need of frequently repeated drug administration and/or the risk of undesired side effects.

The objective underlying the present invention is solved by the attached claims.

The objective underlying the present invention is particularly solved by the use of an anti-angiogenic factor, a neuroprotective factor and/or any other protein or protein-like substance as described herein suitable for (intraocular) treatment of eye diseases, or a fragment or variant thereof, (for the preparation of a pharmaceutical composition) for the (intraocular) treatment of eye diseases or disorders including retinal diseases, particularly retinitis pigmentosa (RP), age-related macular degeneration (AMD), diabetic retinopathy, retinal vessel occlusion and optic neuropathies.

More preferably, the objective underlying the present invention is solved by the use of cells, e.g. mesenchymal stem cells or mesenchymal stromal cells, or any other cell (type), that may be used in the context of the present invention, encoding and secreting an anti-angiogenic factor, a neuroprotective factor and/or any other protein or protein-like substance or a fragment or variant thereof suitable for (intraocular) treatment of eye diseases or disorders as defined herein, (for the preparation of a pharmaceutical composition) for the (intraocular) treatment of eye diseases including retinal diseases, particularly retinitis pigmentosa (RP), age-related macular degeneration (AMD), diabetic retinopathy, retinal vessel occlusion and optic neuropathies, wherein the cells are encapsulated in a (spherical) microcapsule, preferably to prevent a response of the immune system of the patient to be treated. In the context of the present invention the term "cells encoding ... " typically means "cells, which are engineered to contain or comprise nucleic acids encoding ...".

Administration of the anti-angiogenic factor, neuroprotective factor and/or any other protein or protein-like substance suitable for (intraocular) treatment of eye diseases, or a fragment or variant thereof as defined herein via cells embedded in the (spherical) core of the (spherical) microcapsule as described herein allows to circumvent the problem of repeated administration of such factors. The anti-angiogenic factor, neuroprotective factor and/or any other protein or protein-like substance as defined herein may thus be administered by intraocularly providing cells to a patient containing a nucleic acid encoding and expressing such a factor. Implantation of such cells furthermore ensure a longer provision of these factors *in vivo* and, due to secretion of these factors from the grafted cells. They provide these factors directly at the site of interest without the need of repeated administration. It is known in the art that the immune system typically recognizes foreign cells and triggers an immune response in order to protect the organism from such external material. Accordingly, implantation of cells as such capable of expressing the anti-angiogenic factor, neuroprotective factor and/or any other protein or protein-like substance suitable for (intraocular) treatment of eye diseases, or a fragment or variant thereof as defined herein may thus lead to an immune response in the organism. Such a defense response may cause considerable and undesirable side effects during treatment and may lead to severe complications or even death of the treated organism. One strategy to circumvent this problem, which is also envisaged herein, is the use of autologous cells, i.e. cells which are derived from the patient itself, which are genetically altered to transiently express the anti-angiogenic factor, neuroprotective factor and/or any other protein or protein-like substance suitable for (intraocular) treatment of eye diseases, or a fragment or variant thereof as defined herein. Such a procedure is preferably limited to the cells of the specific patient to be treated.

In the context of the present invention, the term "eye disease" particularly comprises eye diseases and disorders caused by a degeneration of the retina, including, among other retinal diseases, retinal diseases, selected from retinitis pigmentosa (RP), macular degeneration (MD), age-related macular degeneration (AMD or ARMD), retinopathy, diabetic retinopathy macula edema due to any other reason, abnormalities of the vascular endothelium and pericytes, retinal vessel occlusions, glaucomas, including the trabecular meshwork, and other optic neuropathies, including alterations of the trabecular meshwork, and abnormalities of the corneal endothelium, etc. or any disease or disorder associated thereto.

The cells used for providing the above inventive solution, encoding and secreting an anti-angiogenic factor, a neuroprotective factor and/or any other protein or protein-like substance or a fragment or variant thereof suitable for (intraocular) treatment of eye diseases, are preferably encapsulated in a (spherical) microcapsule to prevent a response of the immune system of the patient to be treated. In the context of the present invention, such a (spherical) microcapsule preferably comprises a (spherical) core (i.e. the core may be spherical or not) and at least one surface coating layer, wherein:
- the (spherical) core comprises or consists of (a mixture of) typically cross-linked polymers and cells, e.g. mesenchymal stem cells or mesenchymal stromal cells, or any other cell (type), that may be used in the context of the present invention, and which encode and secrete an anti-angiogenic or neuroprotective factor and/or any other protein or protein-like substance or a fragment or variant thereof, as defined herein, preferably suitable for (intraocular) treatment of eye diseases; and
- the at least one surface coating layer comprises or consists of typically cross-linked polymers.

The (spherical) microcapsule, comprising cells as used herein encoding and secreting an anti-angiogenic factor, a neuroprotective factor and/or any other protein or protein-like substance or a fragment or variant thereof, typically comprises a particle size, herein referred to as the total diameter of the (spherical) microcapsule. Generally, the total diameter of the (spherical) microcapsule as used herein may vary considerably depending on the specific treatment and administration mode. In the context of the present invention, the treatment is strictly limited to diseases and disorders of the eye as defined herein. As the treatment typically occurs locally by administration of the (spherical) microcapsule as used herein into a specific administration site, e.g. by injection or implantation, the administration mode may limit the total diameter of the (spherical) microcapsule as used herein, e.g. by the diameter of the injection cannula. The total diameter of the (spherical) microcapsule as used herein is furthermore determined by the diameter of the core of the (spherical) microcapsule as well as by the thickness of the at least one surface coating layer(s), as both diameters typically depend at least in part on each other and of course, influence the total diameter of the (spherical) microcapsule.

For the treatment of eye diseases as defined herein, the inventors of the present application have surprisingly found, that a total diameter (particle size) of the (spherical) microcapsule of about 120 µm to about 800 µm, preferably of about 120 µm to about 700 µm, more preferably a total diameter of about 150 µm to about 650 µm, and even more preferably a total diameter of about 165 µm to about 600 µm may be used. Particularly, a total diameter (particle size) of the (spherical) microcapsule of about 120 µm to about 300 µm, more preferably a total diameter of about 150 µm to about 250 µm, even more preferably a total diameter of about 165 µm to about 225 µm, and most preferably a total diameter of about 180 µm to about 200 µm, e.g. about 180, 185, 190 or 200 µm, is advantageous. (Spherical) microcapsules, comprising such a total diameter, are typically retained in the selected site of injection in the eye to be treated and do not migrate into the surrounding tissue. This allows providing a continuous expression of an anti-angiogenic factor, a neuroprotective factor and/or any other protein or protein-like substance or a fragment or variant thereof suitable for (intraocular) treatment of eye diseases at the site of injection during treatment for a sufficient period of time to provide the entire spectrum of beneficial effects exerted by these factors.

In the above context of (spherical) microcapsules (or a (spherical) core as defined herein), the term "spherical" is understood in its broadest meaning. A spherical particle is preferably understood to have a sphere-like shape, whereby the shape may be symmetrical or asymmetrical, e.g. a (spherical) microcapsule and/or its core may have ellipsoidal shape. In a less preferred embodiment the microcapsule or core used according to the present invention may not be spherical within the above meaning, but may have an arbitrary shape with e.g. protruding or invading segments on the surface of the microcapsule. Whereever in the present disclosure "spherical" microcapsules or cores are mentioned, "non-spherical" microcapsules or cores may be provided, prepared or used as well.

As defined herein, the (spherical) microcapsule as used herein preferably comprises a (spherical) core (i.e. the core may be spherical or not), wherein the (spherical) core comprises or consists of (a mixture of) cross-linked polymers and cells, e.g. mesenchymal stem cells or mesenchymal stromal cells, or any other cell (type), that may be used in the context of the present invention, and which encode and secrete an anti-angiogenic factor, a neuroprotective factor and/or any other protein or protein-like substance or a fragment or variant thereof suitable for (intraocular) treatment of eye diseases, as defined herein.

In the context of the present invention, the typically cross-linked polymers of the (spherical) core of the (spherical) microcapsule form a scaffold structure embedding the cells, e.g. mesenchymal stem cells or mesenchymal stromal cells, or any other cell (type), that may be used in the context of the present invention, in its cavities. These cells may be embedded in the scaffold structure individually or, typically, as aggregates, e.g. as (a pool of) aggregated cells of about 10 to about 10,000 cells, e.g. about 10 to about 500, about 10 to about 1000 or about 10 to about 10000 cells. Preferably, the (spherical) core comprises a homogenous distribution of the cross-linked polymers and of embedded cells as defined herein. Preferably, the core, including the scaffold structure and the embedded cells as defined herein, is prepared according to a method as disclosed below. In this context, it is of critical importance to embed the encapsulated cells of the (spherical) microcapsule, e.g. mesenchymal stem cells or mesenchymal stromal cells, autologous cells or any other cell (type), which may be used in the context of the present invention, entirely in the polymer matrix when preparing (spherical) microcapsules for the use according to the present invention.

The embedded cells, e.g. mesenchymal stem cells or mesenchymal stromal cells, or any other cell (type) as defined herein, that may be used for the (spherical) microcapsule in the context of the present invention, may be present in the core in a concentration of about 1 x 10⁵, about 1 x 10⁶ or about 1 x 10⁷ cells/ml cross-linked scaffold polymer to about 5 x 10⁸ cells/ml cross-linked scaffold polymer, more preferably in a concentration of about 1 x 10⁵, about 1 x 10⁶, or about 1 x 10⁷ cells/ml cross-linked scaffold polymer to 1 x 10⁸ cells/ml cross-linked scaffold polymer and most preferably in a concentration of about 1 x 10⁵, about 1 x 10⁶, or about 2 x 10⁷ cells/ml cross-linked scaffold polymer to 6 x 10⁷ cells/ml cross-linked scaffold polymer.

In the context of the present invention, the core of the (spherical) microcapsule used according to the present invention typically has a diameter (particle size) of not more than the diameter of the total diameter of the (spherical) microcapsule as defined herein. Typically, the core of the (spherical) microcapsule used according to the present invention has a diameter of about 50 µm to about 220 µm, preferably a diameter of about 100 µm to about 200 µm, likewise preferably a diameter of about 115 µm to about 185 µm, more preferably a diameter of about 130 µm to about 170 µm, and even more preferably a diameter of about 145 µm to about 155 µm, e.g. about 120, 125, 130, 135, 140, 145, 150 or 155 µm. Particuarly preferred, the core of the (spherical) microcapsule, as used according to the present invention, has a diameter, which is preferably about 1 to about 80 µm less than the total diameter of the (spherical) microcapsule as defined herein, more preferably about 15 to about 70 µm less than the total diameter of the (spherical) microcapsule as defined herein, and most preferably about 30 to about 60 µm less than the total diameter of the (spherical) microcapsule as defined herein. In other words, the diameter of the core of the (spherical) microcapsule, as used according to the present invention, may have a size of about 50 µm, of about 60 µm, of about 70 µm, of about 80 µm, of about 90 µm, of about 100 µm, of about 110 µm, of about 120 µm, of about 125 µm, of about 130 µm, of about 135 µm, of about 140 µm, of about 145 µm, of about 150 µm, of about 155 µm, of about 160 µm, of about 165 µm, of about 170 µm, of about 175 µm, of about 180 µm, of about 185 µm, of about 190 µm, of about 195 µm, of about 200 µm, of about 205 µm, of about 210 µm, of about 215 µm, or even of about 220 µm, or may comprise any range selected from any two of the above mentioned specific values.

The core of the (spherical) microcapsule as defined herein comprises cells, encoding and secreting an anti-angiogenic factor, a neuroprotective factor and/or any other protein or protein-like substance or a fragment or variant thereof suitable for (intraocular) treatment of eye diseases, as defined herein. Such cells, e.g. mesenchymal stem cells or mesenchymal stromal cells, or any other cell (type), that may be used in the context of the present invention for the (spherical) core, being located at the core periphery or cells protruding out of the scaffold structure may evoke immunological problems, since the immune system will recognize these microcapsules as foreign components and, thus, these microcapsules will be attacked by the immune system.

Although this effect may be avoided by lowering the cell concentration in the initial solution, the present invention allows improving the efficacy of the microcapsule by increasing the core's cell portion. The higher the concentration of cells in the core, the smaller the total volume of the resultant microcapsules to be transplanted, i.e. the more efficient the microcapsules may work at the site of injection. In order to avoid immunological problems when using high concentrations of cells in the (spherical) core of the (spherical) microcapsule, the invention provides at least one surface coating layer applied on the (spherical) core. This surface coating layer does not allow an immune response to occur, even if cells are located very closely to the core periphery, since these cells are not accessible for the host's immune system due to the surface coating layer acting as barrier. This surface coating layer is typically composed of a cross-linked polymer as defined herein without containing any cells. According to a particular preferred embodiment the afore defined (spherical) core is coated with at least one or more than one surface coating layer(s), e.g. 1, 2, 3, 4, 5, 5-10 or more surface coating layer(s), more preferably 1, 2 or 3 surface coating layer(s), most preferably with only one surface coating layer . Typically, each surface coating layer comprises a uniform thickness around the core.

The thickness of the surface coating layer(s) of the (spherical) microcapsule, as used according to the present invention, may be varied almost arbitrarily and is typically in a range of about 1 to about 80 µm, more preferably in a range of about 15 to about 70 µm, and most preferably in a range of about 20 to about 40 µm, e.g. about 30 µm.

The (spherical) core of the (spherical) microcapsule as used herein (and optionally of the at least one surface coating of the (spherical) microcapsule) comprises or consists of (a mixture of) cross-linked polymers. In this context, any pharmaceutically acceptable (cross-linkable) polymer known in the art and being suitable for encapsulation may be used for the formation of the (spherical) core and, independent from each other, the at least one surface coating layer(s) of the (spherical) microcapsule, as defined according to the present invention. Preferably, such polymers are used, which, on the one hand, are permeable in their cross-linked state for supply of oxygen and nutrients from outside, and, on the other hand, allow diffusion of the peptide encoded and secreted by the core cells from the microcapsule into the patient's tissue or body fluids. Furthermore, the cross-linked polymers prevent intrusion of components of the body's immune system through the matrix. By way of example, polymers may be used such as synthetic, semi-synthetic and natural watersoluble (bio)polymers, e.g. from natural polymers such as selected proteins or polymers based on proteins (e.g. collagens, albumins etc.), polyamino acids (e.g. poly-L-lysine, poly-L-glutamic acid, etc.), polysaccharides and their derivatives (e.g. carboxyl methyl cellulose, cellulose sulfate, agarose, alginates including alginates of brown algae (e.g. of species Laminarales, Ectocarpales, Fucales), carrageenans, hyaluronic acid, heparin and related glycosamino sulfates, dextranes and its derivatives, chitosan and their derivatives). Synthetic polymers may also be used such as e.g. aliphatic polyesters (e.g. polylactic acid, polyglycolic acid, polyhydroxybutyrates, etc.), polyamides, polyanhydrides, polyorthoesters, polyphosphazenes, thermoplastic polyurethanes, polyvinyl alcohols, polyhydroxyethylmethacrylates, polymethylmethacrylates and polytetrafluoroethylenes, etc.

Furthermore, block polymers may be used herein accordingly, i.e. polymers derived by combination of two or more of the aforementioned polymers. Such block polymers may be selected by a skilled person depending on the desired properties, e.g. pore size, cross-linking status, toxicity, handling, biocompatibility, etc. Any of the above polymers is defined as a "chemically different polymer" in the context of the present invention, i.e. each of these polymers typically does not exhibit an identical molar mass and structure with any other of the above polymers. In contrast, "chemically identical polymers" means, that the polymers exhibit an identical molar mass and structure.

Finally, mixtures of the above polymers are also encompassed herein, wherein the amounts of polymers contained in such a mixture may be selected by a skilled person depending on the desired properties, e.g. as outlined above. In this respect, mixtures of polymers may be regarded as chemically identical to another polymer mixture ("chemically identical polymers"), if the overall molar mass of the resultant polymer mixture and the corresponding molar percentage of the single polymers of the mixture are identical to the other polymer mixture.

Preferably, the (mixture of) cross-linked polymers of the (spherical) core of the (spherical) microcapsule as used herein (and optionally of the at least one surface coating layer of the (spherical) microcapsule) comprise or consist of alginate(s). Alginates, if used according to present invention as a polymer for the formation of the (spherical) core and/or of the at least one surface coating layer are particularly advantageous due to their biocompatibility and cross-linking properties. From a chemical point of view, alginates are anionic polysaccharides derived from homopolymeric groups of β-D-mannuronic acid and α-L-guluronic acid, separated by heteropolymeric regions of both acids. Alginates are water soluble and form high viscosity solutions in the presence of monovalent cations such as sodium or potassium. A cross-linked water insoluble hydrogel is formed upon interaction of single alginate chains with bi-, tri- or multivalent cations (such as calcium, barium or polylysin). Preferably, purified alginates (e.g. according to DE 198 36 960, the specific disclosure of which is incorporated herein by reference) are used for encapsulation, more preferably potassium or sodium alginates in physiological saline solution. Such alginates typically exhibit an average molar mass of about 20 kDa to about 10,000 kDa, more preferably a molar mass of about 100 kDa to about 1,200 kDa. Alginates used for the formation of the core and/or of the at least one surface coating layer of the (spherical) microcapsule as used according to the present invention, may be provided as a solution, more preferably as an aqueous solution, e.g. the viscosity of a 0.2% (w/v) aqueous alginate solution of the alginate to be used may be in the range of about 2 to about 50 mPa s, more preferably in the range of about 3 to about 10 mPa s. If alginates are used according to the present invention, those, which are rich in α-L-guluronic acid, are preferred. In other words, alginates containing at least 50% α-L-guluronic acid (and less than 50% β-D-mannuronic acid) are preferred. More preferably, the alginate to be used contains 50% to 70% α-L-guluronic acid and 30 to 50% β-D-mannuronic acid. Alginates suitable for preparing (spherical) microcapsules as used according to the present invention are obtainable by extraction from certain algae species including, without being limited thereto, brown algae, e.g. Laminarales, Ectocarpales, Fucales, etc., and other species of algae producing alginates. Alginates may be isolated from fresh algae material or dried material according to any method for preparing alginates known to a skilled person.

Cross-linked polymers as defined herein, used for preparation of the (spherical) core of the (spherical) microcapsule as used herein and cross-linked polymers as defined herein and/or for preparation of the at least one surface coating layer of the (spherical) microcapsule may be identical or different with respect to the selected polymer and with respect to the chosen concentrations.

According to a first embodiment the cross-linked polymers used for preparation of the (spherical) core and the at least one surface coating layer may comprise chemically identical polymers in identical or differing concentrations. Preferably, the polymers present in the (spherical) core and the at least one surface coating layer are prepared using a non-cross-linked polymer solution selected from any of the polymers a defined above. In this polymer solution, the non-cross-linked polymers are typically present in a concentration of about 0.1 % (w/v) to about 8 % (w/v) of the non-cross-linked polymer, more preferably in a concentration of about 0.1 % (w/v) to about 4 % (w/v) of the non-cross-linked polymer, even more preferably in a concentration of about 0.5 % (w/v) to about 2.5 % (w/v) of the non-cross-linked polymer and most preferably in a concentration of about 1 % (w/v) to about 2 % (w/v) of the non-cross-linked polymer. If alginates as disclosed above are used as polymers for the preparation of the (spherical) core of the (spherical) microcapsule as used herein and cross-linked polymers as defined herein and/or for preparation of the at least one surface coating of the (spherical) microcapsule, the concentration of the polymer solution for preparing the (spherical) core and the concentration of the polymer solution for preparing the at least one surface coating layer of the (spherical) microcapsule, may be selected independently upon each other from a concentration of 0.1 to 4% (w/v) of the non-cross-linked polymer, preferably from a concentration of 0.4 to 2% (w/v) of the non-cross-linked polymer. The alginate concentration for both solutions may be identical. Alternatively, different alginate concentrations may be used for preparing the (spherical) core and the at least one surface coating layer of the (spherical) microcapsules used according to the present invention. Preferably, the non-cross-linked polymers used for preparation of the (spherical) core and/or the at least one surface coating layer comprise chemically identical polymers, more preferably in identical concentrations, e.g. in concentrations as defined herein with polymers as defined herein. In this context the term "% (w/v)" refers to the concentration of non-cross-linked polymers and is typically determined on the basis of a certain amount of a polymer in its dry form *versus* the total volume of the polymer solution, e.g. after solubilising the non-cross-linked polymer in a suitable solvent (before the cross-linkage). However, the above concentrations may instead also be meant to correspond to "% v/v" concentrations, if applicable, e.g. if polymers are used, which are present in a fluid aggregate state at standard conditions (room temperature, normal pressure, etc.).

According to a second embodiment the cross-linked polymers used for preparation of the (spherical) core and the at least one surface coating layer may comprise chemically different polymers in identical or differing concentrations. Thereby, concentrations and polymers may be chosen separately as defined herein for the (spherical) core and the at least one surface coating layer independent upon each other. Furthermore, polymers may be chosen from polymers as defined herein, including e.g. natural polymers, synthetic polymers, and combination of polymers, i.e. block polymers. The difference in the nature of the polymers used for the core or the at least one surface coating layer may also be due to different molecular weight of the polymers used and/or due to different cross-linkage of identical polymers, etc.

In case the (spherical) microcapsules comprise more than one surface coating layer, the polymers in each of the at least one surface coating layers may be identical or different, i.e. the cross-linked polymers of each surface coating layer may comprise chemically identical or different polymers in identical or differing concentrations, e.g. the (spherical) microcapsule, as used according to the present invention, may comprise at least one surface coating layer, as defined herein, consisting of any polymer as defined herein, and an additional external surface coating layer consisting of polycations, e.g. polyamino acids as defined herein, e.g. poly-L-lysine, poly-L-glutamic acid, etc. Likewise, the difference in the nature of the polymers used for the differing surface coating layers may be due to a different molecular weight of the polymers used and/or due to different cross-linkage of identical polymers, etc.

The (spherical) core of the (spherical) microcapsule as used herein additionally comprises cells. Such cells are typically selected from stem cells or stromal cells, such as mesenchymal stem cells or mesenchymal stromal cells, or from any other cell (type), that may be used in the context of the present invention, encoding and secreting an anti-angiogenic factor, a neuroprotective factor and/or any other protein or protein-like substance or a fragment or variant thereof suitable for (intraocular) treatment of eye diseases. Such cells are typically obtainable by stably transfecting a cell with a nucleic acid or rather a vector containing a nucleic acid encoding an an anti-angiogenic factor, a neuroprotective factor and/or any other protein or protein-like substance suitable for (intraocular) treatment of eye diseases, or a fragment or variant thereof, as defined below.

Cells suitable for the (spherical) core of the (spherical) microcapsule as used herein may be chosen from (non-differentiated) stem cells including totipotent, pluripotent, or multipotent stem cells. Stem cells used in the present context preferably comprise embryonic stem cells or stem cells derived from the ectoderm, the mesoderm or the endoderm, or adult stem cells such as (human) mesenchymal stem cells or mesenchymal stromal cells (MSC, hMSC) (e.g. derived from human bone marrow or from fat tissue), hematopoietic stem cells, epidermal stem cells, neural stem cells and immature fibroblasts, including fibroblasts from the skin (myofibroblasts), etc. These (undifferentiated) stem cells are typically capable of symmetric stem cell division, i.e. cell division leading to identical copies. Stem cells maintain the capacity of transforming into any cell type. Moreover, stem cells are capable of dividing asymmetrically leading to a copy of the stem cell and another cell different from the stem cell copy, e.g., a differentiated cell. Stem cells as defined herein, particularly mesenchymal stem cells or mesenchymal stromal cells, suitable for the (spherical) core of the (spherical) microcapsule as used herein may additionally produce a set of endogenous trophic factors that support the effect of the encoded and secreted anti-angiogenic factor, neuroprotective factor and/or any other protein or protein-like substance suitable for (intraocular) treatment of eye diseases or of a fragment or variant thereof. Biologically active factors for this paracrine cytoprotective mechanism of the mesenchymal stromal cells may be e.g. the cytokines GRO, IL-6, IL-8, MCP-1 and the growth factors VEGF, GDNF and Neurotrophin-3. According to a particularly preferred embodiment, the cells in the (spherical) core of the (spherical) microcapsules therefore secrete endogenous proteins or peptides as paracrine factors that are released through the capsule in therapeutic levels selected from VEGF, IL6, IL8, GDNF, NT3, and MCP1, etc.

The core of (spherical) microcapsule as used herein, may alternatively contain cells which are chosen from (differentiated) cells, e.g., obtainable from the above stem cells or stromal cells, e.g., cells of the connective tissue family, e.g., (mature) fibroblasts, cartilage cells (chondrocytes), bone cells (osteoblasts/osteocytes, osteoclasts), fat cells (adipocytes), or smooth muscle cells, or blood cells including lymphoid progenitor cells or cells derived therefrom, e.g., NK cells, T-cells, B-cells or dendritic cells, or common myeloid progenitor cells or cells derived therefrom, e.g., dendritic cells, monocytes, macrophages, osteoclasts, neutrophils, eosinophils, basophils, platelets, megakaryocytes or erythrocytes, or macrophages, neuronal cells including astrocytes, oligodendrocytes, etc., or epithelial cells, or epidermal cells. These differentiated cells are typically capable of symmetric cell division, i.e. cell division leading to identical copies of the differentiated parent cell. Moreover, in some cases these differentiated cells may be capable of dividing asymmetrically leading to an identical copy of the parent cell and another cell different from the parent cell, i.e. a cell being further differentiated than the parent cell. Alternatively, in some cases differentiated cells as defined herein may be capable of differentiating further without the need of cell division, e.g., by adding selective differentiation factors.

Furthermore, cells embedded in the (spherical) core of the (spherical) microcapsule, as used according to the present invention, may be cells taken from the patient to be treated himself (autologous cells) or may be taken from allogenic cells (e.g. taken from an established cell line cultivated *in vitro,* e.g., HEK293 cells, hTERT-MSC cells, etc.). Due to the surface coating layer embedding the (spherical) core in the (spherical) microcapsule, as used according to the present invention, it allows the use of allogenic cells without evoking any undesired immune response by the patient to be treated.

Cells embedded in the (spherical) core of the (spherical) microcapsule used according to the present invention, may furthermore be a combination of (differentiated and/or non-differentiated) cell types as defined herein. The (spherical) core of the (spherical) microcapsule, as used according to the present invention, may contain, e.g., human mesenchymal stem cells or human mesenchymal stromal cells, wherein a portion of these cells may be differentiated *in vitro* or *in vivo* into a cell type, such as defined herein, e.g. adipocytes (suitable for transplantation into fat tissue), etc. Accordingly, various cell types (derived e.g. from a specific stem cell type) may be allocated in the core, e.g. sharing a common lineage.

In summary, cells suitable for preparing the (spherical) core of the (spherical) microcapsule used according to the present invention may be selected from non-differentiated or differentiated cells. According to one embodiment non-differentiated cells as defined herein may be preferred. Such non-differentiated cells may provide advantageous properties, e.g. a prolonged effect of the (spherical) microcapsules used according to the present invention, e.g. the prolonged capability to express and secrete an anti-angiogenic factor, a neuroprotective factor and/or any other protein or protein-like substance suitable for (intraocular) treatment of eye diseases, or a fragment or variant thereof, e.g. due to a longer life span of such non-differentiated cells. In an alternative embodiment, differentiated cells as defined herein may be preferred for preparing the (spherical) core of the (spherical) microcapsule used according to the present invention, since they typically do not proliferate any more and, thus, do not lead to any undesired proliferation of cells within the (spherical) core of the (spherical) microcapsule, as used according to the present invention. Specific differentiation of cells may be carried out by a skilled person *in vitro* according to methods known in the art by adding selected differentiation factors to precursor cells. Preferably, cells are differentiated in such a way that a vast majority of cells (or at least 90%, more preferably at least 95 % and most preferably at least 99%) embedded in the (spherical) core of the (spherical) microcapsule used according to the present invention, belongs to the same cell type. In particular, mesenchymal stem cells as defined herein may be differentiated *in vitro,* e.g., into osteoblasts, chondrocytes, adipocytes such as fat cells, neuron-like cells such as brain cells, etc., and used herein accordingly. As to whether non-differentiated or differentiated cells are used for preparing the (spherical) core of the (spherical) microcapsule, as defined herein, may be dependent on specific requirements of the disease to be treated, e.g. the site of affliction, the administration mode, the tissue chosen for implant, etc. A selection of appropriate cells may be carried out by a skilled person evaluating these criteria.

Furthermore, cells suitable for preparing the (spherical) core of the (spherical) microcapsule as defined herein may be immortalised or non-immortalised cells, preferably immortalised cells. If immortalised cells are used, these cells preferably retain their capability of symmetric and/or asymmetric cell division as discussed above. According to the present invention cells are defined as immortal when they exceed the double life span of normal cells (i.e. of non-immortalised cells). The maximum life span of normal diploid cells *in vitro* varies dependent on the cell type (e.g. foetal *versus* adult cell) and culture conditions. Thus, the maximum life span of cultured normal cells *in vitro* is approximately 60-80 population doublings. For example, keratinocytes may divide around 80 times, fibroblasts more than 50 times, and lymphocytes about 20 times. Normal bone marrow stromal cells may exhibit a maximum life span of 30-40 population doublings. Preferably, a cell line used for preparation of the (spherical) core of an (spherical) microcapsule, as used according to the present invention, may continuously grow past 350 population doublings and may still maintain a normal growth rate characteristic of young cells.

Methods for immortalising cells for preparing the (spherical) core of the (spherical) microcapsule as defined herein are widely known in the art and may be applied here accordingly (see e.g. WO 03/010305 or WO 98/66827, which are incorporated herein by reference). An exemplary method (according to WO 03/010305) comprises e.g. following steps:
a) culturing cells, e.g., stem cells, in particular stem cells derived from human bone marrow (e.g. (human) mesenchymal stem cells (MSC, hMSC)), in accordance with standard conventional cell culturing methods known to the skilled person;
b) transducing said cell cultures with a retroviral vector, comprising at least a fragment of the human telomerase reverse transcriptase (hTERT) gene or a variant thereof, by
   b1) culturing a packaging cell line (e.g. PA317 cells, PG13 cells, Phenix, etc.), wherein the packaging cell line are cells in which the retroviral vector is produced,
   b2) constructing a retroviral vector (e.g. derived from Moloney murine leukaemia virus, etc.), wherein the retroviral vector comprises at least a fragment of the catalytic subunit of the human telomeric repeat (hTRT) gene or a variant thereof, more preferably a hTERT cDNA fragment, e.g. a 3452 base pair EcoRI fragment from pGRN145 (Geron Corporation),
   b3) transfecting said packaging cell line, with said retroviral vector,
   b4) transducing said packaging cell line with said transfected cells, preferably by centrifuging the cells with the retroviral vector,
   b5) transducing cultured cells according to step a) above with the packaging cells of step b4), said cells comprising said retroviral vector.
c) obtaining an immortal cell line, wherein said immortalised cell line has substantially identical characteristics and properties compared to the cells of step a). As a result the inserted polynucleotide sequence derived from the human telomeric subunit (hTRT) gene may be transcribed and translated to produce a functional telomerase. One of skill will recognize that due to codon degeneracy a number of polynucleotide sequences will encode the same telomerase. In addition, telomerase variants are included, which have sequences substantially identical to a wildtype telomerase sequence and retain the function of the wildtype telomerase polypeptide (e.g. resulting from conservative substitutions of amino acids in the wildtype telomerase polypeptide).

Cells embedded in the (spherical) core of the (spherical) microcapsule used according to the present invention, are typically engineered to encode and secrete or naturally encode and secrete an anti-angiogenic factor, a neuroprotective factor and/or any other protein or protein-like substance suitable for (intraocular) treatment of eye diseases, or a fragment or variant thereof as defined herein.

In the context of the present invention, an anti-angiogenic factor, a neuroprotective factor and/or any other protein or protein-like substance suitable for (intraocular) treatment of eye diseases, or a fragment or variant thereof as defined herein may be selected from any protein or peptide known by a skilled person to exhibit an anti-angiogenic and/or neuroprotective or a further positive effect with respect of eye diseases, e.g. an antiapoptotic effect.

The neuroprotective aspect or, more precisely, the cell protective aspect of an intraocular drug therapy using the anti-angiogenic factor, neuroprotective factor and/or any other protein or protein-like substance suitable for (intraocular) treatment of eye diseases, or a fragment or variant thereof as defined herein may have an impact on almost any disorder affecting the retina including the optic nerve (as extension of the retina), the corneal endothelium (in case of progressive corneal endothelial dystrophies or other disorders), and the trabecular meshwork in the anterior chamber angle (as the site of an increased aqeous humour outflow resistance leading to elevation of intraocular pressure and consequently to high-pressure glaucoma). The aim of such a cell protective therapy is to prevent or to at least slow down the loss of cells necessary for the function of the eye. The neuroprotective aspect of a therapy may particularly be important for diabetic retinopathy, since the retinal cells loss due to the diabetic retinal microangiopathy is the most important reason for decreased visual performance of eyes with diabetic retinopathy. An intraocular cell protective therapy may also include the corneal endothelium and the capillary pericytes, which are particularly affected by the diabetic process, in an attempt to keep these capillaries open for blood perfusion, or to re-canalize them to improve an impaired blood supply to the retinal tissue.

The anti-angiogenic factor, neuroprotective factor and/or any other protein or protein-like substance suitable for (intraocular) treatment of eye diseases, or a fragment or variant thereof as defined herein may be administered directly or via cells embedded in the (spherical) core of the (spherical) microcapsule as described herein.

If the anti-angiogenic factor, neuroprotective factor and/or any other protein or protein-like substance suitable for (intraocular) treatment of eye diseases, or a fragment or variant thereof as defined herein are to be administered via cells embedded in the (spherical) core of the (spherical) microcapsule as described herein, the cells embedded in the (spherical) core of the (spherical) microcapsule are typically transfected prior to preparing the (spherical) core with nucleic acid sequences encoding such an anti-angiogenic factor, neuroprotective factor and/or any other protein or protein-like substance suitable for (intraocular) treatment of eye diseases, or a fragment or variant thereof. Such transfection enables the cells cells to express and secrete the anti-angiogenic factor, neuroprotective factor and/or any other protein or protein-like substance suitable for (intraocular) treatment of eye diseases, or a fragment or variant thereof.

According to a particularly preferred embodiment, the anti-angiogenic, neuroprotective factor and/or any other protein or protein-like substance suitable for (intraocular) treatment of eye diseases, or a fragment or variant thereof as defined herein may be selected from the anti-angiogenic factor Endostatin. Endostatin is the naturally-occurring 20-kDa C-terminal cleavage product of collagen XVIII. It is reported to serve as an anti-angiogenic agent, similar to angiostatin and thrombospondin. Endostatin is an angiogenesis inhibitor exhibiting a broad spectrum and may interfere with the pro-angiogenic action of growth factors such as basic fibroblast growth factor (bFGF/FGF-2) and vascular growth factor (VEGF).

According to another preferred embodiment, the anti-angiogenic, neuroprotective factor and/or any other protein or protein-like substance suitable for (intraocular) treatment of eye diseases, or a fragment or variant thereof as defined herein may be selected from a GLP-1 peptide as defined herein.

The neuroprotective factor GLP-1 is located on the well studied glucagon gene, which encodes preproglucagon (see e.g. White, J.W. et al., 1986 Nucleic Acid Res. 14(12) 4719-4730). The preproglucagon molecule as a high molecular weight precursor molecule is synthesized in pancreatic alpha cells and in the jejunum and colon L cells. Preproglucagon is a 180 amino acid long prohormone and its sequence contains, in addition to glucagon, two sequences of related structure: glucagon-like peptide-1 (GLP-1) and glucagon-like peptide-2 (GLP-2). In the preproglucagon molecule, between GLP-1 and GLP-2 is a 17 amino acid peptide sequence (or rather a 15 amino acid sequence plus the C-terminal RR cleavage site), intervening peptide 2 (IP2). The IP2 sequence (located between GLP-1 and GLP-2 in the precursor molecule) is normally cleaved proteolytically after aa 37 of GLP-1 *in vivo.* The preproglucagon module is therefore cleaved into various peptides, depending on the cell, and the environment, including GLP-1 (1-37), a 37 amino acid peptide in its unprocessed form. Generally, this processing occurs in the pancreas and the intestine. The GLP-1 (1-37) sequence can be further proteolytically processed into active GLP-1 (7-37), the 31 amino acid processed form, or its further degeneration product GLP-1 (7-36) amide.

Accordingly, the designation GLP-1 (7-37) means that the fragment in question comprises the amino acid residues (starting) from (and including) number 7 to (and including) number 37 when counted from the N-terminal end of the parent peptide, GLP-1. The amino acid sequence of GLP-1 (7-36), GLP-1 (7-36)amide and of GLP-1 (7-37) is given in formula I (SEQ ID NO: 25):
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-X (I)
which shows GLP-1 (7-36)amide when X is NH₂ or GLP-1 (7-36), when X is absent, and GLP-1 (7-37) when X is Gly-OH.

GLP-1 is a gut hormone and is the most potent endogenous insulinotropic agent with actions that include stimulating adenylate cyclase and protein kinase activity in the beta-cell. Physiologically, together with gastric inhibitory polypeptide from the upper gut, it functions as an incretin hormone lowering the blood glucose level. Accordingly, GLP-1, secreted in response to food intake, has multiple effects on, e.g., the stomach, liver, pancreas and brain that work in concert to regulate blood sugar. Consequently, Glucagon-like peptide GLP-1 (7-36)amide, and its non-amidated analogue GLP-1 (7-37) have attracted considerable interest because of their potent actions on carbohydrate metabolism and its potential applicability to the treatment of diabetes, including type 2 diabetes. Further neuroprotective properties have only been shown in the treatment of coronary heart diseases.

According to one embodiment of the present invention, the GLP-1 peptide may therefore be selected from any known GLP-1 peptide sequence, e.g. as defined herein. In this context, the GLP-1 peptide may be secreted by cells embedded in the (spherical) core of the (spherical) microcapsule which thus may be transfected preferably prior to preparing the (spherical) core with nucleic acid sequences encoding a GLP-1 peptide as defined herein such that these cells express and secrete the GLP-1 peptide. Preferably a GLP-1 peptide as used herein, which may be encoded and secreted by a cell embedded in the (spherical) microcapsule, may be selected from a group consisting of a peptide comprising aa 7 - 35 of (wt) GLP-1 and a peptide showing an identity of at least 80 %, 90 %, 95 % or even 99 % with this peptide. In general, the GLP-1 peptide may be selected from group consisting of (i) a peptide comprising aa 1 - 37 of (wt) GLP-1, (ii) a peptide comprising aa 7 - 35, 36 or 37 of (wt) GLP-1, (iii) GLP-1(7-36)amide and (iv) a peptide showing an identity of at least 80 %, 90 %, 95 % or even 99 % with any of these peptides, including modified peptides. In this context, a "modified GLP-1 peptide" is intended to mean any GLP-1 variant or a GLP-1 fragment, including combinations, e.g. a fragment of a variant, which retain the biological function of (wt) GLP-1. Variants and fragments are categorized as modifications of the unmodified GLP-1 sequence, e.g. GLP-1 (7-35, 36 or 37). Within the meaning of the present invention any variant or fragment has to be functional, e.g. has to exert the same or a similar biological activity as the unmodified (GLP-1) peptide. The term "activity" refers to the biological activity (e.g. one or more of the biological activities comprising receptor binding, activation of the receptor, exhibition of beneficial effects known for GLP-1, e.g. its activity to powerfully reduce the damages caused by ischemia or oxygen shortage and potential death of heart tissue as mentioned above in connection with the effects of GLP-1 as described in the prior art, which may be compared under the same conditions for the naturally occurring GLP-1 peptide as defined herein and any fragment or variant thereof. Preferably, a variant or fragment of a GLP-1 peptide as defined herein exerts at least 25% activity of a GLP-1 (7-35, 36 or 37), more preferably at least 50% (biological) activity, even more preferably 60, 70, 80 or 90% (biological) activity and most preferably at least 95 or 99% (biological) activity of a GLP-1 (7-35, 36 or 37) as defined herein. The biological activity may be determined by a standard assay, which preferably allows to determine the activity as an incretin hormone lowering the blood glucose level, e.g. using an animal model for diabetes type 2, etc.

According to a particularly preferred embodiment, the GLP-1 peptide or a GLP-fusion peptide as defined herein, which may be as encoded by cells embedded in the (spherical) core of the (spherical) microcapsule, does not include at its N-terminus the naturally occurring amino acids 1 to 6 of a (native) GLP-1 (1-37) sequence as defined herein. Even , more preferably, the GLP-1 peptide as defined herein or a GLP-fusion peptide as defined below does not include at its N-terminus the naturally occurring amino acids 1, 2, 3, 4, 5 and/ or 6 of a native GLP-1 (1-37) sequence as defined herein. This proviso preferably refers to GLP-1 peptides as defined herein, e.g. selected from the group consisting of a peptide comprising aa 7 - 35, 36 or 37 of GLP-1, GLP-1 (7-36)amide and a peptide showing an identity of at least 80 %, 90 %, 95 % or even 99 % with any of these peptides, including modified peptides, and to fusion GLP-1 fusion peptides containing such GLP-1 peptides. However, this proviso does not exclude, that such a GLP-1 peptide as defined herein or a GLP-1 fusion peptide as defined herein, comprises an N-terminal (or C-terminal) sequence modification or additional amino acids or peptides fused thereto, e.g. signal peptide sequences and/or leader peptide sequences, etc., however being distinct from the sequence of amino acids 1 to 6 of wt GLP-1. In another preferred embodiment, any amino acid attached to the N-terminus of GLP-1 (7 - 35, 36 or 37) of homologs thereof does not correspond to the naturally occurring amino acid at position 6 of GLP-1(7 - 35, 36 or 37). According to a further preferred embodiment, any amino acid (directly) attached to the N-terminus of GLP-1 (7 - 35, 36 or 37) of homologs thereof does not correspond to the naturally occurring amino acid 6, to the naturally occurring amino acids 5 and 6, to the naturally occurring amino acids 4, 5 and 6, to the naturally occurring amino acids 3, 4, 5, and 6, to the naturally occurring amino acids 2, 3, 4, 5, and 6 or to the naturally occurring amino acids 1, 2, 3, 4, 5, and 6 of native GLP-1, preferably in their native order in GLP-1. According to a particularly preferred embodiment, any amino acid attached to the N-terminus of GLP-1 (7 - 35, 36 or 37) of homologs thereof does not correspond to the sequence of preproglucagon.

Native GLP-1, particularly GLP-1 (7-36), suffers from a short half life *in vivo* and therefore is of limited use in therapeutic treatments in general, where a frequent administration is strictly to be avoided or where a long-term administration is envisaged. GLP-1 is rapidly degraded in plasma within minutes by DPP-IV (dipeptidyl peptidase IV) between residues 8 and 9, resulting in an inactive NH₂-terminally truncated metabolite GLP-1 (9-36). Additionally, native GLP-1 typically undergoes renal excretion. These factors raise the issue, as to which peptide, GLP-1 (7-36) or the NH₂-terminally truncated metabolite GLP-1 (9-36), is the active moiety *in vivo* and as to whether physiological effects are exerted in therapeutic applications by the native GLP-1 or its fragments. As a consequence and due to its rapid degradation *in vivo,* native GLP-1 or its fragments may be used as a suitable tool for a short-term metabolic control, such as intensive care units potentially useful in patients with acute eye diseases.

To avoid such fast degradation, various attempts have been made to synthesize stabilized (against degradation by DPP-IV) analogues of naturally occurring GLP-1 (e.g. GLP-1 (7-37)).

In particular, the 8^{th} residue, which *in vivo* is Ala, was replaced by another residue, for instance, Gly, Ser or Thr (Burcelin, R. et al. (1999) Metabolism 48, 252-258). The Gly8 or G8 analogue has been extensively tested, both as synthesized molecule, and produced by cell lines genetically engineered to secrete the mutant polypeptide (Burcelin, R., et al. (1999), Annals of the New York Academy of Sciences 875: 277-285). Various other modifications have been introduced into e.g. GLP-1(7-37) to enhance its *in vivo* stability without compromising its biological activity.

Such an approach circumvents the problem of short half life by stabilization of GLP-1 against degradation by DPP-IV, e.g. by additionally administering a DPP-IV inhibitor with the GLP-1 peptide. Additionally administering a DPP-IV inhibitor with the GLP-1 peptide is complicated and typically does not lead to the desired long-term treatment as the DPP-IV inhibitor may only be used efficiently in *in vitro* systems. Furthermore, the patient in need of GLP-1 still has to receive one or multiple doses of GLP-1 or its analogs or variants during a long period of time, i.e. as long as he or she suffers from the disease to be treated, or even worse, for a whole life span. It implies that repeated administrations of the drug become necessary. The administration into the eye can be, however, painful and harbour the risk of injection related intraocular infections or mechanical lesions of intraocular structures. In addition, the relatively short half life of GLP-1 *in vivo* requires re-applications of the drug in short intervals. Since it is an invasive procedure, the doses of GLP-1 have to be administered by a medical doctor. They cannot be administered by the patient himself, since the risk of a lesion of the lens (leading to cataract) or retina (leading to retinal detachment) would be unacceptably high. Furthermore, the repeated intraocular application of drugs may also be associated with the cumulative risk of iatrogenic lesions to the lens and retina, and with the cumulative risk of infections, when administration is repeated often. It is therefore highly desirable to develop procedures and techniques which markedly decrease the number of necessary re-applications.

Therefore, according to an alternative embodiment, the anti-angiogenic, neuroprotective factor and/or any other protein or protein-like substance suitable for (intraocular) treatment of eye diseases, or a fragment or variant thereof as defined herein may be selected from a GLP-1 fusion peptide. The GLP-1 fusion peptide as used herein may be encoded and secreted by cells embedded in the (spherical) core of the (spherical) microcapsule as defined herein. In this context, cells embedded in the (spherical) core of the (spherical) microcapsule, as defined herein, are typically transfected prior to preparing the core with nucleic acid sequences encoding the GLP-1 fusion peptide such that these cells encode, express and secrete the GLP-1 fusion peptide.

The GLP-1 fusion peptides as defined herein have at least two components, e.g. components (I) and (II), components (I) and (III) or components (I), (II) and (III), exhibit GLP-1's biological activity as defined herein and, simultaneously, confer stability to component (I) of GLP-1 fusion peptides typically by a C-terminal elongation. Component (I) of GLP-1 fusion peptides as defined herein typically contains a sequence of a GLP-1 peptide as defined above, preferably a sequence having at least 80 %, more preferably at least 85 % and even more preferably at least 90 % sequence identity with SEQ ID NO: 1. SEQ ID NO: 1 represents the native ami no acid sequence of GLP-1(7-37) (length of 31 ami no acids), which is strictly conserved among mammalians. According to a particularly preferred embodiment, component (I) of GLP-1 fusion peptides as defined herein contains a sequence being identical to SEQ ID NO: 1 or a sequence, which lacks amino acids 36 and/or 37 of SEQ ID NO: 1.

Component (II) of the GLP-1 fusion peptide, which may be encoded and secreted by cells embedded in the (spherical) core of the (spherical) microcapsule as defined herein, (or more generally any GLP-1 peptide including fragments or variants of fusion peptides) typically contains a peptide sequence having at least nine amino acids. The GLP-1 fusion peptide may typically have in its component (II) sequence length of 9 to 30, preferably 9 to 20, and most preferably 9 to 15 amino acids. Generally spoken, shorter sequences in component (II) may be preferred due to their superior binding activity to the GLP receptor over longer sequences. The sequence of component (II), even though it is not a prerequisite, may preferably be neutral or may have a negative charge at pH7. Component (II) of the GLP-1 fusion peptide furthermore may contain at least one proline residue in its sequence. Proline residues are common amino acids within a -turn forming tetrameric amino acid sequence. Thus, component (II) of the GLP-1 fusion peptide may form a β-turn like structure. A β-turn structure is a typical secondary structure element of proteins or peptides. It is typically formed by a stretch of four amino acids, which reverts the direction of the peptide's or protein's backbone chain direction. If present in the GLP-1 fusion peptide, the proline residue is commonly located at position 2 or 3, preferably at position 2, of a tetrameric β-turn sequence motif occurring in component (II) of the GLP-1 fusion peptide.

Component (II) of the GLP-1 fusion peptide, which may be encoded and secreted by cells embedded in the (spherical) core of the (spherical) microcapsule as defined herein, (or more generally any GLP-1 peptide including fragments or variants of fusion peptides) may contain a sequence motif selected from the group consisting of VAIA, IAEE, PEEV, AEEV, EELG, AAAA, AAVA, AALG, DFPE, AADX, AXDX, and XADX, wherein X represents any amino acid (naturally occurring or a modified non-natural amino acid). These tetrameric motifs may be located anywhere in the sequence of component (II). In a particularly preferred embodiment, the inventive fusion peptide component (II) is a peptide sequence being linked to the C-terminus of component (I) by its N-terminal sequence motif selected from the group consisting of AA, XA, AX, RR, RX, and XR, wherein X represents any amino acid (naturally occurring or a modified non-natural amino acid).

Particularly preferred as component (II) of a GLP-1 fusion peptide, which may be encoded and secreted by cells embedded in the (spherical) core of the (spherical) microcapsule as defined herein, is a peptide sequence containing a sequence according to SEQ ID NO: 48: X₁X₁DFPX₂X₂X₃X₄, corresponding to a partial sequence of human or murine IP-2, wherein each X₁ is typically selected independently upon each other from any naturally occurring amino acid, preferably arginine (R) or alanine (A), more preferably alanine (A), or may be absent; wherein each X₂ is typically selected independently upon each other from aspartic acid (D) or glutamic acid (E), and wherein each X₃ and X₄ is typically selected independently upon each other from any naturally occurring amino acid, preferably alanine (A), glycine (G), isoleucine (I), leucine (L), threonine (T), or valine (V). X₄ also may be absent.

Even more preferred as component (II) of a GLP-1 fusion peptide, which may be encoded and secreted by cells embedded in the (spherical) core of the (spherical) microcapsule as defined herein, is a peptide sequence containing a sequence according to SEQ ID NO: 22 (RRDFPEEVAI), SEQ ID NO: 27 (DFPEEVAI), SEQ ID NO: 28 (RDFPEEVA), or SEQ ID NO: 29 (RRDFPEEV), SEQ ID NO: 30 (AADFPEEVAI), SEQ ID NO: 31 (ADFPEEVA), or SEQ ID NO: 32 (AADFPEEV), (all peptide sequences given in the one-letter-code) or a sequence having at least 80% sequence identity with SEQ ID NO: 22, 27, 28, 29, 30, 31 or 32. SEQ ID NO: 22 is a partial sequence of the full-length IP-2 (intervening peptide 2) sequence, which contains the 10 N-terminal amino acids of the 15 amino acid long full-length IP-2 sequence. IP-2 is a preferred example of a component (II) as used herein. Accordingly, other stronger preferred sequences being contained in component (II) of the herein defined GLP-1 fusion peptide are longer partial amino acid sequences of IP-2, such as the 14 N-terminal amino acid sequence occurring in humans (SEQ ID NO: 23 (RRDFPEEVAIVEEL)) or its murine counterpart (SEQ ID NO: 24 (RRDFPEEVAIAEEL)), or sequences (SEQ ID NO: 33 (AADFPEEVAIVEEL)) or (SEQ ID NO: 34 (AADFPEEVAIAEEL)), or a sequence having at least 80% sequence identity with SEQ ID NOs: 23, 24, 33 or 34. Most preferred as elements being contained in component (II) of the GLP-1 fusion peptide are full-length IP-2 sequences having all 15 amino acids of the naturally occurring IP-2 sequence (SEQ ID NO: 2 (RRDFPEEVAIVEELG), human, or SEQ ID NO: 3 (RRDFPEEVAIAEELG), murine, or SEQ ID NO: 35 (AADFPEEVAIVEELG), or SEQ ID NO: 36 (AADFPEEVAIAEELG)) or a sequence having at least 80% sequence identity with SEQ ID NOs: 2, 3, 35 or 36. Within the scope of the present invention are also all mammalian isoforms of IP2 (natural variants of IP2 among mammalians). More than one copy of a sequence being included into component (II) may be provided, e.g. 2, 3 or even more copies of IP2 or a fragment or variant of IP2.

Accordingly, a GLP-1 fusion peptide, encoded and secreted by cells embedded in the (spherical) core of the (spherical) microcapsule, as defined herein, preferably contains, comprises or consists of sequences according to SEQ ID NO: 8 (HAEGTFTSDVSSYLEGQAAKEFIAWLVKGRGRRDFPEEVAIAEELG), i.e. GLP-1 (7-37) linked without any linker sequence *via* its C-terminus to murine IP2 or according to SEQ ID NO: 12 (HAEGTFTSDVSSYLEGQAAKEFIAWLVKGRGRRDFPEEVAIVEELG), i.e. GLP-1 (7-37) linked without any linker sequence *via* its C-terminus to human IP2, or sequences according to SEQ ID NO: 37 (HAEGTFTSDVSSYLEGQAAKEFIAWLVKGRGAADFPEEVAIAEELG), i.e. GLP-1(7-37) linked without any linker sequence *via* its C-terminus to IP2 or according to SEQ ID NO: 38 (HAEGTFTSDVSSYLEGQAAKEFIAWLVKGRGAADFPEEVAIVEELG), i.e. GLP-1 (7-37) linked without any linker sequence *via* its C-terminus to IP2, or a sequence SEQ ID NO: 39 (HAEGTFTSDVSSYLEGQAAKEFIAWLVKGRGRRDFAEEVAIAEELG), SEQ ID NO: 40 (HAEGTFTSDVSSYLEGQAAKEFIAWLVKGRGRRDAAAAVAIAEELG), SEQ ID NO: 41 (HAEGTFTSDVSSYLEGQAAKEFIAWLVKGRGAADAAAAVAIAAALG), SEQ ID NO.: 42 (HAEGTFTSDVSSYLEGQAAKEFIAWLVKGRGRRDFP), SEQ ID NO: 43 (HAEGTFTSDVSSYLEGQAAKEFIAWLVKGRGRRDFPEEVA), SEQ ID NO: 44 (HAEGTFTSDVSSYLEGQAAKEFIAWLVKGRGRRDFPEEVAIAEELGRRHAC), SEQ ID NO: 45 (HAEGTFTSDVSSYLEGQAAKEFIAWLVKGRGRRDFAEEVAIVEELG), SEQ ID NO: 46 (HAEGTFTSDVSSYLEGQAAKEFIAWLVKGRGRRDAAAAVAIVEELG), SEQ ID NO: 47 (HAEGTFTSDVSSYLEGQAAKEFIAWLVKGRGAADAAAAVAIVAALG), or SEQ ID NO: 48 (HAEGTFTSDVSSYLEGQAAKEFIAWLVKGRGRRDFPEEVAIVEELGRRHAC), i.e. GLP-1 (7-37) linked without any linker sequence via its C-terminus to specific analogs or variants of the IP2 sequence. Variants or fragments thereof having a sequence identity of at least 80 % with SEQ ID NOs: 8, 12, and 37 to 48, or fragments or variants thereof may be used herein as well. Preferred GLP1-fusion peptides in this context may further comprise sequences according to SEQ ID NOs: 13, 14, 19 and 20.

Without being bound to any theory, it is concluded by the inventors of the present invention that the instability of GLP-1 (7-35, 36 or 37), e.g. if secreted *in vivo* into the patients surrounding tissue by cells embedded in the (spherical) core of the implanted (spherical) microcapsule used according to the present invention, is due to its unprotected 3-dimensional structure. Proteases may cleave the GLP-1 (7-35, 36 or 37) peptide and abolish its physiological activity rapidly *in vivo.* By linking a peptide sequence to the C-terminus of GLP-1(7-35, 36 or 37) its structure gains stability towards enzymatic degradation. Such gain in stability may be enhanced, if the additional C-terminal peptide sequence (being contained in component (II) of the fusion peptide according to the invention) folds back, e.g. due to the presence of a β-turn structural element formed by its primary structure and providing rigidity to component (II). The GLP-1 fusion peptide as defined herein, by virtue of its C-terminal peptide extension preferably containing a β-turn structural element, is found to have improved resistance to DPP-IV inactivation. The C-terminal peptide is either not cleaved from the GLP-1 (7-35, 36 or 37) sequence prior to acting on its receptor in target cells or it may be cleaved enzymatically to form GLP-1 (7-35, 36 or 37) *in vivo.* Irrespective of the exact form of the GLP-1 peptide bound at the site of the GLP-1 receptor, a GLP-1 peptide as defined herein exerts its function as an active neuroprotective compound. GLP-1 peptide sequences, which are considered to be suitable for component (II) of a GLP-1 fusion peptide as defined herein due to a primary structure forming a β-turn element, may readily be identified by adequate, e.g., spectroscopic methods, e.g. circular dichroism, or other methods known to the skilled person.

Component (II) and component (I) of a GLP-1 fusion peptide, which may be encoded and secreted by cells embedded in the (spherical) core of the (spherical) microcapsule as defined herein, may be directly linked or linked *via* a linker sequence. Preferably, both components are directly linked with each other. In case they are linked *via* a linker (or spacer), the linker is preferably a peptide linker. The peptide linker typically has a length of 1 to 10 amino acids, preferably 1 to 5, even more preferably 1 to 3 amino acids, in some cases the linker sequence may be even longer comprising 11 to 50 amino acids. The peptide linker may be composed of various (naturally occurring) amino acid sequences. Preferably, the peptide linker will introduce some structural flexibility between components to be linked. Structural flexibility is achieved e.g. by having a peptide linker containing various glycine or proline residues, preferably at least 30%, more preferably at least 40% and even more preferably at least 60 % proline and glycine residues within the linker sequence. Irrespective of the specific sequence the peptide linker may preferably be immunologically inactive.

GLP-1 fusion peptides, which may be encoded and secreted by cells embedded in the (spherical) core of the (spherical) microcapsule as defined herein, may additionally contain a component (III). Generally, component (III) comprises at least four amino acid residues, preferably at least 10 additional amino acid residues, more preferably at least 20, or most preferably at least 30. In functional terms, component (III) is intended to further enhance the stability of a GLP-1 peptide as defined herein. Component (III) is expected not to interfere with the biological function of the GLP-1 fusion peptide, which is approximately comparable to the biological activity of GLP-1 (7-37). Generally spoken, any C-terminal elongation of component (I) as defined herein, whether it is component (II), component (III) or a combination of components (II) and (III) as defined herein, enhances stability of component (I), i.e. a GLP-1 peptide as defined above, e.g. GLP-1(7-35, 36 or 37), or its fragments or variants as defined herein.

Preferably, component (III) of the GLP-1 fusion peptide as defined herein, comprises at least 4, preferably at least 10, more preferably at least 20 additional amino acid residues of the N-terminal sequence of an isoform of GLP-2 of any mammalian organism (other naturally occurring variant of GLP-2 among mammalian), e.g. murine or human isoforms as shown in SEQ ID NOs: 4 and 5. GLP-2 occurs in pro-glucagon and is also involved in carbohydrate metabolism. In the context of the present invention, the term "GLP-2 peptide" preferably means GLP-2 (1-33, 34, or 35), whereas "modified GLP-2 peptide" is intended to mean any GLP-2 fragment or variant, or a fragment or variant of GLP-2(1-33, 34 or 35). Variants or fragments are categorized as modifications of the unmodified sequence, e.g. GLP-2(1-33, 34 or 35). As with the biologically active sequence included in component (I) (GLP-1 peptide), component (III) may also comprise variants or fragments of naturally occurring forms of GLP-2. Alternatively, component (III) may also comprise at least 4, preferably at least 10, more preferably at least 20 additional amino acid residues of the (N-terminal) sequence of GLP-1 (7-37), correspondingly including all mammalian isoforms or - as disclosed herein - all functional fragments or variants thereof. Generally speaking, component (III) may contain any form of a GLP-1 peptide or a modified GLP-1 peptide, which is disclosed herein as suitable for component (I) of the GLP-1 fusion peptide. In a further alternative, component (III) may also contain chimeric forms of GLP-1 (7-37) and GLP-2. A chimeric form may be produced by coupling GLP-1 (7-37) and GLP-2 (or fragments or variants) with each other and by subsequently introducing this chimeric form as component (III) into the GLP-1 fusion peptide. Preferably, the chimeric form is composed of a partial sequence of GLP-1 (7-37) and a partial sequence of GLP-2 linked together. E.g. the chimeric form may include the N-terminal 5 to 30 amino acids of GLP-1 and the C-terminal 5 to 30 amino acids of GLP-2 or vice versa, e.g. amino acids 7 or 8 to 22, 23, 24, 25, 26, 27, or 28 of GLP-1(7-37) and amino acid sequence from position 15, 16, 17, 18, 19, 20, 21, 22, 23 or 24 to e.g. the C-terminus of GLP-2. If modifications of naturally occurring forms of GLP-2 or GLP-1 (7-37), respectively, are contained as component (III), component (III) preferably contains the sequence of SEQ ID NOs: 1, 4 or 5, respectively, or a sequence having at least 80% sequence identity with any of SEQ ID NOs: 1, 4 or 5.

In another embodiment, component (III) of the GLP-1 fusion peptide, which may be encoded and secreted by cells embedded in the (spherical) core of the (spherical) microcapsule as defined herein, may contain a plurality of sequences as described above for components (I), (II) or (III). E.g. component (III) may contain at least two, preferably 2, 3, or 4 copies of GLP-1(7-37) and/or GLP-2 or at least two copies of sequences having at least 80% sequence identity with SEQ ID NOs: 1, 4 or 5. Also, component (III) may contain more than one copy of a chimeric version of GLP-1(7-37) or GLP-2, as disclosed above, e.g. eventually forming a combination of chimeric version(s) together with GLP-1 (7-37) and/or GLP-2 or its modifications with at least 80 % sequence identity. A GLP-1 fusion peptide, which may be encoded and secreted by cells embedded in the (spherical) core of the (spherical) microcapsule as defined herein may also comprise two or more, preferably two, components (III), which may e.g. be (1) linked by its N-terminus to the C-terminus of component (I) or (II) and (2) linked by its C-terminus to the N-terminus of component (I) *via* a linker or directly. If two components (III) are provided, these may be identical or different.

According to a preferred embodiment, a GLP-1 fusion peptide, encoded and secreted by cells embedded in the (spherical) core of the (spherical) microcapsule as defined herein, may comprise the above components (I), (II) and (III). Specific embodiments containing all of these components are preferably selected from a group consisting of: SEQ ID NO: 6 (N-GLP-1(7-37)-IP2(murine)-RR-GLP-1(7-37)-C, also designated murine CM1 herein), SEQ ID NO: 7 (N-GLP-1(7-37)-IP2(murine)-RR-GLP2-C, also designated murine CM2 herein), SEQ ID NO: 10 (N-GLP-1(7-37)-IP2(human)-RR-GLP-1(7-37)-C, also designated human CM1), and SEQ ID NO: 11 (N-GLP-1 (7-37)-IP2(human)-RR-GLP-2-C), also designated human CM2 herein) or a sequence having at least 80% sequence identity with SEQ ID NOs: 6, 7, 10, or 11 or a fragment or variant thereof. In the afore-mentioned sequences the terms "N" and "C" indicate N- and the C-terminus of these fusion peptides. All sequences according to SEQ ID NOs: 6, 7, 10 and 11 contain an RR-Linker (two arginine residues) at the C-terminus of IP2 (component (II)), which may alternatively also be discarded. Component (I) in each of the embodiments according to SEQ ID NOs: 6, 7, 10 or 11 is GLP-1 (7-37), whereas component (III) (in each of these embodiments linked to the C-terminus of component (II)) is either GLP-1(7-37) or GLP-2. Preferred GLP1-fusion peptides in this context may further comprise sequences according to SEQ ID NOs: 15, 16, 17, 18 and 26.

In another preferred embodiment of the present invention, a GLP-1 fusion peptide, which may be encoded and secreted by cells embedded in the (spherical) core of the (spherical) microcapsule, as defined herein, contains in addition to component (I) a component (III) (without any component (II) as defined herein) which is either linked to the C-terminus of component (I) and/or to the N-terminus of component (I). Preferably, component (III) is located at the C-terminus of component (I). Irrespective of whether component (III) is linked to the N-terminus of component (I) (by its C-terminus) or to the C-terminus of component (I) (by its N-terminus), the coupling may be direct or indirect *via* a linker sequence. With regard to the linker sequence it is referred to the above disclosure of GLP-1 fusion peptides for a linker connecting component (I) and component (II) of the GLP-1 fusion peptide.

In an alternative preferred embodiment of the present invention, a GLP-1 fusion peptide, which may be encoded and secreted by cells embedded in the (spherical) core of the (spherical) microcapsule, as defined herein, contains in addition to components (I) and (II) a component (III) which is either linked to the C-terminus of component (II) and/or to the N-terminus of component (I). Preferably, component (III) is located at the C-terminus of component (II). Irrespective of whether component (III) is linked to the N-terminus of component (I) (by its C-terminus) or to the C-terminus of component (II) (by its N-terminus), the coupling may be direct or indirect *via* a linker sequence. With regard to the linker sequence it is again referred to the above disclosure of GLP-1 fusion peptides for a linker connecting component (I) and component (II) of the GLP-1 fusion peptide.

The GLP-1 fusion peptide, which may be encoded and secreted by cells embedded in the (spherical) core of the (spherical) microcapsule, as used according to the present invention, may furthermore comprise in addition to any of the afore mentioned combinations of components of the fusion protein (i.e. components (I) and (II), components (I) and (III) or components (I), (II) and (III)) a carrier protein, in particular transferrin or albumin, as component (IV). Such a component (IV) may be linked to the N- and/or C-terminus of any of the afore mentioned combinations of components of the GLP-1 fusion protein, i.e. components (I) and/or (II), components (I) and/or (III) or components (I), (II) and/or (III), either directly or using a linker as defined herein.

In a specific embodiment of the invention, the GLP-1 (fusion) peptide as defined herein, which may be encoded and secreted by cells embedded in the (spherical) core of the (spherical) microcapsules as used herein, contains as component (I) and/or (III) a modified GLP-1 peptide comprising the amino acid sequence of the following formula II:
Xaa7-Xaa8-Glu-Gly-Thr-Phe-Thr-Ser-Asp-Xaa16-Ser-Xaa18-Xaa19-Xaa20-Glu-Xaa22-Xaa23-Ala-Xaa25-Xaa26-Xaa27-Phe-lle-Xaa30-Trp-Leu-Xaa33-Xaa34-Xaa35-Xaa36-Xaa37,
wherein Xaa7 is L-histidine; Xaa8 is Ala, Gly, Val, Leu, Ile, or Lys, whereby Gly is particularly preferred; Xaa16 is Val or Leu; Xaa18 is Ser, Lys or Arg; Xaa19 is Tyr or Gln; Xaa20 is Leu or Met; Xaa22 is Gly or Glu; Xaa23 is Gln, Glu, Lys or Arg; Xaa25 is Ala or Val; Xaa26 is Lys, Glu or Arg; Xaa27 is Glu or Leu; Xaa30 is Ala, Glu or Arg; Xaa33 is Val or Lys; Xaa34 is Lys, Glu, Asn or Arg; Xaa35 is Gly; Xaa36 is Arg, Gly or Lys or amide or absent; Xaa37 is Gly, Ala, Glu, Pro, Lys, amide or is absent, wherein these amino acids are preferably selected if the GLP-1 (fusion) peptide as defined herein is encoded and secreted by cells embedded in the (spherical) core of the (spherical) microcapsules as used herein, to be administrated to a patient in need thereof, when treating an eye disease as defined herein or wherein Xaa7 is L-histidine, D-histidine, desamino-histidine, 2-amino-histidine, 3-hydroxy-histidine, homohistidine, N-acetyl-histidine, α-fluoromethyl-histidine, α-methylhistidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid, whereby Gly is particularly preferred; Xaa16 is Val or Leu; Xaa18 is Ser, Lys or Arg; Xaa19 is Tyr or Gln; Xaa20 is Leu or Met; Xaa22 is Gly, Glu or Aib; Xaa23 is Gln, Glu, Lys or Arg; Xaa25 is Ala or Val; Xaa26 is Lys, Glu or Arg; Xaa27 is Glu or Leu; Xaa30 is Ala, Glu or Arg; Xaa33 is Val or Lys; Xaa34 is Lys, Glu, Asn or Arg; Xaa35 is Gly or Aib; Xaa36 is Arg, Gly or Lys or amide or absent; Xaa37 is Gly, Ala, Glu, Pro, Lys, amide or is absent, wherein these amino acids are preferably selected if the GLP-1 (fusion) peptide as defined herein is provided directly to a patient in need thereof, when treating an eye disease as defined herein.

In still another specific embodiment of the invention component (I) and/or (III) of the GLP-1 (fusion) peptide as defined herein and as encoded and secreted by cells embedded in the (spherical) core of the (spherical) microcapsules as used herein contains a modified GLP-1 peptide comprising the amino acid sequence of the following formula III:
Xaa7-Xaa8-Glu-Gly-Thr-Phe-Thr-Ser-Asp-Val-Ser-Xaa18-Tyr-Leu-Glu-Xaa22-Xaa23-Ala-Ala-Xaa26-Glu-Phe-lle-Xaa30-Trp-Leu-Val-Xaa34-Xaa35-Xaa36-Xaa37,
wherein Xaa7 is L-histidine; Xaa8 is Ala, Gly, Val, Leu, Ile, Lys,; Xaa18 is Ser, Lys or Arg; Xaa22 is Gly or Glu; Xaa23 is Gln, Glu, Lys or Arg; Xaa26 is Lys, Glu or Arg; Xaa30 is Ala, Glu or Arg; Xaa34 is Lys, Glu or Arg; Xaa35 is Gly; Xaa36 is Arg or Lys, amide or is absent; Xaa37 is Gly, Ala, Glu or Lys, amide or is absent, wherein these amino acids are preferably selected if the GLP-1 (fusion) peptide as defined herein is encoded and secreted by cells embedded in the (spherical) core of the (spherical) microcapsules as used herein, to be administrated to a patient in need thereof, when treating an eye disease as defined herein, or wherein Xaa7 is L-histidine, D-histidine, desamino-histidine, 2-amino-histidine, - hydroxy-histidine, homohistidine, N-acetyl-histidine, α-fluoromethyl-histidine, α-methylhistidine, 3-pyridylalanine, 2-pyridylalanine or 4-pyridylalanine; Xaa8 is Ala, Gly, Val, Leu, Ile, Lys, Aib, (1-aminocyclopropyl) carboxylic acid, (1-aminocyclobutyl) carboxylic acid, (1-aminocyclopentyl) carboxylic acid, (1-aminocyclohexyl) carboxylic acid, (1-aminocycloheptyl) carboxylic acid, or (1-aminocyclooctyl) carboxylic acid; Xaa18 is Ser, Lys or Arg; Xaa22 is Gly, Glu or Aib; Xaa23 is Gln, Glu, Lys or Arg; Xaa26 is Lys, Glu or Arg; Xaa30 is Ala, Glu or Arg; Xaa34 is Lys, Glu or Arg; Xaa35 is Gly or Aib; Xaa36 is Arg or Lys, amide or is absent; Xaa37 is Gly, Ala, Glu or Lys, amide or is absent, wherein these amino acids are preferably selected if the GLP-1 (fusion) peptide as defined herein is provided directly to a patient in need thereof, when treating an eye disease as defined herein.

In a particular preferred embodiment a GLP-1 (fusion) peptide is used, which may be encoded and secreted by cells embedded in the (spherical) core of the (spherical) microcapsule as used herein, wherein component (I) and/or (III) contain a (modified) GLP-1 peptide, which is selected from GLP-1 (7-35), GLP-1 (7-36), GLP-1 (7-36)-amide, GLP-1 (7-37) or a variant, analogue or derivative thereof. Also preferred are GLP-1 (fusion) peptides comprising in their components (I) and/or (III) a modified GLP-1 peptide having a Aib residue in position 8 or an amino acid residue in position 7 of said GLP-1 peptide, which is selected from the group consisting of D-histidine, desamino-histidine, 2-amino-histidine, hydroxy-histidine, homohistidine, N-acetyl-histidine, α-fluoromethyl-histidine, α-methylhistidine, 3-pyridylalanine, 2-pyridylalanine and 4-pyridylalanine, preferably if the GLP-1 (fusion) peptide as defined herein is provided directly to a patient in need thereof, when treating an eye disease as defined herein.

In another particular preferred embodiment a GLP-1 (fusion) peptide is used, which may be encoded and secreted by cells embedded in the (spherical) core of the (spherical) microcapsule as used herein, wherein both embodiments of components (I) and/or (III) of the GLP-1 (fusion) peptide as defined herein by formulae II and III may be combined with the disclosure given above for GLP-1 (fusion) peptide. In other words, general formulae II and III may be combined e.g. with the disclosure given above for component (II), linkers, process of manufacturing, etc.

A GLP-1 peptide or a GLP-1 fusion peptide as defined herein, preferably component (I) of the GLP-1 fusion peptide as defined above, as well as their fragments and variants are preferably protected against proteolytic cleavage as outlined above, more preferably against DPP-IV. Accordingly, such a GLP-1 peptide or a GLP-1 fusion peptide as defined herein as well as their fragments and variants, particularly GLP-1 fusion peptides, may contain a sequence of GLP-1, e.g. GLP-1(7-35, 36 or 37) (in case of GLP-1 fusion peptides as part of component (I) and/or (III)), resistant to the DPP-IV. In this context, resistance of a peptide to degradation by dipeptidyl aminopeptidase IV may be determined e.g. by the following degradation assay: Aliquots of the peptides are incubated at 37°C with an aliquot of purified dipeptidyl aminopeptidase IV for 4-22 hours in an appropriate buffer at pH 7-8 (buffer not being albumin). Enzymatic reactions are terminated by the addition of trifluoroacetic acid, and the peptide degradation products are separated and quantified using HPLC or LC-MS analysis. One method for performing this analysis is: The mixtures are applied onto a Zorbax300SB-C18 (30 nm pores, 5 µm particles) 150 x 2.1 mm column and eluted at a flow rate of 0.5 ml/min with a linear gradient of acetonitrile in 0.1% trifluoroacetic acid (0%-100% acetonitrile over 30 min). Peptides and their degradation products may be monitored by their absorbance at 214 nm (peptide bonds) or 280 nm (aromatic amino acids), and are quantified by integration of their peak areas. The degradation pattern can be determined by using LC-MS where MS spectra of the separated peak can be determined. Percentage intact/degraded compound at a given time is used for estimation of the peptides DPP-IV stability.

In the above context, a GLP-1 peptide or a GLP-1 fusion peptide as defined herein, preferably component (I) of a GLP-1 fusion peptide as defined above, as well as a fragment and/or variant thereof, is defined as DPP-IV stabilized when it is 10 times more stable than the non-modified peptide sequence of GLP-1 (7-37) based on percentage intact compound at a given time. Thus, a DPP-IV stabilized GLP-1 peptide or GLP-1 fusion peptide, preferably component (I) of the GLP-1 fusion peptide as defined above, is preferably at least 10, more preferably at least 20 times more stable than e.g. GLP-1 (7-37). Stability may be assessed by any method known to the skilled person, e.g. by adding DPP-IV to a solution of the peptide to be tested and by determining the degradation of the peptide (see above), e.g. over a period of time, by e.g. a spectroscopic method, Western-Blot analysis, antibody screening etc.

In parallel, a GLP-1 peptide or GLP-1 fusion peptide, preferably component (I) of a GLP-1 fusion peptide as defined above, as well as a fragment and/or variant thereof is defined as a compound, which exerts the effect of GLP-1 (7-37) by e.g. binding to its native receptor (GLP-1 receptor). Preferably, a GLP-1 (fusion) peptide or a GLP-1 fusion peptide, as well as a fragment and/or variant thereof as defined herein has a binding affinity to the GLP-1 receptor, which corresponds to at least 10%, preferably at least 50% of the binding affinity of the naturally occurring GLP-1 peptide. The binding affinity may be determined by any suitable method, e.g. surface plasmon resonance, etc. Moreover, it is preferred, if the GLP-1 (fusion) peptide or GLP-1 fusion peptide, as well as a fragment and/or variant thereof as defined herein, evokes formation of intracellular cAMP by its binding to its extracellular receptor, which transmits the signal into the cell.

According to another preferred embodiment, the anti-angiogenic, neuroprotective factor and/or any other protein or protein-like substance suitable for (intraocular) treatment of eye diseases as defined herein, or the single components of the GLP-1 fusion peptide, particularly components (I), (II) and (III), and/or the entire GLP-1 fusion peptide as described above, may be selected from modified forms of these peptides or proteins sequences. The various modified forms, particularly a modified form of the entire GLP-1 fusion peptide as described above, may be either encoded and secreted by cells embedded in the (spherical) core of the (spherical) microcapsule as used herein or may be used directly in the treatment of eye diseases. These modified forms are disclosed in the following and described in more detail and comprise e.g. fragments, variants, etc., of the above anti-angiogenic factors, neuroprotective factors and/or other protein or protein-like substance suitable for (intraocular) treatment of eye diseases or of single components of the GLP-1 fusion peptide, particularly components (I), (II) and (III), and/or the entire GLP-1 fusion peptide as described above. In this context, fragments and/or variants of these peptides or proteins may have a sequence identity to their native peptides or proteins of at least 40%, 50%, 60%, 70%, 80%, preferably at least 90%, more preferably at least 95% and most preferably at least 99% over the whole length of the native, non-modified amino acid sequence. This likewise may be applied to the respective (coding) nucleic acid sequence.

The term "sequence identity" as defined herein typically means that the sequences are compared as follows. To determine the percent identity of two amino acid sequences, the sequences can be aligned for optimal comparison purposes (e.g., gaps can be introduced in the sequence of a first amino acid sequence). The amino acids at corresponding amino acid positions can then be compared. When a position in the first sequence is occupied by the same amino acid as the corresponding position in the second sequence, then the molecules are identical at that position. The percent identity between the two sequences is a function of the number of identical positions shared by the sequences, e.g. where a particular peptide is said to have a specific percent identity to a reference polypeptide of a defined length, the percent identity is relative to the reference peptide. Thus, a peptide that is 50% identical to a reference polypeptide that is 100 amino acids long can be a 50 amino acid polypeptide that is completely identical to a 50 amino acid long portion of the reference polypeptide. It might also be a 100 amino acid long polypeptide, which is 50% identical to the reference polypeptide over its entire length. Of course, other polypeptides will meet the same criteria. Such a determination of percent identity of two sequences can be accomplished using a mathematical algorithm. A preferred, non-limiting example of a mathematical algorithm utilized for the comparison of two sequences is the algorithm of Karlin et al. (1993), PNAS USA, 90:5873-5877. Such an algorithm is incorporated into the NBLAST program, which can be used to identify sequences having the desired identity to the amino acid sequence of the invention. To obtain gapped alignments for comparison purposes, Gapped BLAST can be utilized as described in Altschul et al. (1997), Nucleic Acids Res, 25:3389-3402. When utilizing BLAST and Gapped BLAST programs, the default parameters of the respective programs (e. g., NBLAST) can be used. The sequences further may be aligned using Version 9 of the Genetic Computing Group's GAP (global alignment program), using the default (BLOSUM62) matrix (values-4 to +11) with a gap open penalty of -12 (for the first null of a gap) and a gap extension penalty of -4 (per each additional consecutive null in the gap). After alignment, percentage identity is calculated by expressing the number of matches as a percentage of the number of amino acids in the claimed sequence. The described methods of determination of the percent identity of two amino acid sequences can be applied correspondingly to nucleic acid sequences.

In the context of the present invention, a "fragment" of an anti-angiogenic factor, of a neuroprotective factor, of any other protein or protein-like substance suitable for (intraocular) treatment of eye diseases, a fragment of single components of the GLP-1 fusion peptide, particularly components (I), (II), or (III), etc., and/or a fragment of the entire GLP-1 fusion peptide as described above, typically refers to any fragment of these peptides or proteins. Typically, such a fragment comprises a shorter peptide which retains the desired biological activity particularly of the native peptide or protein, e.g. of an anti-angiogenic factor, of a neuroprotective factor, of a further protein or a protein-like substance as defined herein, of a single component (I), (II), or (III), etc., and/or of the entire GLP-1 fusion peptide as described above, which is, with regard to its amino acid sequence (or its encoded nucleic acid sequence), N-terminally, C-terminally and/or intrasequentially truncated compared to the amino acid sequence of the native peptide or protein (or its encoded nucleic acid sequence). Such truncation may thus occur either on the amino acid level or correspondingly on the nucleic acid level. Biologically functional fragments may be readily identified by removing amino acids (either on peptide or on amino acid level) from either end of the peptide molecule and testing the resultant peptide or protein for its biological properties as defined herein for e.g. an anti-angiogenic factor, a neuroprotective factor and/or any other protein or protein-like substance suitable for (intraocular) treatment of eye diseases, or a fragment or variant thereof . Alternatively, proteases for removing one or more amino acids at a time from either the N-terminal end and/or the C-terminal end of a native peptide or protein may be used to determine fragments which retain the desired biological activity. Conclusively, fragments may be due to deletions of amino acids at the peptide termini and/or of amino acids positioned within the peptide sequence.

Furthermore, a "variant" of an anti-angiogenic factor, of a neuroprotective factor, of any other protein or protein-like substance suitable for (intraocular) treatment of eye diseases, a variant of single components of the GLP-1 fusion peptide, particularly components (I), (II), or (III), etc., and/or a variant of the entire GLP-1 fusion peptide as described above, preferably comprises a protein sequence or its encoding nucleic acid sequence (or a fragment thereof), wherein amino acids of the native protein or peptide sequences are exchanged. Thereby, (a variant of) an anti-angiogenic factor, of a neuroprotective factor, of any other protein or protein-like substance suitable for (intraocular) treatment of eye diseases, (a variant of) single components of the GLP-1 fusion peptide, particularly components (I), (II), or (III), etc., and/or (a variant of) an entire GLP-1 fusion peptide as described above may be generated, having an amino acid sequence which differs from the native protein or peptide sequences in one or more mutation(s), such as one or more substituted, inserted and/or deleted amino acid(s). Preferably, these variants have about the same or an improved biological activity compared to the full-length native anti-angiogenic factor, neuroprotective factor, other protein or protein-like substance suitable for (intraocular) treatment of eye diseases, single components of the GLP-1 fusion peptide, particularly components (I), (II), or (III), etc., and/or an entire GLP-1 fusion peptide as described above. Such a variant as defined herein can be prepared by mutations in the DNA sequence which encodes the synthesized variants. Any combination of deletion, insertion, and substitution may also be contained in a variant of an anti-angiogenic factor, of a neuroprotective factor, of any other protein or protein-like substance suitable for (intraocular) treatment of eye diseases, a variant of single components of the GLP-1 fusion peptide, particularly components (I), (II), or (III), etc., and/or a variant of an entire GLP-1 fusion peptide as described above, provided that the finally obtained variant possesses the desired biological activity. Obviously, the mutations that will be made in the DNA encoding the variant peptide must not alter the reading frame and preferably will not create complementary regions that could produce secondary mRNA structure.

Accordingly, a variant of an anti-angiogenic factor, a neuroprotective factor, of any other protein or protein-like substance suitable for (intraocular) treatment of eye diseases, of single components of the GLP-1 fusion peptide, particularly of components (I), (II), or (III), etc., and/or of an entire GLP-1 fusion peptide as described above, may also contain additional amino acid residues flanking the N-/ or the C-terminus or even both termini of the amino acid sequence compared to the native anti-angiogenic factor, neuroprotective factor, other protein or protein-like substance suitable for (intraocular) treatment of eye diseases, single component of the GLP-1 fusion peptide, particularly component (I), (II), or (III), etc., and/or of an entire GLP-1 fusion peptide as described above. As an example, such a variant may comprise a GLP-1 peptide or a GLP-1 fusion peptide as defined herein containing additional amino acid residues flanking the N-/ or the C-terminus or even both termini of the amino acid sequence of the GLP-1 peptide or GLP-1 fusion peptide. As long as the resultant GLP-1 peptide or GLP-1 fusion peptide retains its resistance or stability towards proteases and its ability to act as defined herein, one can determine whether any such flanking residues affect the basic characteristics of the core peptide, e.g. by its beneficial effects known for GLP-1, by routine experimentation. The term "consisting essentially of", when referring to a specified GLP-1 peptide as defined herein, means that additional flanking residues can be present which do not affect the basic characteristic of the specified GLP-1 peptide. This term typically does not comprehend substitutions, deletions or additions within the specified sequence.

A "variant" of an anti-angiogenic factor, a neuroprotective factor, of any other protein or protein-like substance suitable for (intraocular) treatment of eye diseases, of single components of the GLP-1 fusion peptide, particularly of components (I), (II), or (III), etc., and/or of an entire GLP-1 fusion peptide as described above, may further refer to a molecule which comprises conservative amino acid substitutions compared to its native sequence. Substitutions in which amino acids which originate from the same class are exchanged for one another are called conservative substitutions. In particular, these are amino acids having aliphatic side chains, positively or negatively charged side chains, aromatic groups in the side chains or amino acids, the side chains of which can enter into hydrogen bridges, e.g. side chains which have a hydroxyl function. This means that e.g. an amino acid having a polar side chain is replaced by another amino acid having a likewise polar side chain, or, for example, an amino acid characterized by a hydrophobic side chain is substituted by another amino acid having a likewise hydrophobic side chain. Insertions and substitutions are possible, in particular, at those sequence positions which cause no modification to the three-dimensional structure or do not affect the binding region. Modifications to a three-dimensional structure by insertion(s) or deletion(s) can easily be determined e.g. using CD spectra (circular dichroism spectra) (Urry, 1985, Absorption, Circular Dichroism and ORD of Polypeptides, in: Modern Physical Methods in Biochemistry, Neuberger et al. (ed.), Elsevier, Amsterdam).

A variant of an anti-angiogenic factor, a neuroprotective factor, of any other protein or protein-like substance suitable for (intraocular) treatment of eye diseases, of single components of the GLP-1 fusion peptide, particularly of components (I), (II), or (III), etc., and/or of an entire GLP-1 fusion peptide as described above may thus also refer to a molecule which is substantially similar to either the entire anti-angiogenic factor, neuroprotective factor, other protein or protein-like substance suitable for (intraocular) treatment of eye diseases, single components of the GLP-1 fusion peptide, particularly components (I), (II), or (III), etc., and/or entire GLP-1 fusion peptide as described above or a fragment thereof. Such variant peptides may be conveniently prepared using methods well known in the art. Of course, such a variant would have similar beneficial effects known for the native anti-angiogenic factor, europrotective factor, other protein or protein-like substance suitable for (intraocular) treatment of eye diseases, single components of the GLP-1 fusion peptide, particularly of components (I), (II), or (III), etc., and/or entire GLP-1 fusion peptide as described above. Such beneficial effect is, e.g. for GLP-1, its activity to powerfully reduce the damages caused by ischemia or oxygen shortage and potential death of heart tissue as the corresponding naturally-occurring GLP-1 peptide.

The types of conservative amino acid substitutions which may be contained in a variant of the anti-angiogenic factor, neuroprotective factor, any other protein or protein-like substance suitable for (intraocular) treatment of eye diseases, single components of the GLP-1 fusion peptide, particularly of components (I), (II), or (III), etc., and/or entire GLP-1 fusion peptide as described above, may be based on analysis of the frequencies of amino acid changes between a homologous protein/peptide of different species. Based upon such analysis, conservative substitutions may be defined herein as exchanges within one of the following five groups:
I. Small, aliphatic, non-polar or slightly polar residues: Ala, Ser, Thr, Pro, Gly;
II. Polar, negatively-charged residues and their amides: Asp, Asn, Glu, Gln;
III. Polar, positively-charged residues: His, Arg, Lys;
IV. Large, aliphatic non-polar residues: Met, Leu, Ile, Val, Cys;
V. Large aromatic residues: Phe, Try, Trp.

Within the foregoing groups, the following substitutions are considered to be "highly conservative": Asp/Glu; His/Arg/Lys; Phe/Tyr/Trp; Met/Leu/lle/Val. Semi-conservative substitutions are defined to be exchanges between two of groups (I) - (IV) above which are limited to supergroup (A), comprising (I), (II), and (III) above, or to supergroup (B), comprising (IV) and (V) above. Substitutions are not limited to the genetically encoded or even the naturally-occurring amino acids. Preferred conservative amino acid substitutions of preferred groups of synonymous amino acid residues within the above meaning particularly include, without being limited thereto:

| Amino Acid | Synonymous Residue |
|---|---|
| Ser | Ser, Thr, Gly, Asn |
| Arg | Arg, Gln, Lys, Glu, His |
| Leu | Ile, Phe, Tyr, Met, Val, Leu |
| Pro | Gly, Ala, (Thr), Pro |
| Thr | Pro, Ser, Ala, Gly, His, Gln, Thr |
| Ala | Gly, Thr, Pro, Ala |
| Val | Met, Tyr, Phe, Ile, Leu, Val |
| Gly | Ala, (Thr), Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gln | Glu, Lys, Asn, His, (Thr), Arg, Gln |
| Asn | Gln, Asp, Ser, Asn |
| Lys | Glu, Gln, His, Arg, Lys |
| Asp | Glu, Asn, Asp |
| Glu | Asp, Lys, Asn, Gln, His, Arg, Glu |
| Met | Phe, Ile, Val, Leu, Met |
| Trp | Trp |

Furthermore, variants of an anti-angiogenic factor, a neuroprotective factor, of any other protein or protein-like substance suitable for (intraocular) treatment of eye diseases, of single components of the GLP-1 fusion peptide, particularly of components (I), (II), or (III), etc., and/or of an entire GLP-1 fusion peptide as described above, may also contain amino acid substitutions, made e.g. with the intention of improving solubility (replacement of hydrophobic amino acids with hydrophilic amino acids).

In one particularly preferred embodiment a GLP-1 peptide or a GLP-1 fusion peptide as defined herein, which may be encoded and secreted by a cell embedded in the (spherical) core of the (spherical) microcapsule as defined herein, includes a GLP-1 peptide (occurring in component (I) and/or (III) of the GLP-1 fusion peptide) characterized by one or more substitution(s) at positions 7, 8, 11, 12, 16, 22, 23, 24, 25, 27, 30, 33, 34, 35, 36, or 37 of the GLP-1 peptide. As an example for the following nomenclature [Arg34-GLP-1 (7-37)] designates a GLP-1 analogue wherein its naturally occurring lysine at position 34 has been substituted with arginine.

Specifically, a GLP-1 peptide or component (I) and/or (III) of a GLP-1 fusion peptide as defined herein may correspond to variants of GLP-1 (7-35, 36, 37 or 38) including, for example, Thrl6-Lysl8-GLP-1 (7-37), and Lysl8-GLP-1 (7-37), Arg34-GLP-1 (7-37), Lys38-Arg26-GLP-1 (7-38)-OH, Lys36-Arg26-GLP-1 (7-36), Arg26,34-Lys38-GLP-1 (7-38), Arg26,34-Lys38-GLP-1 (7-38), Arg26,34-Lys38-GLP-1 (7-38), Arg26,34-Lys38-GLP-1 (7-38), Arg26,34-Lys38-GLP-1 (7-38), Arg26-Lys38-GLP-1(7-38), Arg26-Lys38-GLP-1 (7-38), Arg34-Lys38-GLP-1 (7-38), Ala37-Lys38-GLP-1 (7-38), and Lys37-GLP-1 (7-37). More generally speaking, any GLP-1 variant mentioned herein (in particular according to formulae II or III) may be modified by the addition of a Lys residue at position 38.

If the GLP-1 peptide or GLP-1 fusion peptide as described above is administered directly in the treatment of eye diseases, the GLP-1 peptide or component (I) and/or (III) of a GLP-1 fusion peptide as defined herein may additionally correspond to variants of GLP-1 (7-35, 36, 37 or 38) including Gln9-GLP-1 (7-37), D-Gln9-GLP-1 (7-37), acetyl-Lys9-GLP-1 (7-37).

In a particular preferred embodiment of the invention the GLP-1 peptide or the GLP-1 fusion peptide as defined herein (with respect to component (I) or (III)) is/contains a (modified) GLP-1 peptide, which is selected from GLP-1 (7-35), GLP-1 (7-36), GLP-1 (7-36)-amide, GLP-1 (7-37) or a fragment or variant thereof.

The anti-angiogenic factor, neuroprotective factor, any other protein or protein-like substance suitable for (intraocular) treatment of eye diseases, and/or the GLP-1 fusion peptide as described above, if encoded and secreted by a cell embedded in the (spherical) core of the (spherical) microcapsule or a fragment or variant thereof, may be isolated for *in vitro* control purposes from the cells (and thus from the miocrocapsules) from which it is expressed, for instance using conventional separation techniques. Thus cells may be grown under appropriate conditions, for instance including support and nutrients, *in vitro,* and secreted protein, i.e. the anti-angiogenic factor, neuroprotective factor, any other protein or protein-like substance suitable for (intraocular) treatment of eye diseases, and/or the GLP-1 fusion peptide as described above, if encoded and secreted by a cell embedded in the (spherical) core of the (spherical) microcapsule or a fragment or variant thereof, is recovered from the extracellular medium. The (vector) sequences engineered for transfection into cells thus preferably include signal (peptide) sequences (see below) allowing secretion of the anti-angiogenic or neuroprotective factor as defined herein (see below). In this context, the anti-angiogenic factor, neuroprotective factor, any other protein or protein-like substance suitable for (intraocular) treatment of eye diseases, and/or the GLP-1 fusion peptide as described above, if encoded and secreted by a cell embedded in the (spherical) core of the (spherical) microcapsule, or a fragment or variant thereof, may be fused to a signal sequence, either naturally endogenously or after transfection of encoding nucleic acid sequences introduced into the cell by genetic engineering methods. In an alternative, the engineered gene sequences encoding these peptides do not include such signal peptide sequences, whereby the intracellularly expressed peptides will typically not be secreted, and may be recovered from cells by processes involving cell lysis. In such methods the coding sequences may include purification tags allowing efficient extraction of the product peptide from the medium; tags may be cleaved off to release isolated anti-angiogenic or neuroprotective factor as defined herein. However, this alternative is typically irrelevant to cells of a (spherical) microcapsule, as used according to the present invention, which are implanted into the patient and require delivery of an *in vivo* expressed and secreted anti-angiogenic or neuroprotective factor as defined herein into the surrounding tissue.

Any of the above described embodiments or features may be combined with each other, if not indicated otherwise.

As described above, the anti-angiogenic factor, neuroprotective factor, any other protein or protein-like substance suitable for (intraocular) treatment of eye diseases, and/or the GLP-1 fusion peptide as described above, or a fragment or variant thereof, may be encoded and secreted by cells embedded in the (spherical) core of the (spherical) microcapsule. According to a further particularly preferred embodiment, the cells embedded in the (spherical) core of the (spherical) microcapsule may be further modified or engineered to additionally secrete a factor selected from the group consisting of anti-apoptotic factors, growth factors, VEGF and erythropoietin (EPO), anti-platelet factors, anti-coagulant factors, anti-thrombotic drugs, anti-angiogenic factors, or any further factor, such as a protein or protein-like substance suitable for (intraocular) treatment of eye diseases.

According to one specific embodiment, the cells embedded in the (spherical) core of the (spherical) microcapsule encoding and secreting an anti-angiogenic factor, a neuroprotective factor and/or any other protein or protein-like substance suitable for (intraocular) treatment of eye diseases, or a fragment or variant thereof as defined herein may be engineered to additionally secrete erythropoietin (EPO). Erythropoietin (also known as EPO, epoetin or procrit) is an acidic glycoprotein hormone of approximately 34.000 Dalton molecular weight occurring in multiple forms, including alpha, beta, omega, etc. Erythropoietin stimulates red blood cell production. It is produced in the kidney and stimulates the division and differentiation of committed erythroid precursors in the bone marrow and elsewhere. Generally, erythropoietin is present in very low concentrations in plasma when the body is in a healthy state, in which tissues receive sufficient oxygenation from the existing number of erythrocytes. This normal low concentration is enough to stimulate replacement of red blood cells that are lost normally through aging. The amount of erythropoietin in the circulation is increased under conditions such as hypoxia, when oxygen transport by blood cells in the circulation is reduced. Hypoxia may be caused by loss of large amounts of blood through haemorrhage, destruction of red blood cells by overexposure to radiation, reduction in oxygen intake due to high altitudes or prolonged unconsciousness, or various forms of anaemia or ischemia. In response to tissues undergoing hypoxic stress, erythropoietin will increase red blood cell production by stimulating the conversion of primitive precursor cells in the bone marrow into proerythroblasts which subsequently mature, synthesize haemoglobin and are released into the circulation as red blood cells. When the number of red blood cells in circulation is greater than needed for normal tissue oxygen requirements, erythropoietin in circulation is decreased. Erythropoietin may be expressed by the cells embedded in the (spherical) microcapsule (or may be provided) at a concentration or for a duration that will not induce red blood cell formation or alternatively, increase the hematocrit in a subject, e.g., between about 1 pM and less than 1000 µM, including less than 900 µM, less than 700 µM, less than 500 µM, less than 300 µM, less than 100 µM, or less than 50 µM. In other embodiments, erythropoietin is administered as a function of the subject's body weight. Erythropoietin may typically be provided at a concentration of between about 1 U/kg to 10,000 U/kg of a subject's body weight, including less than 7,500 U/kg, 5,000 U/kg, 2500 U/kg, 1000 U/kg, 750 U/kg, 500 U/kg, 250 Ug/kg, 100 Ug/kg, 50 U/kg, 25 U/kg, 10 U/kg, 5 U/kg, or 1 U/kg. In this context, erythropoietin serum concentration is normally within the range of 5-50 mU/ml. For patients suffering from an eye disease or disorder as defined herein, preferably from a retinal disease as defined herein or other conditions associated thereto, erythropoietin is preferably provided either at a concentration of 50-100 U/kg depending on symptom, body weight, sex, animal species and the like. It is generally assumed that treatment options holding the blood concentration at about 1-100 mU/ml will be preferred.

According to one further specific embodiment, the cells embedded in the core of the (spherical) microcapsule encoding and secreting an anti-angiogenic factor, a neuroprotective factor and/or any other protein or protein-like substance suitable for (intraocular) treatment of eye diseases, or a fragment or variant thereof as defined herein may be engineered to additionally secrete VEGF.

According to another specific embodiment, the cells embedded in the core of the (spherical) microcapsule encoding and secreting anti-angiogenic factor, neuroprotective factor, any other protein or protein-like substance suitable for (intraocular) treatment of eye diseases, and/or the GLP-1 fusion peptide as described above, or a fragment or variant thereof, as defined herein, may be engineered to additionally secrete antiapoptotic factors. Such factors may include, without being limited thereto, APC (apoptosis repressor with caspase recruitment domain), Bcl-2, Bcl-xL, Che-1/AATF, clusterin, insulin, Mcl-1, NF-kB-dependent anti-apoptotic factors, serotonin, survivin, etc. Furthermore, any factor, which acts as an inhibitory factor to an apoptotic factor known in the art, and which may thus be regarded as antiapoptotic factors, is encompassed herewith. Such factors are preferably encoded by a nucleic acid and secreted by the cells encoding and secreting an anti-angiogenic factor, neuroprotective factor, any other protein or protein-like substance suitable for (intraocular) treatment of eye diseases, and/or the GLP-1 fusion peptide as described above, or a fragment or variant thereof. In this context, such antiapoptotic factors may be directed against at least one of the following apoptotic factors or apoptosis related proteins including AlF, Apaf e.g. Apaf-1, Apaf-2, Apaf-3, oder APO-2 (L), APO-3 (L), Apopain, Bad, Bak, Bax, Bcl-2, Bcl-x_{L}, Bcl-x_{S}, bik, CAD, Calpain, Caspase e.g. Caspase-1, Caspase-2, Caspase-3, Caspase-4, Caspase-5, Caspase-6, Caspase-7, Caspase-8, Caspase-9, Caspase-10, Caspase-11, ced-3, ced-9, c-Jun, c-Myc, crm A, cytochrom C, CdR1, DcR1, DD, DED, DISC, DNA-PK_{CS}, DR3, DR4, DR5, FADD/MORT-1, FAK, Fas (Fas-ligand CD95/fas (receptor)), FLICE/MACH, FLIP, fodrin, fos, G-Actin, Gas-2, gelsolin, granzyme A/B, ICAD, ICE, JNK, lamin A/B, MAP, MCL-1, Mdm-2, MEKK-1, MORT-1, NEDD, NF-ₖₐₚₚₐB, NuMa, p53, PAK-2, PARP, perforin, PITSLRE, PKCdelta, pRb, presenilin, prICE, RAIDD, Ras, RIP, sphingomyelinase, thymidinkinase from herpes simplex, TRADD, TRAF2, TRAIL-R1, TRAIL-R2, TRAIL-R3, transglutaminase, etc.

The cells embedded in the (spherical) core of the (spherical) microcapsule used according to the present invention, which encode and secrete an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, may furthermore encode and secrete an additional factor, such as an anti-apoptotic agent, VEGF, etc. as defined herein. For these purposes, the anti-angiogenic or neuroprotective factor as defined herein or its fragments or variants as well as further optional factors, are encoded by at least one nucleic acid sequence, which is typically transfected into the cells prior to preparation of the (spherical) core of the (spherical) microcapsule. These nucleic acid sequences may occur naturally in the cells or may be introduced into the cells by cell transfection techniques prior to the preparation of the (spherical) microcapsule. According to the present invention any suitable nucleic acid sequence may be used.

According to one embodiment, the nucleic acid sequence encoding an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, and optionally an additional factor, such as an anti-apoptotic agent, VEGF, etc. as defined herein may be selected from any nucleic acid, more preferably selected from any nucleic acid suitable to encode a(t least one) peptide or protein, i.e. a coding nucleic acid, e.g. a coding DNA, selected e.g. from genomic DNA, cDNA, DNA oligonucleotides, or a coding RNA, selected e.g. from (short) RNA oligonucleotides, messenger RNA (mRNA), etc. In the context of the present invention, an mRNA is typically an RNA, which is composed of several structural elements, e.g. an optional 5'-UTR region, an upstream positioned ribosomal binding site followed by a coding region, an optional 3'-UTR region, which may be followed by a poly-A tail (and/or a poly-C-tail). An mRNA may occur as a mono-, di-, or even multicistronic RNA, i.e. an RNA which carries the coding sequences of one, two or more proteins or peptides as described above. Such coding sequences in di-, or even multicistronic mRNA may be separated by at least one IRES sequence. The least one nucleic acid sequence may also be a ribosomal RNA (rRNA), a transfer RNA (tRNA), or a viral RNA (vRNA). Furthermore, the least one nucleic acid sequence may be a circular or linear nucleic acid, preferably a linear nucleic acid. Additionally, the at least one nucleic acid sequence may be a single- or a double-stranded nucleic acid sequence (which may also be regarded as a nucleic acid within the above meaning due to non-covalent association of two single-stranded nucleic acids) or a partially double-stranded or partially single stranded nucleic acid, which are at least partially self complementary (both of these partially double-stranded or partially single stranded nucleic acids are typically formed by a longer and a shorter single-stranded nucleic acid or by two single stranded nucleic acids, which are about equal in length, wherein one single-stranded nucleic acid is in part complementary to the other single-stranded nucleic acid and both thus form a double-stranded nucleic acid in this region, i.e. a partially double-stranded or partially single stranded nucleic acid).

Due to degeneracy of the genetic code a plurality of nucleic acid sequences may code for such an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, and optionally an additional factor, such as an anti-apoptotic agent, VEGF, etc. as defined herein. According to a preferred embodiment of the present invention a nucleic acid sequence used for transfection of cells as defined herein may comprise any nucleic acid sequence as defined above, preferably a nucleic acid sequence suitable for transfection of a cell as defined herein which may code for (a) an anti-angiogenic factor, a neuroprotective factor and/or any other protein or protein-like substance suitable for (intraocular) treatment of eye diseases, or a fragment or variant thereof, e.g. Endostatin or a GLP-1 peptide, particularly for the entire GLP-1 aa sequence (GLP-1(1-37) or functional GLP-1 (7-35, 36 or 37) (variant) sequences or any other GLP-1 peptide, including GLP-1 fusion peptides as defined herein, (b) optionally for a protease cleavage sequence at the N-terminus of the GLP-1 sequence according to (a) and, optionally, for a signal peptide sequence upstream from (b), and (c) optionally for a further factor as described above. Preferably, the signal (peptide) sequence is selected from a sequence as defined below. Accordingly, the resulting amino acid sequence may be composed of a signal peptide sequence, an optional protease cleavage sequence and the biologically effective factor, i.e. an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein(, and optionally an additional factor, such as an anti-apoptotic agent, VEGF, etc. as defined herein), (from the N- to the C-terminus). Thereby, the signal peptide sequence and the protease cleavage sequence are preferably heterologous to (the natively occurring sequences in the) host cell, and are, in case of GLP-1(5-37, 6-37, or 7-37) and variants thereof as defined above preferably different from the amino acids 1 to 6 of native GLP-1 within the definitions of the above proviso.

The nucleic acid sequence as defined herein may be contained in a vector. Accordingly, the cell embedded in the (spherical) core of the (spherical) microcapsule used according to the present invention may contain a vector comprising a nucleic acid as defined herein before. This vector may be used to transfect the cell as defined herein to prepare the (spherical) microcapsule as used according to the present invention. Typically, such a vector, in particular an expression vector, contains at least one nucleic acid sequence as defined herein, encoding elements (a) and optionally (b) and/or (c) as described above, and, if necessary, additional elements as described herein, e.g. elements suitable for directing expression of the encoded elements (a) and optionally (b) and/or (c) as described above, and optionally sequences encoding further factors, such as antiapoptotoc factors, VEGF, etc. One class of vectors as used herein utilizes DNA elements that provide autonomously replicating extrachromosomal plasmids derived from animal viruses (e.g. bovine papilloma virus, polyomavirus, adenovirus, or SV40, etc.). A second class of vectors as used herein relies upon the integration of the desired gene sequences into the host cell chromosome.

Such vectors, suitable to transfect the cell prior to embedding it in the (spherical) core of the (spherical) microcapsule used according to the present invention, are typically prepared by inserting at least one nucleic acid sequence encoding elements (a) and optionally (b) and/or (c) as described above, e.g. an anti-angiogenic factor, a neuroprotective factor and/or any other protein or protein-like substance suitable for (intraocular) treatment of eye diseases, or a fragment or variant thereof as defined herein, such as Endostatin, GLP-1 peptides, or a GLP-1 fusion peptide as defined herein, and optionally an additional factor as defined herein into suitable (empty) vectors. Such suitable (empty) vectors are known to a skilled person and may be reviewed e.g. in "Cloning Vectors" (Eds. Pouwels P. H. et al. Elsevier, Amsterdam-New York-Oxford, 1985, ISBN 0 444 904018). Suitable (empty) vectors are also intended to include any vector known to a skilled person, such as plasmids, phages, viruses such as SV40, CMV, Baculo virus, Adeno virus, Sindbis virus, transposons, IS-elements, phasmids, phagemides, cosmides, linear or circular DNA. For integration in mammalian cells linear DNA is typically used. Preferably, the vector type used for the present invention corresponds to the specific host cell requirements. Suitable commercially available expression vectors, into which the inventive nucleic acid sequences and vectors may be inserted, include pSPORT, pBluescriptllSK, the baculovirus expression vector pBlueBac, and the prokaryotic expression vector pcDNAll, all of which may be obtained from Invitrogen Corp., San Diego, CA.

A vector as defined herein suitable for transfecting a cell prior to embedding it in the (spherical) core of the (spherical) microcapsule used according to the present invention, typically combines the nucleic acid sequence as defined above with other regulatory elements, which, e.g., control expression of the encoded amino acid sequences. Such regulatory elements are e.g. 1) specific to a tissue or region of the body; 2) constitutive; 3) glucose responsive; and/or 4) inducible/regulatable. Regulatory elements herein are preferably selected from regulation sequences and origins of replication (if the vectors are replicated autonomously). Regulation sequences in the scope of the present invention are any elements known to a skilled person having an impact on expression on transcription and/or translation of the encoding nucleic acid sequences. Regulation sequences include, apart from promoter sequences so-called enhancer sequences, which may lead to an increased expression due to enhanced interaction between RNA polymerase and DNA. Further regulation sequences of inventive vectors are transcriptional regulatory and translational initiation signals, so-called "terminator sequences", etc. or partial sequences thereof.

Generally, any naturally occurring promoter may be contained in an expression vector suitable for transfecting a cell which may be used for preparing the (spherical) microcapsule as used herein. Such promoters may be selected from any eukaryotic, prokaryotic, viral, bacterial, plant, human or animal, e.g. mammalian promoters. Suitable promoters include, for example, the cytomegalovirus promoter, the lacZ promoter, the gal 10 promoter and the AcMNPV polyhedral promoter, promoters such as cos-, tac-, trp-, tet-, trp-tet-, lpp-, lac-, lpp-lac-, laclq-, T7-, T5-, T3-, gal-, trc-, ara-, SV40-, SP6, I-PR- or the I-PL-promoter, advantageously being found in gram-negative bacteria. Additionally, promoters may be obtained from gram-positive promoters such as amy and SPO2, yeast promoters, such as ADC1, MFa, AC, P-60, CYC1, GAPDH or mammalian promoters such as the cytomegalovirus (CMV) promoter, muscle-specific promoters including mammalian muscle creatine kinase (MCK) promoter, mammalian desmin promoter, mammalian troponin I (TNN12) promoter, or mammalian skeletal alpha-actin (ASKA) promoter, or liver type pyruvate kinase promoters, particularly those fragments which run (-183 to +12) or (-96 to +12) (Thompson, et al. J Biol Chem, (1991). 266:8679-82.; Cuif, et al., Mol Cell Biol, (1992). 12:4852-61); the spot 14 promoter (S14, -290 to +18) (Jump, et al., J. Biol Chem, (1990). 265:3474-8); acetyl-CoA carboxylase (O'Callaghan, et al., J. Biol Chem, (2001). 276:16033-9); fatty acid synthase (-600 to +65) (Rufo, et al., J Biol Chem, (2001). 28:28); and glucose-6-phosphatase (rat and human) (Schmoll, et al., FEBS Left, (1996). 383:63-6; Argaud, et al., Diabetes, (1996). 45:1563-71), or promoters from CaM-Kinasell, Nestin, L7, BDNF, NF, MBP, NSE, beta-globin, GFAP, GAP43, tyrosine hydroxylase, Kainat-receptor-subunit 1, glutamate-receptor-subunit B, or human ubiquitin promoter B (ubiB human), human ferritin H promoter (FerH), etc. Particularly preferred promoters are of human or mammalian origin. Finally, synthetic promoters may be used advantageously. Promoter sequences, as contained in an inventive vector, may also be inducible for *in vitro* control purposes, to allow modulation of expression (e.g. by the presence or absence of nutrients or other inducers in the growth medium). One example is the lac operon obtained from bacteriophage lambda plac5, which can be induced by IPTG. Finally, a promoter as defined herein may be linked with a nucleic acid sequence as defined above, e.g. encoding an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, and optionally an additional factor, such as an anti-apoptotic agent, VEGF, etc. as defined herein, such that the promoter is positioned 5' "upstream" of the GLP-1 encoding nucleic acid sequence. Preferably, human promoters are used, e.g. the human ubiquitin promoter B (ubiB human) or the human ferritin H promoter (FerH).

Enhancer sequences for upregulating expression of the peptides encoded by the above defined nucleic acid sequences are preferably another constituent of a vector or an expression vector as defined herein. Such enhancer sequences are typically located in the non-coding 3' region of the vector. Enhancer sequences as employed in a vector as defined herein may be obtained from any eukaryotic, prokaryotic, viral, bacterial, plant, human or animal, e.g. mammalian hosts, preferably in association with the corresponding promoters as defined herein. Enhancer elements which will be most useful in the present invention are those which are glucose responsive, insulin responsive and/or liver specific. Enhancer elements may include the CMV enhancer (e.g., linked to the ubiquitin promoter (Cubi)); one or more glucose responsive elements, including the glucose responsive element (G1 RE) of the liver pyruvate kinase (L-PK) promoter (-172 to -142); and modified versions with enhanced responsiveness (Cuif *et al.,* supra; Lou, et al., J. Biol Chem, (1999). 274:28385-94); G1RE of L-PK with auxiliary L3 box (-172 to -126) (Diaz Guerra, et al., Mol Cell Biol, (1993). 13:7725-33; modified versions of G1RE with enhanced responsiveness with the auxiliary L3 box; carbohydrate responsive element (ChoRE) of S 14 (-1448 to -1422), and modifications activated at lower glucose concentrations (Shih and Towle, J Biol Chem, (1994). 269:9380-7; Shih, et al., J Biol Chem, (1995). 270:21991-7; and Kaytor, et al., J Biol Chem, (1997). 272:7525-31; ChoRE with adjacent accessory factor site of S 14 (-1467 to - 1422) [*et al*., supra]; aldolase (+1916 to +2329)(Gregori et al., J Biol Chem, (1998). 273:25237-43; Sabourin, et al., J. Biol Chem, (1996). 271:3469-73; and fatty acid synthase (-7382 to -6970) (Rufo, *et al*., supra.), more preferably insulin responsive elements such as glucose-6-phosphatase insulin responsive element (-780 to -722) [Ayala et al., Diabetes, (1999). 48:1885-9; and liver specific enhancer elements, such as prothrombin (940 to -860) [Chow et al., J Biol Chem, (1991) 266: 18927-33; and alpha-1-microglobulin (-2945 to - 2539) [Rouet et al., Biochem J, (1998). 334:577-84), Muscle-specific enhancers such as mammalian MCK enhancer, mammalian DES enhancer, and vertebrate troponin I IRE (TNI IRE, herein after referred to as FIRE) enhancer. Finally, a SV40 enhancer sequence may also be included.

Enhancer elements may further be used along with promoters as defined herein for upregulating expression of a peptide encoded by a nucleic acid sequence as defined herein, e.g. such promoter/enhancer combinations include e.g. the cytomegalovirus (CMV) promoter and the CMV enhancer, the CMV enhancer linked to the ubiquitin promoter (Cubi), the group of liver-specific enhancer elements comprising human serum albumin [HSA] enhancers, human prothrombin [HPrT] enhancers, alpha-1 microglobulin [A1MB] enhancers, and intronic aldolase enhancers used in combination with their corresponding promoters, or HSA enhancers used in combination with a promoter selected from the group of a CMV promoter or an HSA promoter, enhancer elements selected from the group consisting of human prothrombin [HPrT] and alpha-1 microglobulin [A1MB] used in combination with the CMV promoter enhancer elements selected from the group consisting of human prothrombin [HPrT] and alpha-1 microglobulin [A1MB] used in combination with the alpha-1-anti trypsin promoter, etc.

Furthermore, a vector as defined herein suitable for transfecting a cell which may be used as constituent of the (spherical) microcapsule as used according to the present invention, may contain transcriptional and/or translational signals, preferably transcriptional and/or translational signals recognized by an appropriate host, such as transcriptional regulatory and translational initiation signals. Transcriptional and/or translational signals may be obtained from any eukaryotic, prokaryotic, viral, bacterial, plant, preferably human or animal, e.g. mammalian hosts, preferably in association with the corresponding promoters as defined herein. A wide variety of transcriptional and translational regulatory sequences may be employed therefore, depending upon the nature of the host to the extent that the host cells recognizes the transcriptional regulatory and translational initiation signals associated with a nucleic acid sequence encoding a peptide as defined above, e.g. an anti-angiogenic factor, a neuroprotective factor and/or any other protein or protein-like substance suitable for (intraocular) treatment of eye diseases, or a fragment or variant thereof as defined herein, such as Endostatin, GLP-1 peptides, or a GLP-1 fusion peptide as defined herein, and optionally an additional factor as defined herein. The 5' region adjacent to the naturally occurring GLP-1 encoding nucleic acid sequence may be retained and employed for transcriptional and translational regulation in an inventive vector. This region typically will include those sequences involved with initiation of transcription and translation, such as the TATA box, capping sequence, CAAT sequence, and the like. Typically, this region will be at least about 150 base pairs long, more typically about 200 bp, and rarely exceeding about 1 to 2 kb.

Transcriptional initiation regulatory signals suitable for a vector as defined herein may be selected that allow to control repression or activation such that expression of the nucleic acid sequence encoding a peptide as defined above, e.g. an anti-angiogenic factor, a neuroprotective factor and/or any other protein or protein-like substance suitable for (intraocular) treatment of eye diseases, or a fragment or variant thereof as defined herein, such as Endostatin, GLP-1 peptides, or a GLP-1 fusion peptide, and optionally an additional factor as defined herein can be modulated. One such controllable modulation technique is the use of regulatory signals that are temperature-sensitive in order to repress or initiate expression by changing the temperature. Another controllable modulation technique is the use of regulatory signals that are sensitive to certain chemicals. These methods are preferably to be used in *in vitro* procedures, e.g. when preparing the necessary constructs. Furthermore, transcriptional initiation regulatory signals may be use herein, which allow to control repression or activation of expression *in vivo* without any further means from outside the cell, e.g. to obtain a transient expression in the encapsulated cells. Such transcription and/or translational signals include e.g. transcriptional termination regulatory sequences, such as a stop signal and a polyadenylated region. Furthermore, transcriptional termination regulatory sequences may be located in the non-coding 3' region of a vector as defined herein containing the nucleic acid sequence encoding a peptide as defined above, e.g. an anti-angiogenic factor, a neuroprotective factor and/or any other protein or protein-like substance suitable for (intraocular) treatment of eye diseases, or a fragment or variant thereof as defined herein, such as Endostatin, GLP-1 peptides, or a GLP-1 fusion peptide as defined herein, and optionally an additional factor as defined herein. Suitable termination sequences can include, for example, the bovine growth hormone, SV40, lacZ, EF1 alpha and AcMNPV polyhedral polyadenylation signals.

The expression vectors suitable for transfecting a cell which may be used for preparing the (spherical) microcapsule as used according to the present invention, may also include other sequences for optimal expression of the nucleic acid sequence encoding a peptide as defined above, e.g. an anti-angiogenic factor, a neuroprotective factor and/or any other protein or protein-like substance suitable for (intraocular) treatment of eye diseases, or a fragment or variant thereof as defined herein, such as Endostatin, GLP-1 peptides, or a GLP-1 fusion peptide as defined herein, and optionally an additional factor as defined herein. Such sequences include those encoding signal (peptide) sequences, i.e. which encode N-terminally located peptide sequences that provide for passage of the secreted protein into or through a membrane; which provide for stability of the expression product; and restriction enzyme recognition sequences, which provide sites for cleavage by restriction endonucleases. All of these materials are known in the art and are commercially available (see, for example, Okayama (1983), Mol. Cell. Biol., 3: 280).

As defined herein "a signal sequence" is a signal (peptide) sequence which typically comprises about 8 to 30 amino acids located - within the definitions of the above proviso regarding amino acids 1 to 6 of GLP-1 - at the N-terminus of the expressed anti-angiogenic factor, neuroprotective factor and/or further protein or protein-like substance suitable for (intraocular) treatment of eye diseases, or a fragment or variant thereof as defined herein or of further factors to be secreted, i.e. to pass through a cell membrane. Such a signal (peptide) sequence may include the signal (peptide) sequence normally associated with the wild type GLP-1 precursor protein (i.e., the signal (peptide) sequence(s) of the full length proglucagon precursor molecule), as well as signal (peptide) sequences which are not normally associated thereto, i.e. which are heterologous to the wild type GLP-1 precursor protein. A "signal (peptide) sequence" as defined herein can be, for example, a signal peptide sequence or a leader sequence (e.g. a secretory signal (and leader) sequence). Furthermore, signal (peptide) sequences as defined herein preferably provide for cleavage of the precursor peptide by a protease, e.g. a signal sequence protease. Upon cleavage of the signal sequence from the precursor peptide by the protease e.g. the biologically active anti-angiogenic factor, neuroprotective factor and/or further protein or protein-like substance suitable for (intraocular) treatment of eye diseases, or a fragment or variant thereof as defined herein, such as Endostatin, GLP-1 peptides, or a GLP-1 fusion peptide as defined herein, and optionally an additional factor as defined herein, is produced. Such a signal (peptide) sequence typically comprises a region which encodes a cleavage site recognized by a protease for cleavage. Alternatively, a region which encodes a cleavage site recognized by a protease for cleavage can be introduced into the signal (peptide) sequence.

Furthermore, additional (one or more) sequences which encodes a cleavage site recognized by a protease for cleavage can be added to the signal (peptide) sequence.

Examples of signal (peptide) sequences which can be encoded by a vector as defined herein include a signal (peptide) sequence derived from a secreted protein such as GLP-1 or other than GLP-1, such as a cytokine, a clotting factor, an immunoglobulin, a secretory enzyme or a hormone (including the pituitary adenylate cyclase activating polypeptide (PACAP)/glucagon superfamily) and a serum protein. For example, a signal (peptide) sequence as defined herein can be derived from secreted matrix metalloproteinases (MMP), e.g. a stromelysin leader sequence, from secreted human alkaline phosphatase (SEAP), proexendin, e.g. a proexendin-4 leader sequence, pro-helodermin, pro-glucose-dependent insulinotropic polypeptide (GIP), pro-insulin-like growth factor (IGF1), preproglucagon, alpha-1 antitrypsin, insulin-like growth factor 1, human factor IX, human lymphotoxin A (Genbank Accession no. BAA00064), or human clusterin (Genbank Accession No. AAP88927). Particular examples of signal (peptide) sequences as defined herein are sequences which include a coding region for a signal for precursor cleavage by signal peptidase, furin or other prohormone convertases (e.g., PC3). For example, a signal which is cleaved by furin (also known as PACE, see U.S. Pat. No. 5,460,950), other subtilisins (including PC2, PC1/PC3, PACE4, PC4, PC5/PC6, LPC/PC71PC8/SPC7 and SKI-1; Nakayama, Biochem. J., 327:625-635 (1997)); enterokinase (see U.S. Pat. No. 5,270,181) or chymotrypsin can be introduced into the signal (peptide) sequence as defined herein. The disclosure of each of these documents is hereby incorporated herein by reference. Furin is a ubiquitously expressed protease that resides in the trans-golgi and processes protein precursors before their secretion. Furin cleaves at the COOH-terminus of its consensus recognition sequence, Arg-X-Lys-Arg or Arg-X-Arg-Arg, (Lys/Arg)-Arg-X-(Lys/Arg)-Arg and Arg-X-X-Arg, such as an Arg-Gln-Lys-Arg. These amino acid sequences are a signal for precursor cleavage by the protease furin. Thus, a heterologous signal sequence can also be synthetically derived from a consensus sequence compiled from signal sequences (e.g., a consensus sequence compiled from secreted proteins that are cleaved by signal peptidase).

Additionally to regulation sequences as defined herein, an autonomously replicating vector as defined herein typically comprises an origin of replication. Suitable origins of replication include, without being limited thereto, e.g. ColE1, pSC101, SV40, pMPl (ori pMPl) and M13 origins of replication, etc.

Preferably, a vector as defined herein, suitable for expression of a peptide as defined above, e.g an anti-angiogenic factor, a neuroprotective factor and/or any other protein or protein-like substance suitable for (intraocular) treatment of eye diseases, or a fragment or variant thereof as defined herein, such as Endostatin, GLP-1 peptides, or a GLP-1 fusion peptide as defined herein, and optionally an additional factor as defined herein, may additionally contain a suicide gene. In the context of the present invention "a suicide gene" is preferably capable to stop the therapy with (spherical) microcapsules, as used herein, by killing the suicide gene harbouring cell contained in the (spherical) core of the (spherical) microcapsule upon administering a specific substance. In other words, a suicide gene suitable for the present invention may be activated by administering an exogenous activator that typically does not occur in the human or animal body. In this case, typically the suicide gene initiates a cascade causing the cell to undergo an apoptotic event. Alternatively, a suicide gene suitable for the present invention may metabolize an administered exogenous non-toxic prodrug that typically does not occur in the human or animal body. Metabolism of the exogenous non-toxic prodrug preferably renders the prodrug to a cell toxin. The suicide gene may be contained on the same vector encoding the anti-angiogenic factor, neuroprotective factor and/or further protein or protein-like substance suitable for (intraocular) treatment of eye diseases, or a fragment or variant thereof as defined herein, such as Endostatin, GLP-1 peptides, or a GLP-1 fusion peptide as defined herein or alternatively on a second vector. Furthermore, the suicide gene may be regulated by control and regulatory elements of any kind, e.g. control and regulatory elements such as promoters, enhancers, etc. as mentioned herein as constituents of expression vectors, or by their naturally occurring control and regulatory elements. Preferably, suicide genes are selected according to the present invention, which allow any of the above control mechanisms, e.g. suicide genes selected from cytosin deaminase (CD), uracil phosphoribosyl transferase (UPRTase), HSV thymidine kinase (HSV-Tk), suicide genes which may be induced by addition of tetracycline such as the bacterial Tet repressor protein (TetR), etc. As a particular example the cytosine desaminase (CD) may be used. The cytosine desaminase (CD) typically occurs in a variety of organisms and is capable of transforming 5-fluorocytosin (5-FC) into 5-fluorouracil (5-FU), which represents a common chemotherapeutic agent. 5-Fluorouracil (5-FU) is highly toxic for the organism whereas its prodrug 5-fluorocytosin (5-FC) is not toxic to cells. 5-Fluorouracil (5-FU) is subsequently phosphorylated by cellular kinases and is capable of abrogating the cells RNA synthesis. Thus, the prodrug 5-fluorocytosin (5-FC) represents an excellent tool for inducing suicide of a specific cell. Furthermore, 5-Fluoro-dUMP acts as antifolate agent and inhibits the enzyme thymidylat synthase, which catalyses methylation of dUMP to dTMP in the *de novo* synthesis path of desoxyribonucleotides. Thereby, inhibition of DNA synthesis in the cell may be achieved. Also preferably, the HSV-1 thymidin kinase (ATP: Thymidin-5-phosphotransferase) and its corresponding prodrug ganciclovir (GCV) may be used. The guanosin analog GCV is specifically phosphorylated and inhibits elongation of DNA synthesis and thus leads to suicide of the cell.

Transfection of the vectors as defined herein or, alternatively, of naked nucleic acid encoding an anti-angiogenic factor, a neuroprotective factor and/or any other protein or protein-like substance suitable for (intraocular) treatment of eye diseases, or a fragment or variant thereof as defined herein, such as Endostatin, GLP-1 peptides, or a GLP-1 fusion peptide as defined herein, and optionally an additional factor as defined herein into suitable cells used for preparation of (spherical) microcapsules as defined herein, may be accomplished by any method known to a skilled person (see e.g. Maniatis et al. (2001) Molecular Cloning: A laboratory manual, Cold Spring Harbor Laboratory Press, Cold Spring Harbor, NY). If vectors are transfected into suitable cells as defined herein, the vector is preferably present in the form of a plasmid DNA, which carries a GLP-1 peptide encoding nucleic acid. The plasmid DNA is preferably a circular plasmid DNA. Suitable transfection methods include, without being limited thereto, e.g. electroporation techniques including modified electroporation techniques (e.g. nucleofection), calcium phosphate techniques, e.g. the calcium phosphate co-precipitation method, the DEAE-Dextran method, the lipofection method, e.g. the transferring-mediated lipofection method, etc. Preferably, transfection is carried out with plasmid DNA carrying a vector as defined herein using a modified electroporation technique (e.g. nucleofection).

The vector as defined herein or, alternatively, the nucleic acid encoding an anti-angiogenic factor, a neuroprotective factor and/or any other protein or protein-like substance suitable for (intraocular) treatment of eye diseases, or a fragment or variant thereof as defined herein, such as Endostatin, GLP-1 peptides, or a GLP-1 fusion peptide as defined herein, and optionally an additional factor as defined herein, may furthermore be complexed, e.g. for transfection with at least one synthetic polymer or a natural polymer, e.g. polyamino acids, or may be conjugated thereto. At least one polymer constituent may be covalently coupled to the vector as defined herein or, alternatively, the nucleic acid encoding an anti-angiogenic factor, a neuroprotective factor and/or any other protein or protein-like substance suitable for (intraocular) treatment of eye diseases, or a fragment or variant thereof as defined herein. "Conjugated" in the meaning of the present invention is intended to mean "chemically coupled". "Chemically coupled" is intended to mean coupled *via* covalent or non-covalent bonding. While covalent bonding may also be utilized, non-covalent bonding is preferred for transfection purposes. Thereby, the polymer constituent may be linked to the fusion peptide *via* complexation without covalent linkage, e.g. *via* hydrogen bonding or electrostatic, hydrophobic, etc., interaction.

The polymer used herein for coupling the vector as defined herein or, alternatively, the nucleic acid encoding an anti-angiogenic factor, a neuroprotective factor and/or any other protein or protein-like substance suitable for (intraocular) treatment of eye diseases, or a fragment or variant thereof as defined herein (for transfection purposes) may be a physiologically acceptable polymer which includes polymers which are soluble in an aqueous solution or suspension and have no negative impact, such as side effects, to mammals upon administration of the fusion peptide in a pharmaceutically effective amount. There is no particular limitation to the physiologically acceptable polymer used according to the present invention. The polymer may be of synthetic nature or may be a naturally occurring polymer (e.g. a protein).

More generally, the synthetic polymer used with a vector as defined herein or, alternatively, the nucleic acid encoding an anti-angiogenic factor, a neuroprotective factor and/or any other protein or protein-like substance suitable for (intraocular) treatment of eye diseases, or a fragment or variant thereof as defined herein is preferably selected from alkylene glycols, such as polyethylene glycol (PEG), polypropylene glycol (PPG), copolymers of ethylene glycol and propylene glycol, polyoxyethylated polyol, polyolefinic alcohol, polyvinylpyrrolidone, polyhydroxyalkyl methacrylamide, polyhydroxyalkyl methacrylate, such as polyhydroxyethylene methycrylate, polyacrylate, polysaccharides, poly([alpha]-hydroxy acid), polyvinyl alcohol, polyphosphazene, polyoxazoline, poly(N-acryloylmorpholine), polyvinylethyl ether, polyvinlyacetal, polylactic glycolic acid, polylactic acid, lipid polymer, chitin, hyaluronuic acid, polyurethyne, polysialic acid, cellulose triacetate, cellulose nitrate and combinations of any of the foregoing.

The present invention also provides a method for preparing the (spherical) microcapsules as used according to the present invention. These (spherical) microcapsules are preferably prepared according to two or more method steps. According to a method step 1) a core is prepared as disclosed above. According to a method step 2) the core as prepared according to method step 1) is coated by one or more surface coating layer(s). Further optional steps may comprise repetition of method step 2) for the preparation of additional surface coating layers. Preferably, a step identical to method step 2) is carried out for each of such additional surface coating layers. Further optional steps may include washing steps subsequent to preparation of the spherical microcapsule.

Typically, a core as disclosed above is prepared according to method step 1) for preparing (spherical) microcapsules, as used according to the present invention. Such a core is composed of cross-linked polymer and cells encoding and secreting an anti-angiogenic factor, a neuroprotective factor and/or any other protein or protein-like substance suitable for (intraocular) treatment of eye diseases, or a fragment or variant thereof as defined herein, such as Endostatin, GLP-1 peptides, or a GLP-1 fusion peptide as defined herein, and optionally an additional factor as defined herein, which have been transfected according to a method as disclosed above. According to method step 1), a mixture (suspension) of the soluble form of the polymer, e.g. the soluble form of an alginate (e.g. potassium or sodium alginate in physiological saline solution), and of the above defined cells is typically prepared, preferably in a concentration as defined herein for the (spherical) core, e.g. of 1 x 10⁵ up to 6 x 10⁷cells, per ml polymer solution.

As a typical technique the homogenic cell/polymer suspension (e.g. cell/alginate suspension) may be pressed *via* an air injected spray nozzle, consisting of three channels, which are arranged concentrically as three concentric rings around a common centre: an inner channel, an intermediate channel and an outer channel (air ring). Preferably hollow needles are used for the inner channel having an inner diameter of 50 µm up to 2,000 µm. The intermediate channel typically has an inner diameter of 60 µm to 4,000 µm, and the outer channel (air ring) preferably has an inner diameter of 100 µm to 5,000 µm. Exclusively the inner channel and the outer channel (air ring) are used in method step 1) for preparing the core of the (spherical) microcapsule, as used according to the present invention. Thus, a spray nozzle merely consisting of two channels (an inner and an outer channel) may be used in method step 1) as well. Typically, no material flows through the intermediate channel, if an air injected spray nozzle with three channels is used. The suspension of the cell/polymer solution is typically pressed with a speed of 10 µl/min to 5ml/min through the inner channel leading to droplets at the outlet of the channel, which tear off due to the air flow provided by the outer channel (air ring), having a speed of typically 0.5 l/min to 10 l/min. Droplets containing cells and non-cross-linked polymer solution fall down into a cross-linker containing solution (precipitation bath), which is typically positioned in a distance of about 4 cm to about 60 cm under the outlet of the air injected spray nozzle. The droplet preferably rounds during dropping down, thereby receiving a substantially spherical geometrical form. The cross-linker effects ionical cross-linking of the polymers and the core of the spherical (water insoluble) microcapsule is initially formed having a diameter as defined herein for the (spherical) core. The diameter of the core of the (spherical) microcapsule is dependent on size and geometry of the chosen channels used in method step 1). The cross-linker containing solution (precipitation bath) is preferably composed of bivalent cations, e.g. calcium or barium ions (5-100 mM) or other bivalent or multivalent cations, if alginates are used as polymers. Furthermore, the precipitation bath preferably contains a buffer substance (e.g. 1mM - 10mM histidine) and sodium chloride (e.g. 290 mOsmol ± 50 mOsmol). Other suitable cross-linkers and buffers known in the art may be used herein, if other polymers than alginates are used.

Method step 1) provides the core of the (spherical) microcapsule composed of cross-linked polymers and cells as defined herein. Subsequent to method step 1) optional method step(s) may include a washing step. The core of the (spherical) microcapsule, as used according to the present invention, is e.g. washed with a physiological saline solution or any other suitable washing solution and, if applicable, the core is incubated in a sodium sulfate solution, preferably in a sodium sulfate solution according to US 6,592,886, the disclosure of which is incorporated herein by reference. Separation of the cores of the (spherical) microcapsules, as used according to the present invention, from the precipitation bath and/or the washing bath is typically is carried out using a centrifuge or any other suitable method.

According to method step 2) the core of the (spherical) microcapsule, as used according to the present invention, prepared by method step 1) is coated with a surface coating layer substantially of cross-linked polymer. Accordingly, the core of the (spherical) microcapsule, prepared by step 1), is added to a polymer solution containing non-crosslinked polymers as disclosed above comprising no cells. Preferably, the polymers are provided in their non-cross-linked form in a concentration as defined herein. Typically, this mixture containing the polymer solution and the core of the (spherical) microcapsule is pressed through the inner channel of the above-described air injected spray nozzle, e.g. with a speed of 15 µl/min to 2 ml/min, preferably 10 µl/min to 5 ml/min. Simultaneously, a pure non-cross-linked polymer solution without cells, preferably a solution comprising about 0.1% to about 4% (w/v) polymer, e.g. an alginate solution without any cells, is pressed through the intermediate channel with a speed of typically 15 µl/min to 2 ml/min, preferably 10 µl/min to 5 ml/min. Thereby, droplets are formed at the end of the intermediate channel, containing the core and a surface of non-polymerized polymer. These droplets tear off due to the air flow provided *via* the outer channel (air ring) having a speed of typically 0.5 l/min to 10 l/min. The polymer concentration of the core of the (spherical) microcapsule, the polymer solution, into which the core of the (spherical) microcapsules is added, and the polymer concentration of the surface coating layer may differ (see above). The droplets containing the core of the (spherical) microcapsules (prepared according to method step 2) fall into a solution containing the cross-linker (precipitation bath) as defined herein. During dropping down, the droplet preferably rounds to an approximately spherical geometrical form. The cross-linker affects an ionic cross-linkage of the polymers analogous to method step 1). Thereby, water insoluble (spherical) microcapsules are formed having a diameter as defined herein, more preferably a total diameter (particle size) of the (spherical) microcapsule of about 100 µm to about 200 µm, more preferably a total diameter of about 115 µm to about 185 µm, even more preferably a total diameter of about 130 µm to about 1 70 µm, and most preferably a total diameter of about 145 µm to about 155 µm, e.g. about 150 µm. The total diameter of (spherical) microcapsules obtainable by method step 2) is dependent from size and geometry of the chosen channels, as used herein. In order to prepare (spherical) microcapsules as defined herein, with more than one surface coating layer, i.e. the (spherical) microcapsules containing the core as defined herein and 2, 3, 4, 5, 5-10 or more surface coating layers, method step 2) may be repeated as often as necessary. Those further surface coating layers are defined within the above diameter ranges.

Subsequent to method step 2) one or more optional washing steps may follow as defined herein.

According to a further aspect the present invention also provides a method of treatment of eye diseases in an animal, preferably a mammal. Such a method of treatment may therefore be used in the field of either human medicine or veterinary medicine. In the context of the present invention the term mammal typically comprises any animal and human, preferably selected from the group comprising, without being restricted thereto, humans and (mammalian) (non-human) animals, including e.g. pig, goat, cattle, swine, dog, cat, donkey, monkey, ape or rodents, including mouse, hamster and rabbit, cow, rabbit, sheep, lion, jaguar, leopard, rat, pig, buffalo, dog, loris, hamster, guinea pig, fallow deer, horse, cat, mouse, ocelot, serval, etc. Such a treatment typically occurs by administration of (spherical) microcapsules as defined herein to a patient in need thereof, particularly by the administration of cells as defined herein, e.g. mesenchymal stem cells or mesenchymal stromal cells, or any other cell (type), that may be used in the context of the present invention, encoding and secreting to an anti-angiogenic factor, a neuroprotective factor and/or any other protein or protein-like substance suitable for (intraocular) treatment of eye diseases, or a fragment or variant thereof as defined herein, such as Endostatin, a GLP-1 peptide, a GLP-1 fusion peptide, etc., or a fragment or variant thereof, wherein these cells are encapsulated in a (spherical) microcapsule as defined herein to prevent a response of the immune system of the patient to be treated. Such a treatment may furthermore occur by administration of a (pharmaceutical) composition comprising (spherical) microcapsules as defined herein, particularly of cells as defined herein, e.g. mesenchymal stem cells or mesenchymal stromal cells, or any other cell (type), that may be used in the context of the present invention, encoding and secreting to an anti-angiogenic factor, a neuroprotective factor and/or any other protein or protein-like substance suitable for (intraocular) treatment of eye diseases, or a fragment or variant thereof as defined herein, such as Endostatin, a GLP-1 peptide, a GLP-1 fusion peptide, etc., or a fragment or variant thereof, wherein these cells are encapsulated in a (spherical) microcapsule as defined herein to prevent a response of the immune system of the patient to be treated. Preferably, the (spherical) microcapsule as well as all its components as used in the inventive method, e.g., polymers of the polymer matrix of the core or the surface coating layer, etc., is as defined herein. Additionally, such a treatment may also occur by direct administration of an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide, all as defined herein. The above method of treatment according to the invention can be used for human and also for veterinary medical purposes.

The method of treatment defined herein preferably comprises treatment or prevention of eye diseases, particularly of eye diseases and disorders caused by a degeneration of the retina, including, among other retinal diseases, selected from retinitis pigmentosa (RP), macular degeneration (MD), age related macular degeneration (AMD or ARMD), macula edema due ot other reasons, retinal vessel occlusions, diabetic retinopathy, glaucoma and other optic neuropathies, etc., and the treatment or prevention of conditions associated therewith.

Treating or preventing eye diseases or disorders or diseases associated thereto using (spherical) microcapsules as defined herein or a (pharmaceutical) composition comprising these (spherical) microcapsules) or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, preferably results from the above described beneficial effects of the anti-angiogenic factor, neuroprotective factor and/or further protein or protein-like substance suitable for (intraocular) treatment of eye diseases, or a fragment or variant thereof, e.g. Endostatin, GLP-1 peptides, GLP-1 fusion peptides, etc. For example, beneficial effects are known for GLP-1, such as its activity to powerfully reduce the damages caused by ischemia or oxygen shortage and potential death of heart tissue without the need of repeated administration of GLP-1 peptide(s) due to its DPP-IV protease stability and/or the risk of an undesired immune response against e.g. implanted GLP-1 expressing allogenic cells. Beneficial effects known for Endostatin, the cleavage product of collagen XVIII, include its capability to reduce angiogenesis significantly. A "safe and effective amount" of the cells of the (spherical) microcapsule as defined herein or of an anti-angiogenic factor, a neuroprotective factor and/or any other protein or protein-like substance suitable for (intraocular) treatment of eye diseases, or a fragment or variant thereof as defined herein, such as Endostatin, a GLP-1 peptide, a GLP-1 fusion peptide, etc., or a fragment or variant thereof will furthermore vary in connection with the particular eye disease or disorder to be treated and also with the age and physical condition of the patient to be treated, the severity of the condition, the duration of the treatment, the nature of the accompanying therapy, of the particular pharmaceutically acceptable carrier used, and similar factors, within the knowledge and experience of the administering doctor.

As defined herein, the method of treatment or prevention of eye diseases or of any disease or condition associated with such eye diseases comprises administering the (spherical) microcapsule as defined herein, or administering a (pharmaceutical) composition containing such (spherical) microcapsule, to a patient in need thereof or administration of an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein,. "A patient in need thereof" is typically an animal, preferably a mammal. More preferably, "a patient in need thereof" is selected from the group comprising, without being restricted thereto, humans, and animals, including e.g. pig, goat, cattle, swine, dog, cat, donkey, monkey, ape or rodents, including mouse, hamster and rabbit.

Administration in the context of the above method of treatment typically occurs by administering a "safe and effective" amount of the active agent, e.g. of the (spherical) microcapsules, i.e. the cells of the (spherical) microcapsule as defined herein, encoding and secreting an anti-angiogenic factor, a neuroprotective factor and/or any other protein or protein-like substance suitable for (intraocular) treatment of eye diseases as defined herein, such as Endostatin, a GLP-1 peptide, a GLP-1 fusion peptide, etc., or a fragment or variant thereof or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein,. As used herein, "safe and effective amount" means an amount of the active agent as defined above, that is sufficient to significantly induce a positive modification of an eye disease or disorder as mentioned herein. At the same time, however, a "safe and effective amount" is small enough to avoid serious side-effects that is to say to permit a sensible relationship between advantage and risk. The determination of these limits typically lies within the scope of sensible medical judgment. In the context of the present invention the expression "safe and effective amount" preferably means an amount of the anti-angiogenic factor, neuroprotective factor and/or other protein or protein-like substance suitable for (intraocular) treatment of eye diseases, or a fragment or variant thereof, such as Endostatin, GLP-1 peptides, or a GLP-1 fusion peptide as defined herein, or of the cells of the (spherical) microcapsule as defined herein, encoding and secreting an anti-angiogenic factor, a neuroprotective factor and/or any other protein or protein-like substance suitable for (intraocular) treatment of eye diseases as defined herein, such as Endostatin, a GLP-1 peptide, a GLP-1 fusion peptide, etc., or a fragment or variant thereof that is suitable to exert beneficial effects known for such an anti-angiogenic and/or a neuroprotective factor as defined herein or a fragment or variant thereof.

In the above context, the cells of the (spherical) microcapsules as defined herein, encoding and secreting an anti-angiogenic factor, a neuroprotective factor and/or any other protein or protein-like substance suitable for (intraocular) treatment of eye diseases as defined herein, such as Endostatin, a GLP-1 peptide, a GLP-1 fusion peptide, etc., or a fragment or variant thereof, typically secrete an as defined herein, e.g. GLP-1 peptide and/or Endostatin in a concentration of about 0.2 µg per day per ml of (spherical) microcapsules. Thus, a dosage range may be e.g. in the range from about 0.01 µg to 20 mg of secreted biologically active anti-angiogenic, neuroprotective factor or any other protein or protein-like substance suitable for (intraocular) treatment of eye diseases, or a fragment or variant thereof as defined herein, e.g. GLP-1 peptide and/or Endostatin, per day (even though higher amounts in the range of 1-100 mg are also contemplated), such as in the range from about 0.01 µg to 10 mg per day, preferably in the range from 0.01 µg to 5 mg per day and even more preferably in the range from about 0.01 µg to 1 mg per day and most preferably in the range from about 0.01 µg to 500 µg per day. This may also apply, if an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, is administered directly without cells.

Administration in the context of the above method of treatment typically occurs by providing the cells, encapsulated in a (spherical) microcapsule as defined herein, or the (spherical) microcapsule as defined herein, or the (pharmaceutical) composition containing such (a) (spherical) microcapsule(s), or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, into a specific administration site in the patient to be treated. Such a specific administration site is typically the eye, e.g. an intraocular administration, preferably the affected area of the eye of a patient to be treated, more particular a specific tissue or area, such as the retina, the iris, more preferably, 2-3 mm apart the iris, e.g. after the conjunctiva and sclera has been slightly disarranged, into the vitreous cavity of the eye. It is of major importance to realize that the vitreous cavity in the eye can be used as drug reservoir for the treatment of intraocular diseases. Since the eye represents a 10,000^{th} of the body volume, a drug if not administreed intraocularly, would have to be given systemcially in a 10,000 times higher dosage to theoretically achieve the same concentration at the site of requiered action, i.e. the retina or other intraocular structures. It would lead a tremendous risk of systemic side effects. As an example, the relatively small dosage of 25 mg of a crystalline steroid (triamcinolone) given intraocularly has an equivalent dosage of a quarter of a kilogram given systemically. Such a dosage is compatible with survival of the patient. In addition, the direct intaocular application of the drug avoids the problemof the blood-retinal barrier, which limits the avaialbility of the drug in retinal tissue although it may be transported through the retinal blood vessels. Due to relatively tight junctions in the retinal vessel walls, the drugs are often, however, not fully able to leave the vessel and to penetrate into the retinal tissue. In this context, it is also of major importance, that the repeated intraocular application of drugs may also be associated with the cumulative risk of iatrogenic lesions to the lens and retina, and with the cumulative risk of infections, when administration is repeated too often. It is therefore highly desirable to develop procedures and techniques which markedly decrease the number of necessary re-applications as done according to the present invention. The implantation of drug producing cells into the eye, covered by s atructure which protects these cells form being immunologically attacked, which prevents the cells from leaving their "cage", and which simultaneously allows an exchange of substance for the nourishment of the cells for the release of the produced drug, can be compared with the minimisation and translocation of a drug producing fabric into the eye.

Administration sites in the context of the present ionvention furthermore include e.g. an intraocular administration, any surface of the eye or the retina, the iris or its tissues or surrounding areas, which may be treated, without being limited thereto, by injection or implantation. Alternatively, cells, encoding and secreting an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, may also be implanted into the anterior chamber, the lens capsular bag (e.g., after cataract surgery), the subretinal space, and the suprachoroidal space. The implantation of the cells may be performed by injection or any other surgical or non-surgical procedure to bring the cells into the interior of eye, defined as all volume inside of the sclera.

Administration sites may furthermore include any cavity suitable for treatment of an eye disease as mentioned herein.

Administration of the (spherical) microcapsule as defined herein, particularly of cells, encapsulated in a (spherical) microcapsule as defined herein, encoding and secreting an anti-angiogenic factor, a neuroprotective factor and/or any other protein or protein-like substance suitable for (intraocular) treatment of eye diseases as defined herein, such as Endostatin, a GLP-1 peptide, a GLP-1 fusion peptide, etc., or a fragment or variant thereof, or administration of the (pharmaceutical) composition containing such (spherical) microcapsule, into a specific administration site as defined herein or administration of an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, may be carried out using different modes of administration. The cells, encapsulated in a (spherical) microcapsule as defined herein, or the (spherical) microcapsule as defined herein, or the (pharmaceutical) composition containing such (spherical) microcapsule, or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, can be administered, for example, systemically or locally. Routes for systemic administration in general include, for example, transdermal or parenteral routes, including intravenous, subcutaneous, and/or intraarterial injections. Routes for local administration in general include, e.g., topical administration routes but also transdermal, intramuscular, subcutaneous, intracardial, intramyocardial and/or pericardial injections. More preferably, the cells, encapsulated in a (spherical) microcapsule as defined herein, or the (spherical) microcapsule as defined herein, or the (pharmaceutical) composition containing such (spherical) microcapsule, or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, may be administered by an intradermal, subcutaneous, or intramuscular route. For injection purposes, the cells, encapsulated in a (spherical) microcapsule as defined herein, or the (spherical) microcapsule as defined herein, or the (pharmaceutical) composition containing such (spherical) microcapsule or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, may be formulated in a suitable form, e.g. by addition of suitable pharmaceutical carriers, e.g. in the form of liquid pharmaceutical composition, gels, etc.

Other modes of administration, which may be suitable for treatment of any of the afore mentioned eye diseases or disorders, include transplantation of the cells, encapsulated in a (spherical) microcapsule as defined herein, or the (spherical) microcapsule as defined herein, or the (pharmaceutical) composition containing such (spherical) microcapsule into or to an administration site as defined herein. For transplantation purposes, the cells, encapsulated in a (spherical) microcapsule as defined herein, or the (spherical) microcapsule as defined herein, or the (pharmaceutical) composition containing such (spherical) microcapsule may be formulated in a suitable form, e.g. by addition of suitable pharmaceutical carriers, e.g. in the form of gels, capsules, tablets, etc.

The cells, encapsulated in a (spherical) microcapsule as defined herein, or the (spherical) microcapsule as defined herein, or the (pharmaceutical) composition containing such (spherical) microcapsule or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, may be directly delivered to an administration site as defined herein by interventional means, e.g. using a catheter to navigate to the affected area and implant the (spherical) microcapsule by injection into the administration site. Implantation could also be performed using surgical routine methods, e.g. ornon-surgical procedures.

According to a particularly preferred embodiment, the (spherical) microcapsules, particularly cells as defined herein, encoding and secreting an anti-angiogenic factor, a neuroprotective factor and/or any other protein or protein-like substance suitable for (intraocular) treatment of eye diseases as defined herein, such as Endostatin, a GLP-1 peptide, a GLP-1 fusion peptide, etc., or a fragment or variant thereof, or a (pharmaceutical) composition comprising such (spherical) microcapsules, or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, may be administered e.g. *via* injection as already defined above. Particularly preferred, injection occurs by applying an appropriate injection needle, e.g. such as injection needles having a size of from 12 to 26 G, more preferably of from 18 to 22 G. According to another particularly preferred embodiment, the (spherical) microcapsules, particularly cells as defined herein, encoding and secreting an anti-angiogenic factor, a neuroprotective factor and/or any other protein or protein-like substance suitable for (intraocular) treatment of eye diseases as defined herein, such as Endostatin, a GLP-1 peptide, a GLP-1 fusion peptide, etc., or a fragment or variant thereof, or a (pharmaceutical) composition comprising such (spherical) microcapsules, or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, may be administered e.g. *via* transplanting, preferably by using surgical devices, such as scalpels, injection needles an anti-angiogenic factor, a neuroprotective factor and/or any other protein or protein-like substance suitable for (intraocular) treatment of eye diseases as defined herein, etc.

The invention furthermore encompasses the use of (spherical) microcapsules as defined herein comprising cells encoding and secreting an anti-angiogenic factor, a neuroprotective factor and/or any other protein or protein-like substance suitable for (intraocular) treatment of eye diseases, such as Endostatin, GLP-1 peptides, or a GLP-1 fusion peptide as defined herein, or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, for the manufacture of a product, e.g. a (pharmaceutical) composition or a kit, for the treatment of eye diseases or disorders or diseases or disorders associated thereto in an animal, preferably a mammal as defined herein, such as a human being. In this context, such inventive pharmaceutical compositions may be used in the field of either human medicine or veterinary medicine. The invention furthermore encompasses the use of cells encoding an anti-angiogenic factor, a neuroprotective factor and/or any other protein or protein-like substance suitable for (intraocular) treatment of eye diseases, or a fragment or variant thereof as defined herein, such as Endostatin, GLP-1 peptides, or a GLP-1 fusion peptide as defined herein, or of an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, for the manufacture of ((spherical) microcapsules as defined herein, preferably for preparing a product, e.g.) a (pharmaceutical) composition or a kit, for the treatment of eye diseases or disorders or diseases or disorders associated thereto in an animal, preferably a mammal as defined herein, such as a human being. If cells are used, the cells as used for such a purpose are preferably cells as defined herein, e.g. mesenchymal stem cells or mesenchymal stromal cells, or any other cell (type), that may be used in the context of the present invention, encoding and secreting an anti-angiogenic factor, a neuroprotective factor and/or any other protein or protein-like substance suitable for (intraocular) treatment of eye diseases, or a fragment or variant thereof as defined herein, such as Endostatin, GLP-1 peptides, or a GLP-1 fusion peptide as defined herein, wherein these cells, are encapsulated in a (spherical) microcapsule to prevent a response of the immune system of the patient to be treated.

Another aspect of the present invention is a (pharmaceutical) composition containing (spherical) microcapsules as defined herein comprising cells encoding and secreting an anti-angiogenic factor, a neuroprotective factor and/or any other protein or protein-like substance suitable for (intraocular) treatment of eye diseases, or a fragment or variant thereof as defined herein, such as Endostatin, GLP-1 peptides, or a GLP-1 fusion peptide as defined herein or comprising an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein,. Such a (pharmaceutical) composition may be applied to a patient suffering from the above defined eye diseases or disorders, preferably to administration sites as defined herein in a mode as defined herein.

Preparation of (pharmaceutical) compositions which contain (spherical) microcapsules as defined herein comprising cells encoding and secreting an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein or containing or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, as an "active ingredient", is generally well understood in the art, as e.g. exemplified by US Patents 4,608,251; 4,601,903; 4,599,231; 4,599,230; 4,596,792; and 4,578,770, all incorporated herein by reference.

Typically, (pharmaceutical) compositions are prepared as injectables either as liquid solutions or suspensions, preferably containing water (aqueous formulation) or may be emulsified. The term "aqueous formulation" is defined as a formulation comprising at least 50 % w/w water. Likewise, the term "aqueous solution" is defined as a solution comprising at least 50% w/w water, and the term "aqueous suspension" is defined as a suspension comprising at least 50 % w/w water.

For intramuscular, intravenous, cutaneous or subcutaneous injection, or any further injection into an administration site as defined herein, the cells or (spherical) microcapsules as defined herein or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, or a pharmaceutical composition as defined above, will be in the form of a parenterally acceptable aqueous solution which is pyrogen-free and has suitable pH, isotonicity and stability. Liquid (pharmaceutical) compositions generally include a liquid vehicle such as water. Preferably, the liquid vehicle will include a physiological saline solution, dextrose ethanol or other saccharide solution or glycols such as ethylene glycol, propylene glycol or polyethylene glycol or combinations thereof may be included. Further examples include other isotonic vehicles such as physiological salt solutions, e.g. Ringers solution or Lactated Ringer's solution.

If the inventive (pharmaceutical) composition comprises an aqueous solution of cells or (spherical) microcapsules as defined herein or of an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, and e.g. a buffer, said (spherical) microcapsule or an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, is typically present in the (pharmaceutical) composition in a concentration from 0.1 mg/ml or above, and said (pharmaceutical) composition usually has a pH from about 2.0 to about 10.0, preferably from about 7.0 to about 8.5.

It is possible that other ingredients may be present in the inventive (pharmaceutical) composition. Such additional ingredients may include wetting agents, emulsifiers, antioxidants, bulking agents, pH buffering agents (e.g. phosphate or citrate or maleate buffers), preservatives, surfactants, stabilizers, tonicity modifiers, cheating agents, metal ions, oleaginous vehicles, proteins (e.g. human serum albumin, gelatin or proteins) and/or a zwitterion (e.g. an amino acid such as betaine, taurine, arginine, glycine, lysine and histidine). Such ingredients are selected by a skilled person according to the specific requirements of the cells embedded in the (spherical) core of the (spherical) microcapsule, as used according to the present invention or of an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, i.e. the ingredients are not cytotoxic and ensure viability of the cells. Furthermore, such ingredients may stabilize an anti-angiogenic factor, a neuroprotective factor and/or any other protein or protein-like substance suitable for (intraocular) treatment of eye diseases, or a fragment or variant thereof, which may be encoded and secreted by the cells embedded in the (spherical) core of the (spherical) microcapsule, as used according to the present invention.

With regard to buffers these are preferably selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethane, hepes, bicine, tricine, malic acid, succinate, maleic acid, fumaric acid, tartaric acid, aspartic acid or mixtures thereof. Each one of these specific buffers constitutes an alternative embodiment of the invention.

The use of all of the afore-mentioned additives in (pharmaceutical) compositions as defined herein, is well-known to the skilled person, in particular with regard to concentration ranges of the same. For convenience reference is made to Remington: The Science and Practice of Pharmacy, 19th edition, 1995.

Inventive (pharmaceutical) compositions containing (spherical) microcapsules comprising cells as defined herein, encoding and secreting an anti-angiogenic factor, a neuroprotective factor and/or any other protein or protein-like substance suitable for (intraocular) treatment of eye diseases as defined herein or containing an anti-angiogenic factor, a neuroprotective factor and/or any other protein or protein-like substance suitable for (intraocular) treatment of eye diseases, or a fragment or variant thereof, such as Endostatin, GLP-1 peptides, or a GLP-1 fusion peptide as defined herein, are preferably administered in a manner as defined herein for treatments in general. Such administrations are preferably compatible with the dosage formulation, and comprise preferably a safe and effective amount of the active ingredients, e.g. of the cells as defined herein or an anti-angiogenic factor, a neuroprotective factor and/or any other protein or protein-like substance suitable for (intraocular) treatment of eye diseases, or a fragment or variant thereof, i.e. such amount which is regarded as safe but therapeutically effective. The quantity of such cells or of an anti-angiogenic factor, a neuroprotective factor and/or any other protein or protein-like substance suitable for (intraocular) treatment of eye diseases as defined herein, to be administered with an inventive (pharmaceutical) composition (or, if required, alone), depends on the subject and the disease to be treated, including, e.g., the severity of the patient's disease. Suitable dosage ranges depend on the amount of a biologically active anti-angiogenic, neuroprotective factor or further protein or protein-like substance suitable for (intraocular) treatment of eye diseases as defined herein directly provided or secreted by the (spherical) microcapsules (as contained in the inventive (pharmaceutical) composition) during a predetermined time period and typically range in the order of one to several hundred micrograms (anti-angiogenic and/or a neuroprotective factor as defined herein and/or further suitable factor) per day as defined herein.

The present invention may furthermore comprise combinations of the above described embodiments and features if not described otherwise and is not intended to be limited to these particularly defined single embodiments.

### Figures:

The invention is illustrated further in the accompanying Figures. However, it is not intended to limit the scope of the invention to the content of the Figures as shown in the following.
- **Fig. 1:**: shows a non-limiting overview over exemplary constructs a - m (see also Example 1), which may be contained in cells used for preparation of the (spherical) microcapsules, as used according to the present invention.
- **Fig. 2:**: depicts the results of transient expression of different GLP-1 constructs in hTERT-MSC and HEK293 cells and of active GLP-1 after transient transfection (see also Example 2). Only marginal active GLP-1 levels can be found in the monomeric GLP-1 constructs #103 and #317 (having just one copy of GLP-1 (7-37)). An enormous gain in expression was observed in the dimeric GLP-1 construct #217 (having GLP-1(7-37) as component (I) and as component (III)) both in hTERT-MSC and in HEK293 cells.

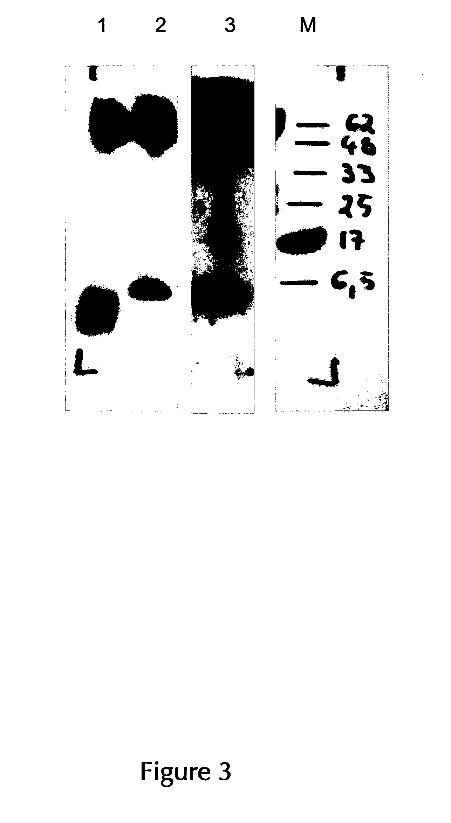

- **Fig. 3:**: shows a Western Blot Analysis of a cell culture supernatant from GLP-1 secreting cells (see also Example 3). Lane 1: 100 ng synthetic GLP-1 (7-37) dissolved in supernatant of mock transfected hTERT-MSC cells; Lane 2: supernatant of hTERT-MSC cells (clone 79TM217/13) secreting dimeric GLP-1 from construct #217; Lane 3: supernatant of AtT20 cells (clone 81-A-217/3) secreting dimeric GLP-1 from construct #217; Lane M: prestained protein marker [kDa]). The results show that peptides as defined herein containing GLP-1 (7-37) and a C-terminal appendix (2 and 3 in Fig. 3) are secreted from the transfected cell lines and can be detected using an anti-GLP-1 antibody, which binds to the mid-molecular epitopes of GLP-1 (7-37).
- **Fig. 4:**: describes plasma stability tests (*in vitro*) carried out with GLP-1 peptides as used according to the present invention. Therefore, HEK293 cells were transiently transfected with constructs (1) #103 GLP-1 (7-37), (2) #317 GLP-1 (7-37)-IP2-extended with 11 AA and (3) #217 GLP-1(7-37)-IP2-GLP-1(7- 37). HEK293 cells are effective hosts for the gene construct (see also Example 4).
- **Fig. 5:**: describes a plasma stability kinetic (*in vitro*) carried out with supernatant of stably transfected hTERT-MSC cell clone 79TM217/18K5 secreting GLP-1 peptide CM1 produced by construct #217 GLP-1(7-37)-IP2-GLP-1(7-37) and synthetic GLP-1 (7-37) as control. The results are obtained from three independent experiments. Active GLP-1 was measured using the GLP-1 (active) ELISA (Linco).

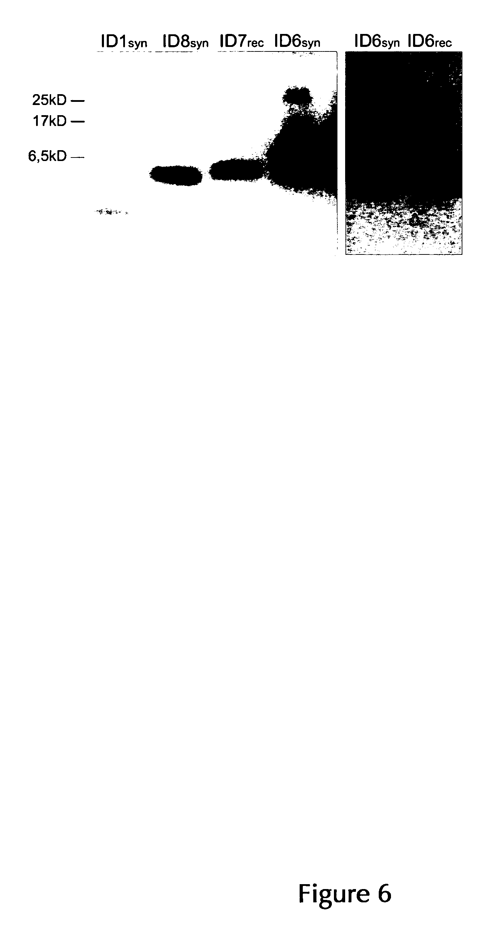

- **Fig. 6:**: shows a Western Blot for the peptides indicated below. The following values are given: SEQ ID NO: 1 (ID1syn) corresponds to GLP-1 (7-37), 31 aa, 3,3 kD; SEQ ID NO:8 (ID8 syn, CM3) corresponds to GLP-1(7-37)-IP2, 46 aa, 5,1 kD; SEQ ID NO: 7 (ID7rec, CM2) corresponds to GLP-1(7-37)-IP2-RR-GLP2, 83 aa, 9,4 kD; SEQ ID NO: 6 (ID6syn, CM1) corresponds to GLP-1(7-37)-IP2-RR-GLP1(7-37), 79 aa, 8,7 kD (see also Example 5).
- **Fig. 7:**: illustrates dose response curves for GLP-1 receptor mediated cAMP increase in the bioassay cell line 11CHO349/18. Stimulation was done with serially diluted conditioned medium of 79TM217/18K5 cells secreting CM1 produced by construct #217 GLP-1(7-37)-IP2-GLP-1(7-37). No detectable cAMP response was found in the parental hMSC-TERT cell line. The graph was prepared from five independent experiments. The peptide dose that produces a half maximal effect (ED50) in the cAMP bioassay has been determined to be 353 pM (see also Example 6).
- **Fig. 8:**: depicts an exemplary vector used for transient and stable gene expression. The vector consists of two separate transcription units, one for the gene of interest (GOI) and one for the fusion of the suicide gene HSV thymidine kinase and the resistance gene blasticidin. For the first transcription unit, the human ubiquitin B promoter was used, and for the second transcription unit the human ferritin promoter was used (see also Example 9).

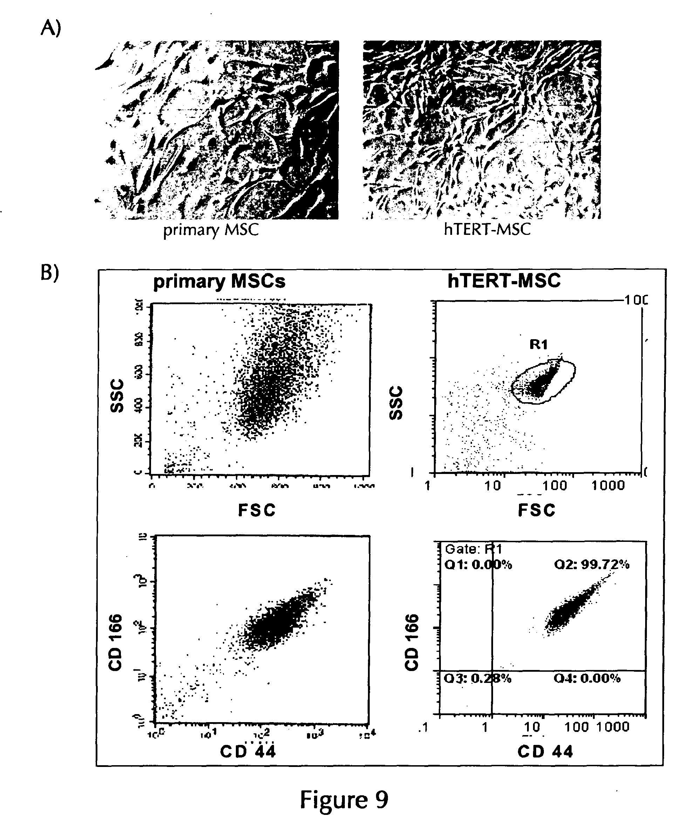

- **Fig. 9:**: illustrates characterization of cells used for (spherical) microcapsules as defined herein, after immortalising the cells in advance. As may be seen from Fig. 9 A, immortalised cells are still able to differentiate into adipocytes, osteocytes and chondrocytes as their non-immortalised counterparts (left, right). Immortalised cells have fibroblastic morphology and are more homogeneous regarding size and granularity as the mortal MSCs as shown by flow cytometry e.g. using CD 44 and CD166 epitope markers which are characteristic for the primary cells used here. Immortalised cells express the same CD markers as their non immortalised counterparts (see Figure 9B).
- **Figs. 10:**: shows the anti-apoptotic efficacy of the C-terminal elongated GLP-1 analogue CM1. Apoptosis is induced in RIN-5F cells by addition of the protein biosynthesis inhibitor cycloheximid (CHX) in a final of 10µg/ml and 100µg/ml respectively. The presence of different concentrations of the recombinantly in E. coli produced dimeric GLP-1 fusion peptide CM1 result in a significant (p<0.01) increase of cell viability, which is quantified after an incubation period of 24 hours.

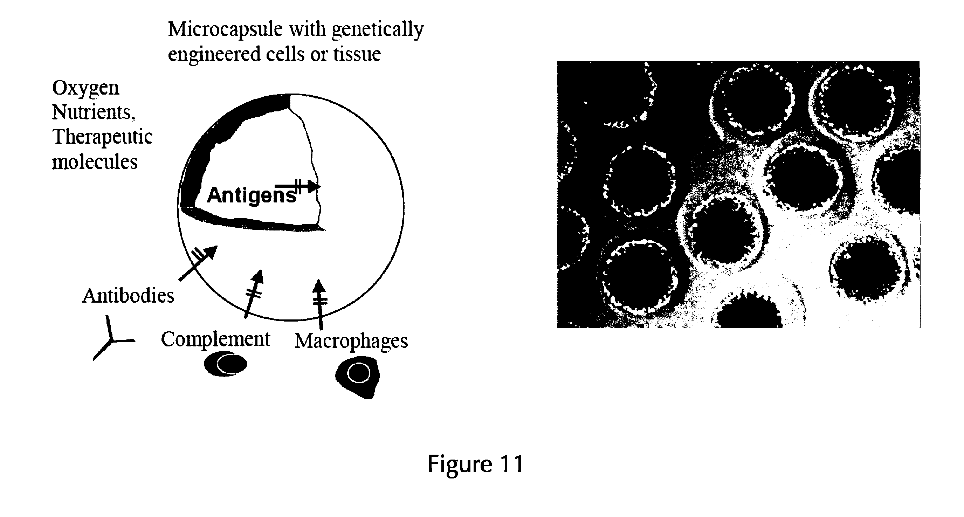

- **Fig. 11:**: is a schematic diagram of the inventive concept using (spherical) microcapsules encoding and secreting GLP-1 as utilized in the treatment of eye diseases, particular macular degeneration (MD). Cells, e.g. mesenchymal stem cells, mesenchymal stromal cells or allogeneic cells are encapsulated in a thin selectively permeable alginate matrix forming (spherical) microcapsules encoding and secreting GLP-1. The alginate matrix is permeable for oxygen and nutrients supplying the encapsulated cells, as well as for GLP-1 or the GLP-1 fusion peptide encoded and secreted by the cells. On the other hand, cells and components of the immune system cannot pass this barrier as depicted above. *Left:* schematic diagram of the inventive concept using (spherical) microcapsules encoding and secreting GLP-1. The cell containing corebead (cream-coloured) is surrounded by a layer of pure alginate (grey) *Right:* (spherical) microcapsules encoding and secreting GLP-1 *in vitro.*
- **Fig. 12**: shows the body weight development of rabbits implanted with GLP-1 and Endostatin encoding and secreting microcapsules according to the present invention (herein also termed GLP-1 and Endostatin Cell Beads).
- **Fig. 13:**: shows explanted beads from the right eye on day 3 (CB-087 GLP-1 CellBeads).
- **Fig. 14:**: shows explanted beads from the left eye on day 3 (CB-102 Endostatin CellBeads).
- **Fig. 15:**: shows explanted beads from the right eye on day 14 (CB-087 GLP-1 CellBeads).
- **Fig. 16:**: shows explanted beads from the left eye on day 14 (CB-102 Endostatin CellBeads).
- **Fig. 17:**: shows explanted beads from the right eye on day 56 (CB-087 GLP-1 CellBeads) (PJ: propidium iodate).
- **Fig. 18:**: shows explanted beads from the left eye on day 56 (CB-102 Endostatin CellBeads) (PJ: propidium iodate).
- **Fig. 19:**: shows GLP-1 Concentrations in Vitreous Humour at Days 3, 14 and 56.
- **Fig. 20:**: shows GLP-1 Concentrations in Aqueous Humour up to Day 56.
- **Fig. 21:**: shows endostatin concentrations in aqueous humour up to day 56
- **Fig. 22:**: shows endostatin concentrations in vitreous humour on days 3, 14 and 56
- **Fig. 23:**: shows TUNEL stain on retinas derived from GLP-1 and Endostatin CellBead treated rabbits.
- **Fig. 24:**: shows TUNEL stain on retinas derived from GLP-1 and Endostatin CellBead treated rabbits.
- **Fig. 25:**: shows TUNEL stain on retinas derived from GLP-1 and Endostatin CellBead treated rabbits.
- **Fig. 26:**: shows TUNEL stain on retinas derived from GLP-1 and Endostatin CellBeads treated rabbits - replicate analysis.
- **Fig. 27:**: shows Auto-fluorescence of retinas derived from GLP-1 and Endostatin CellBeads treated rabbits - replicate analysis.

### Examples

The invention is illustrated further in the accompanying examples. However, it is not intended to limit the scope of the invention to the content of the Examples as shown in the following.

### Example 1

### Creation ofgenetic constructs

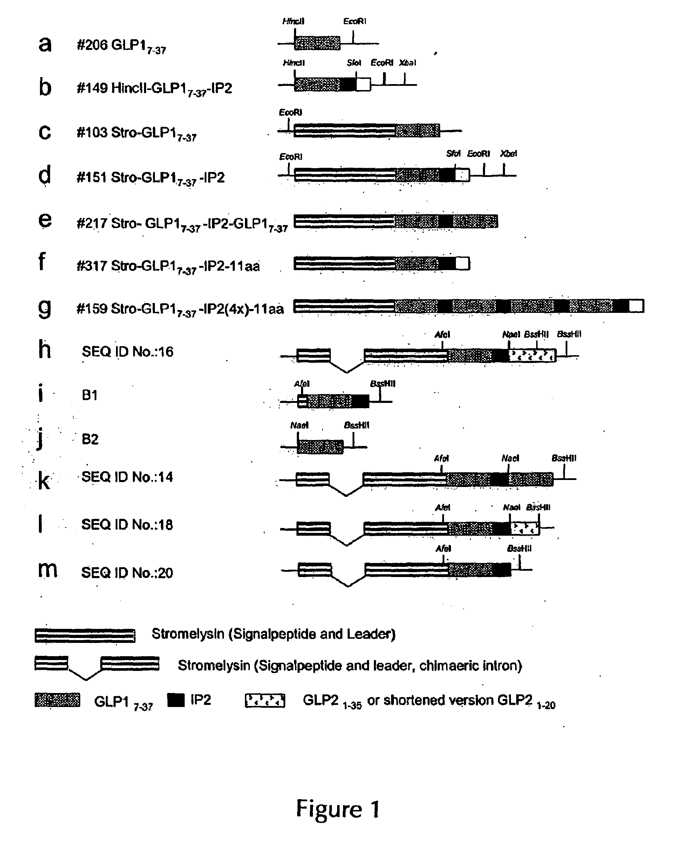

The coding sequence for GLP-1 (7-37) cDNA was synthesized synthetically, in a sequence including Hincll and EcoRI sites as indicated in Fig. 1a. Separately the cDNA illustrated in Fig. 1b was synthesized, including the coding sequences for GLP-1(7-37), IP2 and restriction sites for *Sfo*I, *Eco*RI and *Xba*I*,* as illustrated in Fig. 1b. To direct GLP-1 to the secretory pathway, the heterologous signal sequence of stromelysin 3 (Acc. No. NM_005940) was used. Therefore the cDNA, encoding stromelysin signal and leader sequence was reverse transcriptase PCR amplified from human RNA, and used with the construct of Fig. 1a or Fig. 1b to form the construct shown in Fig. 1c and Fig. 1d, respectively.

The *Hinc*II/*Eco*RI fragment of the Fig. 1a construct is cloned into the SfoI site of the sequence of Fig. 1d to form the construct Fig. 1e. Similarly, the *Eco*RI fragment of Fig. 1d is cloned into the *Eco*RI site of an eukaryotic expression plasmid, to produce the construct shown in Fig. 1f. To form the construct shown in Fig. 1g, the *Hinc*II/*Xba*I fragment of the construct shown in Fig. 1b is repetitively cloned into the *Sfo*I*lXba*I site of the construct shown in Fig. 1d. Figure 1h shows a synthesized, codon optimized sequence encoding the stromelysin leader and signal sequences interrupted by a shortened endogenous intron sequence, fused to sequences encoding human GLP-1(7-37), IP2 and GLP-2(1-35). The DNA sequence of the construct Fig. 1h is SEQ ID NO: 16, while SEQ ID NO: 15 also shows the sequence of the translated peptide.

Also synthesized are the sequences in Figs 1i and 1j. These are then used to form the construct in Fig. 1k, by cloning the *Nae*I/*Bss*HII fragment of Fig. 1j into the NaeI/BssHII linearised sequence of Fig. 1h. The DNA sequence of the construct Fig. 1k is SEQ ID NO: 14, while SEQ ID NO: 13 also shows the sequence of the translated peptide. The construct of Fig. 1l is formed by *Bss*HII digest and religation of the sequence of Fig. 1h. The DNA sequence of the construct Fig. 1l is SEQ ID NO: 18, while SEQ ID NO: 17 also shows the sequence of the translated peptide. The construct of Fig. 1 m is formed by cloning the AfeI/BssHII fragment of the sequence of Fig. 1i into the *Afe*I*lBss*HII linearised sequence of Fig. 1h. The DNA sequence of the construct Fig. 1 m is SEQ ID NO: 20, while SEQ ID NO: 19 also shows the sequence of the translated peptide.

The above constructs may be made by a person skilled in the art using routine techniques.

### Example 2

### Transfection, clonal selection and GLP 1 expression ofmammalian cells

Source of the cells: HEK293 (human embryonic kidney cell line, # ACC 305, DSMZ Cell Culture Collection, Germany), AtT20 (Mouse LAF1 pituitary gland tumour cell line, #87021902, European Cell Culture Collection, UK), hTERT-MSC cells are generated and provided by Prof. Kassem, University Hospital of Odense, Denmark.

For transfection of 10⁶ cells 0.5-2 µg plasmid DNA with different GLP-1 constructs was used. The constructs were generated as described in Example 1. HEK293 cells were transfected by standard calcium phosphate co-precipitation method as described in Current Protocols in Molecular Biology (Ausubel et al. 1994ff Harvard Medical School Vol2., Unit 9.1). AtT20 cells were transfected using FuGene (Roche) as described in Current Protocols in Molecular Biology (Ausubel et. al. 1994ff, Harvard Medical School Vol 2., Unit 9.4). Transfection of hTERT-MSC cells was performed using the Nucleofector technology (Amaxa), a non-viral method which is based on the combination of electrical parameters and cell-type specific solutions. Using the Nucleofector device (program C17) and the Nucleofector solution VPE-1001 transfection efficiencies >60% have been achieved. 48 hours after transfection selection of cell clones with stable integration of DNA into the chromosome was performed by adding the selective agent blasticidin (2 µg/ml) into the culture medium. 12-15 days later, stable transfected cell clones could be isolated and expanded for characterization.

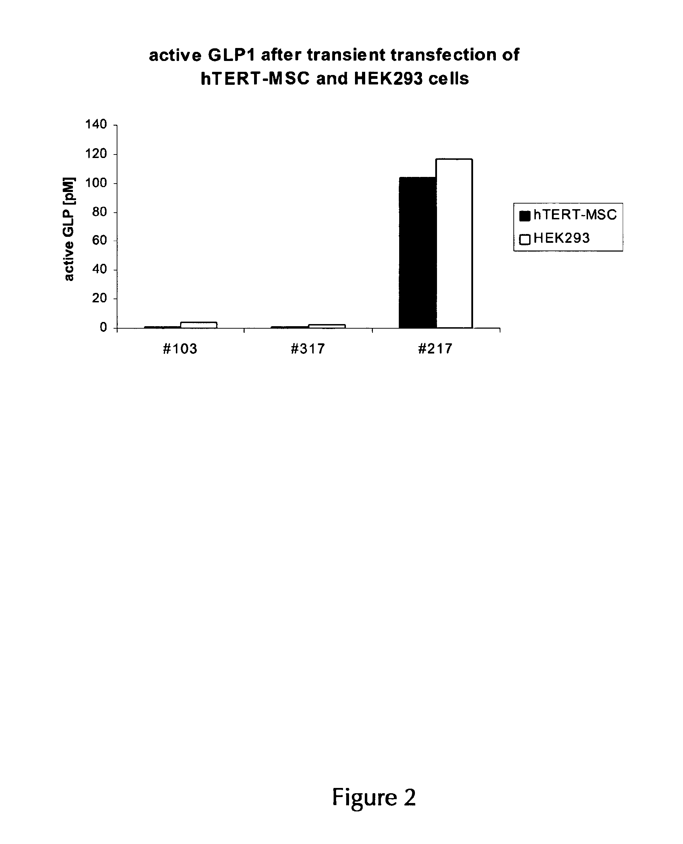

Transient expression of different GLP-1 constructs was measured in hTERT-MSC and HEK293 cells. Whereas only marginal active GLP-1 level can be found in the monomeric GLP-1 constructs #103 and #317 (having just one copy of GLP-1 (7-37) an enormous gain in expression can be found in the dimeric GLP-1 construct #217 (having GLP-1 (7-37) as component (I) and as component (III)) both in hTERT-MSC and in HEK293 cells. Results are summarized in Figure 2. An elongation of the construct to the GLP-1 construct #159 (having four IP2 copies as component (II)) results in no further significant increase (not shown). After transfection of hTERT-MSC cells with different constructs clones were selected, which stably express GLP-1. The expression levels are shown in Table 1.

**Table 1**

| **construct** | **cell clone** | **active GLP per 10⁶ cells and** **hour [pmol]** |
|---|---|---|
| #103 GLP1₍₇₋₃₇₎ | 49TM113/13 | 0.4 |
| #317 GLP1₍₇₋₃₇₎-IP2-11aa | 71TM169/1 | 0.6 |
| #217 GLP1₍₇₋₃₇₎-IP2-GLP1 ₍₇₋₃₇₎ | 79TM217/13 | 2.7 |

### Example 3

### Western Blot Analysis of GLP-1 peptides, secreted from mammalian cells

Cell culture supernatant from GLP-1 secreting cells was separated in a 10%-20% gradient SDS PAGE (120V, 90 minutes) and transferred to a PVDF membrane (lmmobilon-P Membrane 0.45 µm Millipore IPVH 00010) by semi-dry blotting (2.0 mA/cm2, 60 minutes). After methanol fixation and blocking (3% (w:v) BSA, 0.1% (v:v) Tween-20 in TBS) the membrane was immunoblotted with 1 µg/ml anti-GLP-1 antibody (HYB 147-12, Antibodyshop) at 4°C o/n. After washing and incubation with 0.02 µg/ml detection antibody (Anti Mouse IgG, HRP conjugated, Perkin Elmer PC 2855-1197) at RT for 4 hours, chemiluminescence detection reveals the location of the protein.

Western Blot Analysis is shown in Figure 3 (1: 100 ng synthetic GLP-1(7-37) dissolved in supernatant of mock transfected hTERT-MSC cells, 2: supernatant of hTERT-MSC cells (clone 79TM217/13) secreting dimeric GLP-1 from construct #217, 3: supernatant of AtT20 cells (clone 81-A-217/3) secreting dimeric GLP-1 from construct #217; M: prestained protein marker [kDa]). The results show that peptides containing GLP-1 (7-37) and a C-terminal appendix (2 and 3 in Fig. 3) are secreted from the transfected cell lines and can be detected using an anti-GLP-1 antibody, which binds to the mid-molecular epitopes of GLP-1(7-37).

### Example 4

### In vitro plasma stability of GLP-1 peptides secreted from human cells

HEK293 and hTERT-MSC cells were transfected with constructs, encoding the heterologous stromelysin signal sequence, which is linked to GLP-1 variants encoding the following peptides:
1: GLP-1(7-37) (construct #103)
2: GLP-1(7-37)-IP2-extended with 11 AA (construct #317)
3: GLP1(7-37)-IP2-GLP1(7-37) (construct #217)

Cell culture supern'cont, containing GLP-1 peptides secreted from cells or synthetic GLP-1 (7-37) (Bachem) was incubated with human lymphocyte enriched plasma containing dipeptidylpeptidase activity at 37°C and 5% CO₂, for 3 or additionaly 6 and 9 hours. Synthetic GLP-1(7-37) in supernatant from mock transfected cells was used as a positive control for DPP-IV activity, which was shown to be inhibited by addition of a DPP-IV inhibitor (#DPP4, Biotrend). Active GLP was measured using the GLP-1 (Active) ELISA (#EGLP-35K, Biotrend), using an antibody which binds to the N-terminal epitope of GLP-1(7-37) discriminating the DPP-IV degraded, inactive GLP-1(9-37) peptide.

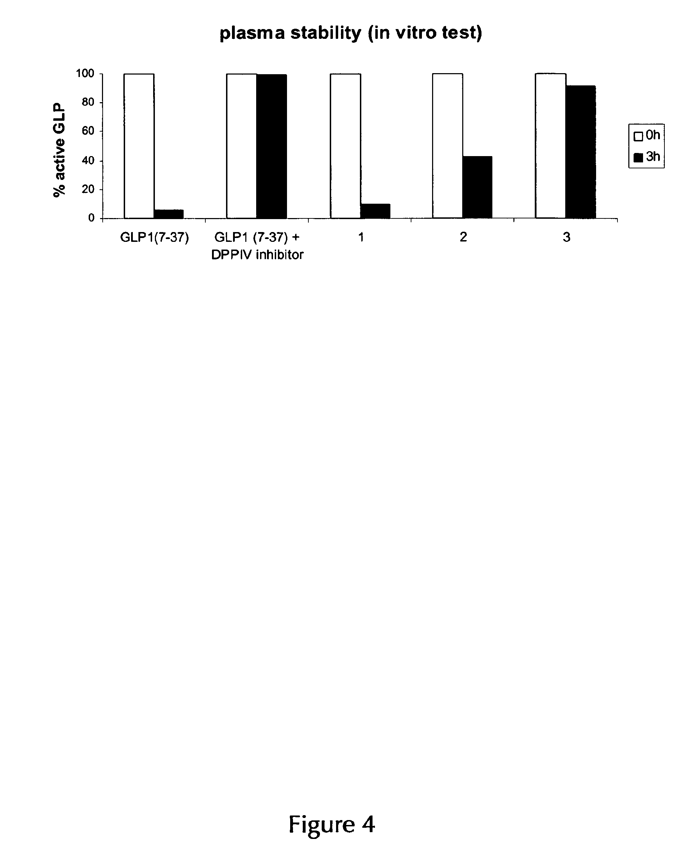

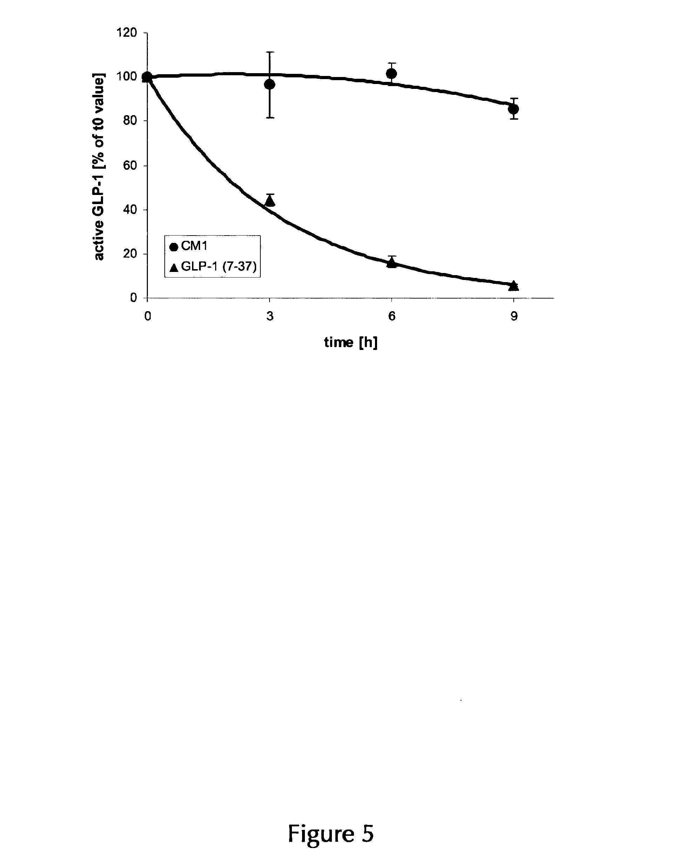

The results are shown in Figures 4 (HEK293 cells) and 5 (hTERT-MSC cells). HEK293 and hTERT-MSC cells are both effective hosts for the gene construct. The numbering of the results for the transfected cells is 1: supernatant of cells secreting GLP-1(7-37) from construct #103, 2: supernatant of cells secreting GLP-1 extended by IP2 and 11 aminoacids from construct #317, 3: supernatant of cells secreting dimeric GLP-1 from construct #217. While construct 1 produces wild type GLP-1 which is inactivated by DPP-IV in a similar way to synthetic GLP-1, the C-terminally elongated GLP-1 forms (2 and 3 in Figure 4, 3 in Figure 5) are more resistant to degradation. The C-terminal extended GLP-1 peptides are significantly stabilized in human plasma *in vitro.* The peptide with the dimeric GLP-1 sequence (3) is nearly fully stabilized to DPP-IV degradation *in vitro.*

### Example 5

### In vitro bioactivity of GLP-1 peptides measured by cAMP release

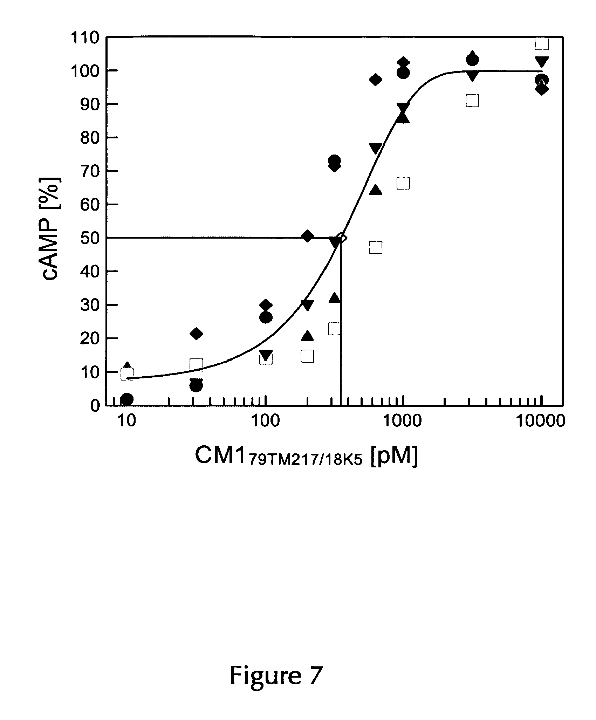

GLP-1 (7-37) exerts its biological actions through the seven-transmembrane-spanning, G protein coupled GLP-1 receptor, which leads to activation of protein kinase A signalling through the second messenger cyclic AMP. To ensure that the C terminal elongation of CM1 does not interfere with GLP-1's mode of action, CM1 bioactivity was quantified in an in vitro bioassay, which determines cAMP increase in a GLP-1 receptor expressing cell line after incubation with different concentrations of the peptide. The GLP-1 receptor expressing cell line used for the study (clone 111CH0349/18) is a CHO (chinese hamster ovary) cell line stably transfected with the human GLP-1 receptor. The dose response curves for CM1 produced in the 79TM217/18K5 cells outline the bioactivity of the peptide is shown in figure 7. The peptide dose that produces a half maximal effect (ED50) in the cAMP bioassay has been determined to be 353 pM.

### Example 6

### In vitro human plasma stability of GLP-1 ^{CM} peptides

Synthetic GLP-1 peptides (SEQ ID NO:1_{syn}, SEQ ID NO:65_{syn}, SEQ ID NO:7_{rec}, SEQ ID NO:8_{syn}) were incubated at concentrations of 20 ng/ml with human plasma at 37°C and 5% CO₂ for 3 hours. Dipeptidylpeptidase activity of the plasma was inhibited by a DPP-IV inhibitor (#DPP4, Biotrend). Active GLP was measured using the GLP-1 (Active) ELISA (#EGLP-35K, Biotrend).

In contrast to the native GLP-l ₍₇₋₃₇₎ (SEQ ID NO:1) the C-terminal elongated GLP-1 peptides SEQ ID NO:6, SEQ ID NO:7, and SEQ ID NO:8 are significantly stabilized in human plasma *in vitro* (Fig. 7). As control (on the right hand side) the results obtained for experiments with addition of DPP-IV inhibitor are shown. GLP-1 activity is completely maintained in these control experiments.

### Example 7

### Plasmid creation

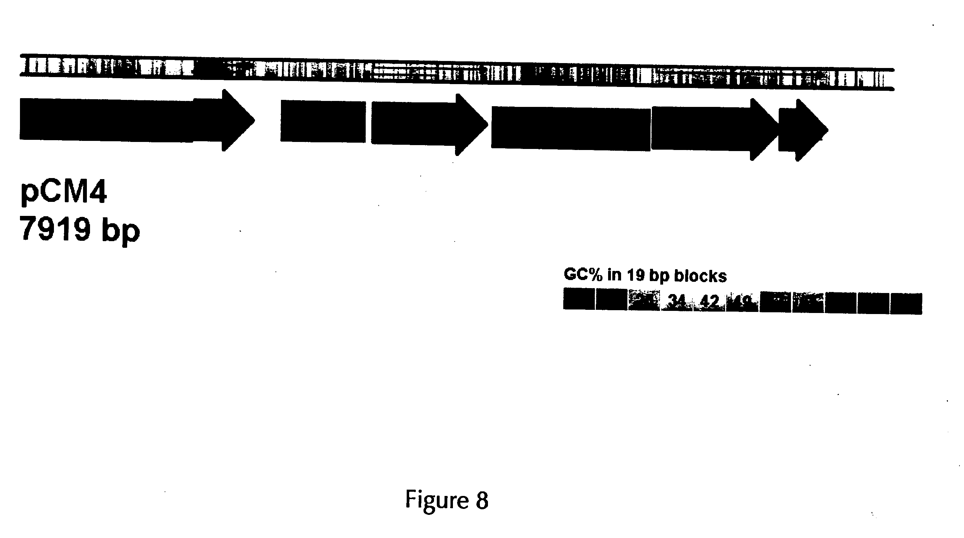

The vector for transient and stable gene expression consists of two separate transcription units, one for the gene of interest (GOI) and one for the fusion of the suicide gene HSV thymidine kinase and the resistance gene blasticidin. For the first transcription unit, the human ubiquitin B promoter was used, and for the second transcription unit the human ferritin promoter was used. The plasmid is based on plasmid pCM4, having 7,919 base pairs, shown schematically in Figure 8.

As shown in Figure 8, transcription unit 1, comprises the following components:
- CMVenh:: immediate early enhancer human cytomegalovirus
- ubiB human:: ubiquitin promoter B
- Stro-GLP:: fusion gene, coding for signal peptide and leader sequence of stromelysin and GLPI constructs
- ori pMBI:: E coli minimal origin of replication.
- Hygro:: hygromycin B resistance gene.
Transcription unit 2,
- SV 40 enh:: SV40 enhancer.
- FerH:: Human ferritin H promoter combined with 5'UTR of the murine EFI gene.
- Tk-bla:: fusion gene coding for herpes simplex virus type 1 thymidine kinase and blasticidine resistance gene.

For transient expression the circular plasmid was used. For the selection of stable expressing cell clones, the plasmid was linearised and bacterial sequences (pMB1 origin and hygromycin gene) eliminated.

### Example 8

### Production of mesenchymal stem cell lines or mesenchymal stromal cell lines (MSC).

The *mesenchymal stem cell line* was generated by Prof. Kassem, University Hospital of Odense, Denmark (published in Simonsen et al., 2002, Nature Biotechnology 20m, 592-596) according to following criteria:

### Origin

The production cell line consists of mesenchymal stem cells (MSC), isolated from bone marrow aspirates of a healthy male donor (age 33).

### Immortalisation

Cells were immortalised by introduction of the coding sequence of the telomerase reverse transcriptase. Retroviral transduction was performed by packaging the GCsam retroviral vector in which the expression of the transgene is driven by the Moloney murine leukaemia virus long terminal repeat in PG13. Transduction was performed on day 9 (PDL 12) of culture. The cell line has so far been cultivated until population doubling level (PDL) of 260.

The insertion locus was tested by fluorescence *in situ* hybridization and southern blot. There is only one insertion locus of ecotopic hTERT on chromosome 5 (5q23-31). Analysis was performed at PDL 186. Giemsa banding and comparative genomic hybridization revealed that hMSC-TERT did not develop any numerical or structural chromosomal abnormalities at PDL 96 and maintained a normal diploid male karyotype. Tumourigeneity was tested in immunodeficient mice after subcutaneous implantation for six months and was found negative for PDL 80.

### Flow cytometry (FACS) analysis

Cells were cultured in standard growth medium to 80% confluence. Cells were trypsinised and assayed for size and granularity by FACScan flow cytometer (Becton-Dickinson). For surface marker studies typsinised cells were stained with antibodies directly conjugated to a fluorescent dye (FITC-conjugated mouse anti human CD44 monoclonal antibody, #CBL154F, Cymbus Biotechnology; phycoerythrin-conjugated mouse anti human CD166 monoclonal antibody, #559263, BD Pharmingen) for 30 min on ice. Samples were washed and fixed with 1% of paraformaldehyde until analysis with FACScan (Becton-Dickinson).

### Characterization

immortalised cells are still able to differentiate into adipocytes, osteocytes and chondrocytes as their non-immortalised counterparts (see Figure 9A). Immortalised cells have fibroblastic morphology and are more homogeneous regarding size and granularity as the mortal MSCs as shown by flow cytometry e.g. using CD 44 and CD166 epitope markers which are characteristic of the primary cells used here. Immortalised cells express the same CD markers as their non immortalised counterparts (see Figure 9B).

### Cultivation

- Serum containing medium:: 7% Earles MEM
10% FCS
2mM L-Glutamine
1 mM Sodiumpyruvate
100 U/ml Penicillin
0.1 mg/ml Streptomycin

The population doubling is between 26 and 30 hours.

### Transfection and clonal selection

For transefection of 10⁶ cells 0.5-2µg plasmid DNA with different GLP1 constructs was used. HEK293 cells were transfected by standard calcium phosphate co-precipitation method. AtT20 cells were transfected using FuGene (Roche).

Transfection of hTERT-MSC cells was performed using the Nucleofector technology (amaxa), a non-viral method which is based on the combination of electrical parameters and cell-type specific solutions. Using the Nucleofector device (programme C17) and the Nucleofetor solutionVPE-1001 transfection efficiencies >60% have been achieved.

48 hours after transfection selection of cell clones with stable integration of DNA into the chromosome was performed by adding the selective agent blasticidin (2µg/ml) into the culture medium. 12-15 days later, stable transfected cell clones could be isolated and expanded for characterization.

### Expression

Transient expression of different GLP constructs was measured in hTERT-MSC and HEK293 cells. An active GLP1 level can be found in the monomeric GLP1 constructs #103 (Stro-GLP1₍₇₋₃₇₎) and #317 (Stro-GLP1₍₇₋₃₇₎-IP2-extended with 11 aa) and an enormous gain in expression can be found in the dimeric GLP1 construct #217 (Stro-GLP1₍₇₋₃₇₎-IP2-GLP1₍₇₋₃₇₎) both in hTERT-MSC and in HEK293 cells. An elongation of construct #317 to the tetrameric GLP1 construct #159 (Stro-GLP1₍₇₋₃₇₎-IP2 (4x)-11aa) results in an similar activity (see also above Figure 2). After transfection of hTERT-MSC cells with different constructs clones were selected, which stably express GLP1 (see above Figures 4 and 5, Example 4).

### Example 9

### Encapsulation

The cultivated cells to be encapsulated were washed with PBS (PAA, Austria) and separated using trypsin/EDTA (PAA, Austria). The reaction was quickly stopped using medium (dependent on cell type, for example RPMI, PAA, Austria) and the cell suspension centrifuged off (8 min at 1,200 rpm) The pellet was resuspended in PBS and the cell count determined. The desired quantity of 4 × 10⁷ cells was centrifuged off again (8 min at 1,200 rpm). The PBS was then completely removed by suction and the 50 µl pellet was resuspended without air bubbles in 50 µl 0.9% saline buffered by 5 mM I-histidine to a pH of 7.4. This cell suspension was taken up in 900 µl of 1.5 - 1.7 % (w/v) sodium alginate solution (an alginate with a viscosity of approximately 5 mPa.s of 0.2 % (w/v) aqueous solution at room temperature was used).

To mix the resuspended cells with the alginate solution, the solution was drawn up in a 1 ml syringe with cannulas and homogeneously mixed with the cells by way of repeated slow drawing up and drawing off. A cell concentration of 4 x 10⁷ cells/ml resulted.

For producing the microcapsules with a diameter of about 200 µm, a cannula with an internal diameter of 120 µm was used in an air-charged spray nozzle. An air ring with an opening of 2.0 mm was screwed over the inner cannula. The device is an adapted version of the device described in WO 00/09566. The homogeneous cell/alginate solution mixture was dripped through the described spray nozzle. For this purpose, the 1 ml syringe containing the mixture was placed on the cannula by means of a luer connector. The cell/alginate solution mixture was pressed through the cannula at a speed of 50 µl/min. The airflow was conveyed though the outer air ring at a speed of 2.5 l/min. The resulting microcapsules precipitated into a barium-containing precipitation bath (20 mM BaCl, 5 mM L-histidine, 124 mM NaCl, pH 7.0 ± 0.1, 290 mOsmol ± 3) which was constructed approximately 10 cm below the spray nozzle. After a dwell time of 5 min in the barium-containing precipitation bath the microcapsules were washed five times with 20 ml PBS in each case.

500 µl of the single-layer microcapsules were then taken up in 500 µl of a 1.5 - 1.7 % (w/v) alginate solution the same as used for the core, above and homogeneously mixed. This suspension was taken up in a 1 ml syringe and connected by means of a luer connector to the inner channel (internal diameter: 200 µm) of the spray nozzle and pressed at a speed of 50 µl/min therethrough. A 5 ml syringe with a 1.5 - 1.7 % alginate solution was connected by means of a luer connector to the second inner channel (internal diameter: 700 µm) and pressed there through at a speed of 250 µl/min. The airflow was conveyed through the outer air ring at a speed of 2.9 l/min. The resultant microcapsules precipitated into a barium-containing precipitation bath (20 mM BaCl, 5 mM L-histidine, 124 mM Nail pH 7.0 ± 0.1, 290 mOsmol ± 3) which is constructed approximately 10 cm below the spray nozzle. After a dwell time of 5 min in the barium-containing precipitation bath, the microcapsules were washed four times with 20 ml PBS in each case and once with medium. Two-layer microcapsules with a total diameter of approximately 180 - 200 µm (including the alginate layer) were produced by this process, wherein the diameter of the inner, cell containing core is 120 - 150 µm.
The concentration of cell in the core is about 4 × 10⁷ cell/ml alginate. This results in (spherical) microcapsules (CellBeads) with a bead volume of 0.002 - 0.004 µl containing approximately 100 cells per bead. A (spherical) microcapsule encoding and secreting GLP-1 produces on average 0.2 fmol active GLP-1 per hour.

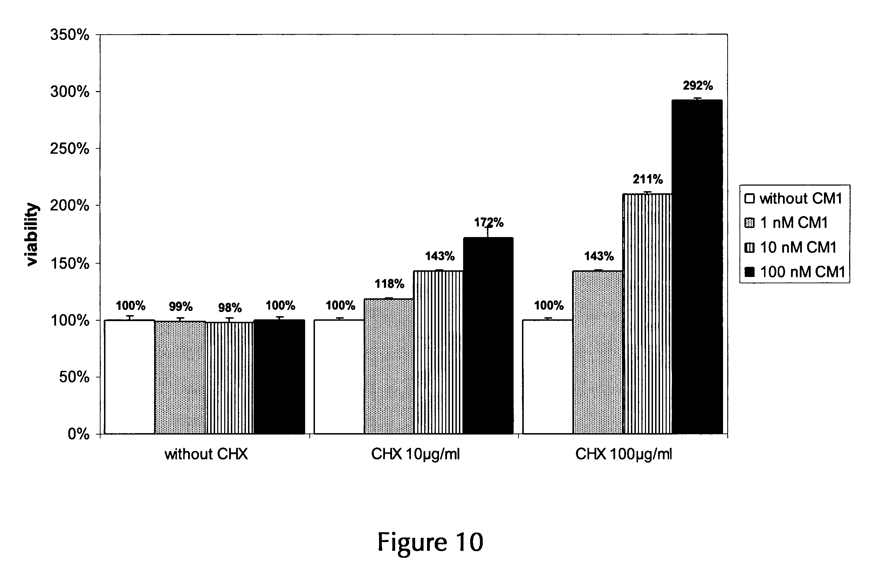

A micrograph of Cellbeads containing encapsulated GLP-1 secreting hTERT-MSC cells in the core are shown in Figure 10.

For producing the microcapsules with a diameter of about 600 µm, a cannula with an internal diameter of 400 µm was used in an air-charged three-channel spray nozzle for the inner channel. The cannula was fixed in an outer nozzle with an internal diameter of 700 µm. An air ring with an opening of 1.5 mm was screwed over the two inner cannulas. The device is an adapted version of the device described in WO 00/09566. The homogeneous cell/alginate solution mixture was dripped through the described spray nozzle. For this purpose, the 1 ml syringe containing the mixture was placed on the inner channel by means of a luer connector. The cell/alginate solution mixture was pressed through the inner channel at a speed of 300 µl/min. The airflow was conveyed though the outer air ring at a speed of 2.5 l/min. The resulting microcapsules precipitated into a barium-containing precipitation bath (20 mM BaCl, 5 mM L-histidine, 124 mM NaCl, pH 7.0 ± 0.1, 290 mOsmol ± 3) which was constructed approximately 10 cm below the spray nozzle. After a dwell time of 5 min in the barium-containing precipitation bath the microcapsules were washed five times with 20 ml PBS in each case.

500 µl of the single-layer microcapsules were then taken up in 500 µl of a 0.8 % (w/v) alginate solution the same as used for the core, above and homogeneously mixed. This suspension was taken up in a 1 ml syringe and connected by means of a luer connector to the inner channel (internal diameter: 400 µm) of the spray nozzle and pressed at a speed of 50 µl/min therethrough. A 5 ml syringe with a 0.8 % alginate solution was connected by means of a luer connector to the second inner channel (internal diameter: 700 µm) and pressed there through at a speed of 250 µl/min. The airflow was conveyed through the outer air ring at a speed of 2.9 l/min. The resultant microcapsules precipitated into a barium-containing precipitation bath (20 mM BaCl, 5 mM L-histidine, 124 mM NaCl, pH 7.0 l 0.1, 290 mOsmol ± 3) which is constructed approximately 10 cm below the spray nozzle. After a dwell time of 5 min in the barium-containing precipitation bath, the microcapsules were washed four times with 20 ml PBS in each case and once with medium. Two-layer microcapsules with a total diameter of approximately 600 µm ± 100 µm (including the alginate layer) were produced by this process, wherein the diameter of the inner, cell containing core is 380µm ± 20 µm. The concentration of cell in the core is about 2-3 x 10⁷ cell/ml alginate. This results in inventive spherical microcapsules (CellBeads) with a bead volume of 0,065 - 0,180 µl containing approximately 1000 cells per bead. A CellBead with GLP-1 secreting cells produces on average 5 fmol active GLP per hour.

### Example 10

### Anti-apoptotic efficacy of C-terminally elongated GLP-1

The cytoprotecitve efficacy of the C-terminally elongated GLP-1 analougue CM1 was tested *in vitro* using the rat insulinoma cell line Rin-5F. 40.000 Rin-5F cells were seeded per 96 well and cultivated for 2 days in RPMI supplemented with 1 % L-Glutamin and 10 % fetal calf serum. Apoptosis is induced after change to serumfree conditions (RPMI supplemented with 1 % L-Glutamin) by addition of the protein biosynthesis inhibitor cycloheximid (CHX) in the presence of different concentrations of the recombinantly in E. coli produced dimeric GLP-1 fusion peptide CM1. After 24 hours cell viability is quantified using AlamarBlue. A significant anti-apoptotic effect (p<0.01) was observed already in the presence of 1 nM GLP-1 analouge CM1. The results are given in Figure 10.

### Example 11

### Cytokine profile of the GLP-1 producing h TER T-MSC cell line

To investigate GLP-1 independent, cytoprotective effects, the GLP-1 secreting cell line 79TM217/18K5 cell line was examined for the secretion of cytokines, chemokines and growth factors.

The cell line originates from a human stromal cell and therefore secretes a characteristic cytokine profile. A multiplex assay kit (Biosource Cytokine 30-plex) was used for measuring the 30 most abundant human cytokines, chemokines and growth factors simultaneously. No expression was found regarding the cytokines IL-1RA, IL-1β, IL-2, IL-2R, IL-4, IL-5, IL 7, IL-10, IL-12(p40/p70), IL-13, IL-15, IL-17, IP-10, EGF, Eotaxin, FGF-basic, IFN-a, IFNγ, GM CSF, G-CSF, HGF, MIG, MIP-b, MIP-1α, RANTES and TNFα (detection limit of each analyte 20 pg per 10⁵ cells and 24h). The cytokines, which are expressed at detctable levels are summarized in table 1.

**Table 1: Expression level of growth factors Vascular endothelial growth factor (VEGF), neurotrophin-3 (NT-3), glial cell line-derived neurotrophic factor (GDNF) and the cytokines Interleukin 6 (IL-6), Interleukin 8 (IL-8) and Monocyte chemotactic protein 1 (MCP-1). The factors have been quantified in cell culture supernatant of the CM1 secreting cell line 79TM217/18K5 using the VEGF ELISA (#ELH-VEGF-001; RayBio), NT-3 ELISA (#TB243, Promega), GDNF ELISA (#TB221, Promega) and the the human IL-6, IL-8 and MCP-1 ELISA Kits (RayBio).**

| Growth factor / Cytokine [pg / 10⁶ cells and hour] | 79TM217/18K5 |
|---|---|
| VEGF | 973.0 ± 78.3 |
| NT-3 | 20.9 ± 3.5 |
| GDNF | 10.7 ± 1.5 |
| IL-6 | 378.4 ± 4.0 |
| IL-8 | 3608.7 ± 53.8 |
| MCP-1 | 16.8 ± 0.1 |

### Example 12

### Use of Cell Beads for a novel cell-based therapy for eye diseases

### 1. Introduction

As discussed initially, blindness can be caused by loss of retinal tissue including the retinal ganglion cells and retinal pigment epithelium cells, which is the case e.g. in the glaucomas, diabetic retinopathy, retinitis pigmentosa inflammatory and neurodegenerative disorders. In order to protect the retinal cells from further deterioration, repeated injections of neuroprotective agents, like CNTF would be beneficial. Glucagon-like peptide-1 (GLP-1), which has shown to have an anti-apoptotic and neuroprotective effect in intracerebral applications, was surprisingly found to be a promising candidate for such a neuroprotective effect on the retina and was therefore used for the present experiment.

Exudative age-related Macular Degeneration (AMD) is characterized by abnormal blood vessel growth in the eye. The faulty blood vessels leak fluids and blood, which results in vision loss. Endostatin, the cleavage product of collagen XVIII is known to reduce angiogenesis significantly and therefore could be a potential treatment for AMD.

The dry form of agerelate dmacular degeneration is characterized by a degeneration and atrophy of the retinal pigment epithelium und secondary changes in the photroreceptor layer. A neuroprotective therapy to help these cells survive will be highly desirable since dry agee-related macular degeneration is one of the most common ocular disorders leading to vision loss up to visual impairment.

Repeated injections of the drug substance and the associated risk of infections could be avoided, if intravitreal cell implants would be available that continuously produce and secrete these therapeutic proteins.

In the following experiments approximately 100 GLP-1 CellBeads and approximately 100 Endostatin Cell Beads were be implanted into the vitreous cavity of the right and left eye, respectively, of nine rabbits. The aim was to monitor the viability of the encapsulated cells, the CellBead integrity and the production rates of the transgene in the aqueous humour after implantation. These experiments provided first evidence for the suitability of intravitreal Cell Bead implants as a treatment method for retinal atrophy and AMD and further eye diseases.

The following parameters were scheduled for examination:
- Characterization of GLP-1 CellBeads and Endostatin CellBeads approximately 3, 14 and 56 days after implantation in the vitreous cavity
- Integrity of the CellBeads
- Vitality of the encapsulated cells
- GLP-1 fusion peptide and endostatin secretion after explantation
- Quantification of CellBead secreted factors (GLP-1 fusion peptide, endostatin) in the aqueous humour before implantation and at Days 3, 7, 14, 21, 28, 35, 42, 49 and 56
- Histological evaluation of the retinas (HE stain, PAS [optional])
- Apoptosis stain of the retinas (TUNEL)

### 2. Methods and Materials

### 2.1 Test items

### 2.1.1. CLP-1 Cel/Beads

- Batch number: CB-087
- Composition: GLP-1 fusion peptide secreting cell line 79TM217/18K5 encapsulated in sodium alginate as described above
- Safety precautions: Routine hygienic procedures for the handling of genetically modified organisms (bio safety level 1)

### 2.1.2 Endostatin Cel/Beads

- Batch number: CB-102
- Composition: Endostatin secreting cell line 58TM131/3K7 encapsulated in sodium alginate according to the procedure described above for GLP-1 CeIIBeads
- Safety precautions: Routine hygienic procedures for the handling of genetically modified organisms (bio safety level 1)

### 2.1.3. Vehicle

None, no empty CellBeads were implanted. The respective other eye served as control.

### 2.2. Test system

- Test system: New Zealand White Rabbit
- Rationale: The size of the animal, especially the eye is suitable for the intended tests.
- Group allocation: 3 females per time point (Days 3, 14 and 56)
- Total number of animals: 9
- Identification: By unique cage card and corresponding tattoo number
- Acclimatization: Under laboratory conditions after health examination. Only animals without any visual signs of illness were used for the study. Body weight at acclimatization is given in the results section and in the raw data.

### 2.3. Additional material

Dipeptidylpeptidase IV inhibitor (Linco Research)
GLP-1 active ELISA (Linco Research)
Endostatin ELISA (R&D)
Novesine 0.4% eye drops (OmniVision GmbH, Puchheim)
PVP-Jod-AT2.5%(NRF15.13)
Refobacin eye drops (Merck)
Polvidon-lodide solution for disinfection

### 2.4. List of Abbreviations

| | |
|---|---|
| AMD | Age-related Macular Degeneration |
| a.m./p.m. | Ante meridiem/Post meridiem |
| °C | Degree Celsius |
| cm | Centi metre |
| CO₂ | Carbon dioxide |
| CNTF | Ciliary neurotrophic factor |
| DPP4 | Dipeptidylpeptidase 4 |
| fmol | Femto mol |
| G | Gauge |
| GDNF | Glial cell derived neurotrophic factor |
| GLP-1 | Glucagon-like petide-1 |
| HE | Haematoxylin Eosin |
| IL-6 | Interleukin 6 |
| IL-8 | Interleukin 8 |
| MCP-1 | Monocyte chemoattractant protein-1 |
| ml | Milli litre |
| µl | Micro litre |
| µm | Micro metre |
| ng | Nano gram |
| NT-3 | Neurotrophin 3 |
| PAS | Periodic acid-Schiff staining |
| pg | Pico gram |
| PJ | Propidium iodate |
| sec | Second(s) |
| TUNEL | Terminal Transferase dUTP Nick End Labelling |
| VEGF | Vascular endothelial growth factor |

### 2.5. Treatment

Nine rabbits were implanted with 50-100 GLP-1 CellBeads into the intravitreal cavity of the right eye and 50-100 Endostatin Cell Beads into the intravitreal cavity of the left eye. Aqueous humour was sampled from both eyes of the animals before implantation and at Days 3, 7, 14, 21, 28, 35, 42, 49 and 56.
Three animals each were sacrificed at Days 3, 14 and 56 and the implanted CellBeads and retinas examined.

### 2.5.1 Application of the test item

### 2.5.1.1. Preparation of the test item before implantation

Cells encoding and secreting GLP-1 as defined herein and (spherical) microbeads containing these cells (GLP-1 CellBeads^{®}) as well as Cells encoding and secreting the anti-angiogenic factor Endostatin and (spherical) microbeads containing these cells (Endostatin CellBeads^{®}), both for implantation into the eye, are being developed according to good manufacturing practice (GMP) according to the experiments as shown above in previous Examples 1 to 11. The cells, which are encapsulated within the alginate and secrete the GLP-1 fusion peptide are derived from the cell line 79TM217/18K5 (GLP-1 secreting cells) or are derived from the cell line 58TM131/3K7 (Endostatin secreting cells), are defined as the drug substance.

The GLP-1 fusion peptide as expressed by the GLP-1 CellBeads^{®} in this experiment is a dimeric GLP-1 construct, which is arranged in analogy to the native preproglucagon gene. It is a 79 amino acid dimeric GLP-1 / Intervening Peptide 2 (IP-2) / GLP-1 protein with a molecular weight of 8.7 kDa and corresponds to SEQ ID NO: 10 in the accompanied sequence listing. The advantages of this C-terminally elongated GLP-1 fusion peptide in comparison to the native GLP-1 are the higher expression levels and the decreased susceptibility to naturally occurring degradation by dipeptidyl peptidase IV. Bioactivity of the fusion protein is maintained. For safety reasons, the plasmid used allows co-expression of the GLP-1 fusion peptide and a suicide gene. The suicide gene codes for the most widely used Herpes simplex virus Type 1 Thymidine Kinase (HSV1tk). This enzyme converts intracellularly the non-toxic prodrug Ganciclovir into a toxic product, therefore allowing the destruction of transfected cells in the case of unexpected cell proliferation. Thus, systemic application of Ganciclovir to a patient treated with GLP-1 CellBeads^{®} containing degenerated cells leads to destruction of the transplanted cells. The characterization of the cell line 79TM217/18K5 was performed taking into account the recommendations of the ICH Q5B as set forth in:
"Analysis of the Expression Construct in Cells Used for Production of r-DNA Derived Protein Products"; and
"Q5D Derivations and Characterisation of Cell Substrates Used for Production of Biotechnological /Biological Products"; and the
"Note for Guidance on the Quality, Preclinical and Clinical Aspects of Gene Transfer Medicinal Products" (EMEA/273974/2005).

Endostatin as expressed by the Endostatin CellBeads^{®} in these experiments is the cleavage product of collagen XVIII and is known to reduce angiogenesis significantly and therefore could be a potential treatment for AMD.

The GLP-1 CellBeads^{®} and the Endostatin CellBeads^{®} as used for this experiment consist of cells from a human mesenchymal stromal cell line, embedded in a spherical shaped alginate matrix (180 - 200 µm in diameter). The cells are designed to secrete GLP-1 fusion peptide as defined herein, which seem to have an antiapoptotic effect, or the anti-angiogenic factor Endostatin. The alginate matrix, which entraps the cells, is generated during the herein described production process by cross-linking of the alginate with barium ions. The alginate itself has no pharmacological effect but provides a mechanical scaffold for the cells and protects them against attacks of the patient's immune system (see Figure 11 exemplarly for GLP-1). Therefore, the alginate is regarded as an excipient. From the patient's view, the alginate matrix is considered as a safety component, because it restricts the cells locally to the point of application and prevents a free floating of the cells. To fulfil these functions properly, GLP-1 CellBeads^{®} and Endostatin CellBeads^{®} are composed of a corebead, which is an alginate matrix enclosing the GLP-1 fusion peptide secreting cells or the Endostatin secreting cells. This corebead again is surrounded by a shell consisting of pure alginate to assure the complete encapsulation of all cells.

The cells were cryopreserved until use. The cryopreserved CellBeads were then thawed and washed in Ringer solution under aseptic conditions according to standard operation procedure CM:P-142 Anlage 5. The CellBeads were stored in Ringer solution at room temperature. The implantation procedure was completed in about 4 hours and was within the 6 hours time frame set to ensure vitality of the beads.

For the injection 100 GLP-1 CellBeads or 100 Endostatin CellBeads were aspirated under aseptic conditions in a sterile 1 ml syringe. The Ringer solution was deducted to give a bead pellet located in the outlet of the syringe.

### 2.5.1.2. Preparation of the test animal for implantation

The day before implantation of the CellBeads antibiotic eye drops (Refobacin, Merck) were applied. For the aspiration of aqueous humour and CellBead injection the animals were anaesthetized by isofluran inhalation delivered through a face mask continuously. To assure aseptic conditions the area around the eye of the animals were intensively treated with Polyvidon-lodide solution for disinfection. Additionally, the animals were covered by sterile cloths. An eye lock was placed to keep the lids apart. If required eye drops (Novesine 0.4%, OmniVision) were applied to anaesthetize the eye locally (exposure time 30 sec). Disinfection was performed using PVP-Jod-AT 2.5% eye drops (exposure time 2 min).

### 2.5.1.3.Application of test items

For the implantation of the above prepared CellBeads a 20 G permanent venous catheter was used. The needle was vertically injected 2-3 mm apart the iris, after the conjunctiva and sclera has been slightly disarranged. After the catheter was inserted for approx. 1 cm the needle was pulled out leaving the flexible tube in the vitreous cavity. The 1 ml syringe containing 100 CellBeads was attached, vitreous humour aspirated to resuspend the beads and injected intravitreally afterwards. The flexible tube was removed carefully after fixing the conjunctiva. The injection site was checked for closure of the puncture and leaking CellBeads. Syringe and catheter were checked for residual CellBeads that have not been injected. These beads were counted to estimate the actual number of beads in the eye.

Each of the nine animals received GLP-1 CellBeads into the intravitreal cavity of the right eye and Endostatin CellBeads into the intravitreal cavity of the left eye. To care for potential inflammation an antibiotic (Refobacin, 2-3 drops) was dripped in the conjunctiva at termination of the procedure.

The success of CellBeads implantation, the number of beads found in the syringe/catheter and any other observations are given below in section 3 under "Explantation".

The rabbits were subjected to continuous health monitoring throughout the experiment. The health status was monitored at least each second day and is given below. Body weight was determined once weekly starting on the week of acclimatization and with lower frequency at the later stages of the study.

### 2.5.2. Sampling of aqueous humour

100 - 200 µl aqueous humour (100µl minimum requirement, 200µl will allow for repetition of the assay) were collected directly before implantation and at Days 3 , 7, 14, 21, 28 , 35, 42, 49 and 56 in a reaction tube containing 2 µl of DPP-4 inhibitor. Sampling was performed on isofluran anaesthetized animals using a syringe equipped with a 30 G needle from both eyes under aseptic conditions according to 3.5.1. The samples are stored at -20°C immediately after collection.

The aqueous humour was analysed for active GLP-1 and Endostatin concentrations using the GLP-1 active ELISA (Linco Research) and the Endostatin ELISA, respectively. The results are documented in the corresponding laboratory notebooks.

### 2.5.3. Necropsy

Three animals each were sacrificed at Days 3, 14 and 56 post implantation. The animals were euthanatized compliant with standard operation procedure PL:A-207, whereby inhalation of isofluran was used to anaesthetise the animals prior euthanisation by inhalation of CO₂. The documentation of the explantation was conducted as shown below. In brief, the vitreous was opened, the CellBeads explanted and the retinas preserved in formalin.
a) Characterization of the explants
   The explanted CellBeads were washed three times in warm culture medium and characterised by the following parameters:
   i) Microphotographs were taken from all explanted CellBeads to evaluate CellBead integrity. Microphotographs were documented in the corresponding laboratory notebooks.
   ii) Transgene secretion after explantation
      The explanted CellBeads were incubated with culture medium according to standard operation procedure CM:Q-158/02. The culture medium volume was adapted to the actual number of CellBeads (100 µl per GLP-1 CellBead and 10µl per Endostatin CellBead). The results were documented.
   iii) Vitality of the encapsulated cells
      To evaluate vitality of the encapsulated cells, the CellBeads were stained with SYBR Green / Propidium iodide according to standard operation procedure CM:Q-140/01. Fluorescence microphotographs were taken from all CellBeads and documented.
b) Evaluation of the retinas
   Retinas were preserved in 4 % Formalin and stored until send to Prof. Jonas (Universitätsklinikum Mannheim) for histopathological evaluation using HE and PAS staining. Furthermore, parts of the retinas will be used for a TUNEL staining to detect apoptosis. The statistical analysis is carried out by measuring the GLP-1 and Endostatin levels in the aqueous humour of both eyes and evaluating the differences between different sampling time points by statistical function of MS Excel as given in the respective results section. The application of the test items, mortality/viability of the animals, clinical signs, body weights as well as the results of the explantation was recorded.

### 3. Results

There was no mortality observed during the experiment. Clinical signs noted were irritations at the injection sites and conjunctiva in all animals including swelling and reddening. Symptoms disappeared generally within a few days except single animals where recovery from irritations took up to 3 weeks. Most likely the prolonged appearance of symptoms was due to repeated sampling of aqueous humour.

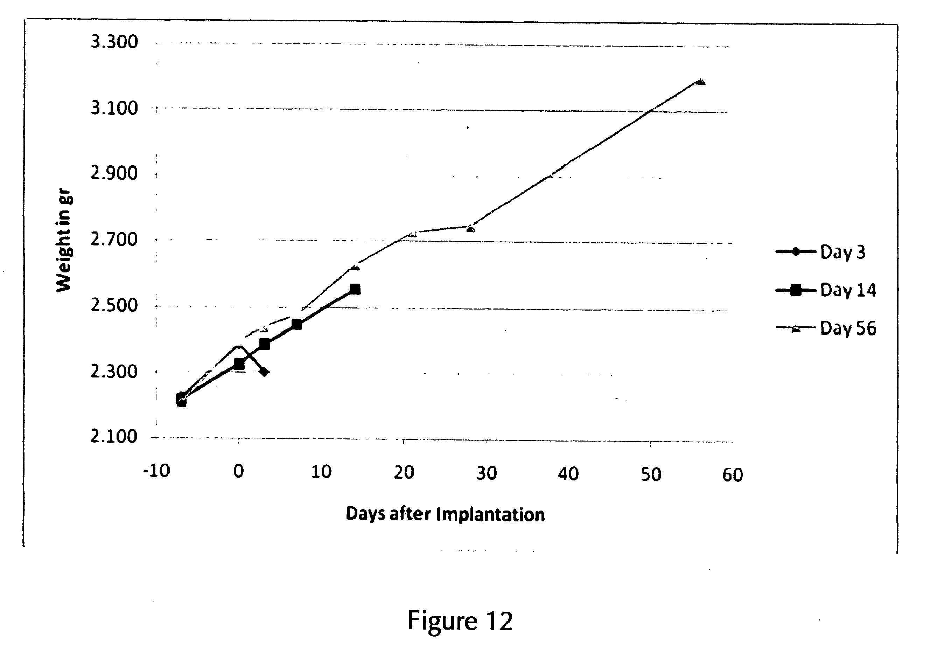

Body weight development was not affected by the implantation procedure except for animal 9 which lost about 10% of weight within 3 days post implantation. The results are shown in Figure 12. (Note: 3 Groups of 3 animals each sacrificed at Days 3, 14 and 56).

### 3.1. implantation

The implantation of CellBeads into the vitreous was successful as the minimum target of 50 or more beads per eye were injected, except for animal 1 where only 37 Endostatin beads were administered (see Table 1 below). Generally, implantation efficiency was comparable between both CellBead types with an average implantation of 78 and 83 beads for GLP-1 and Endostatin, respectively.

### 3.2. Explantation

At post implantation Days 3, 14 and 56 each 3 animals were sacrificed to extract the beads from the eyes. Results are summarized in Table 1 and Table 2. The recovery rate of the GLP-1 CellBeads was comparable at Days 3 and 14 with about 50% been found. At Day 56 4.5% were successfully dissected from the vitreous. The Endostatin CellBeads showed increased recovery rates with progressing implantation duration as 58%, 72% and 80% of the implanted beads were located at Days 3, 14 and 56, respectively. These findings were reflected by the results of the analysis of the aqueous humour sampled in weekly intervals.

**Table 1: Overview of Implanted and Explanted Cell Beads**

| | No. of GLP-1 Beads (CB-087) | | | No. of Endostatin Beads (CB-102) | | |
|---|---|---|---|---|---|---|
| Animal No.* | Implanted | Explanted | % Recovery | Implanted | Explanted | % Recovery |
| 1 | 79 | 2 | 2.5 | 37 | 35 | 94.6 |
| 2 | 72 | 40 | 55.6 | 84 | 72 | 85.7 |
| 3 | 63 | 0 | 0 | 88 | 80 | 90.9 |
| 4 | 80** | 8 | 10.0 | 92 | 50 | 54.3 |
| 5 | 95 | 48 | 50.5 | 89 | 23 | 25.8 |
| 6 | 86 | 3 | 3.5 | 99 | 95 | 96.0 |
| 7 | 93 | 39 | 41.9 | 88 | 56 | 63.6 |
| 8 | 75 | 43 | 57.3 | 89 | 60 | 67.4 |
| 9 | 59 | 57 | 96.6 | 84 | 46 | 54.8 |
| Average | 78 | 27 | 35.3 | 83 | 57 | 70.4 |

| | | | | | | |
|---|---|---|---|---|---|---|
| * Animals 1, 5 and 9 allocated to Day 3 sacrifice, animals 2, 7, and 8 for Day 14 and animals 3, 4 and 6 for Day 56. ** Approximate value due to technical problems. | | | | | | |

**Table 2: Average Implantation and Explantation Results at Days 3, 14 and 56**

| | No. of GLP-1 Beads (CB-087) | | | No. of Endostatin Beads (CB-102) | | |
|---|---|---|---|---|---|---|
| Time point | Implanted | Explanted | % Recovery | Implanted | Explanted | % Recovery |
| Day 3 | 77.6 | 35.6 | 49.8 | 70.0 | 34.6 | 58.4 |
| Day 14 | 80.0 | 40.6 | 51.6 | 87.0 | 62.6 | 72.2 |
| Day 56 | 74.5 | 3.6 | 4.5 | 93.0 | 75.0 | 80.4 |

### 3.3. Viability of the CellBeads

The viability of the CellBeads was analysed by staining with propidium iodide and SYBR Green at explantation Days 3, 14 and 56. Furthermore, the amount of GLP-1 and Endostatin in the aqueous/vitreous humour and secreted into culture medium after explantation was checked at each occasion according to the standard procedures (see section 3.4 below). The results of the determinations of GLP-1 and Endostatin found in the aqueous/vitreous humour and culture media are given below.

### 3.3.1. Explantation after 3 days

Date of explantation: 18.07.08

| Animal No. | No. of Beads: Right eye (CB-087 GLP-1 CellBeads) | No. of Beads: Left eye (CB-102 Endostatin CellBeads) |
|---|---|---|
| 1 | 2 | 35 |
| 5 | 48 | 23 |
| 9 | 57 | 46 |

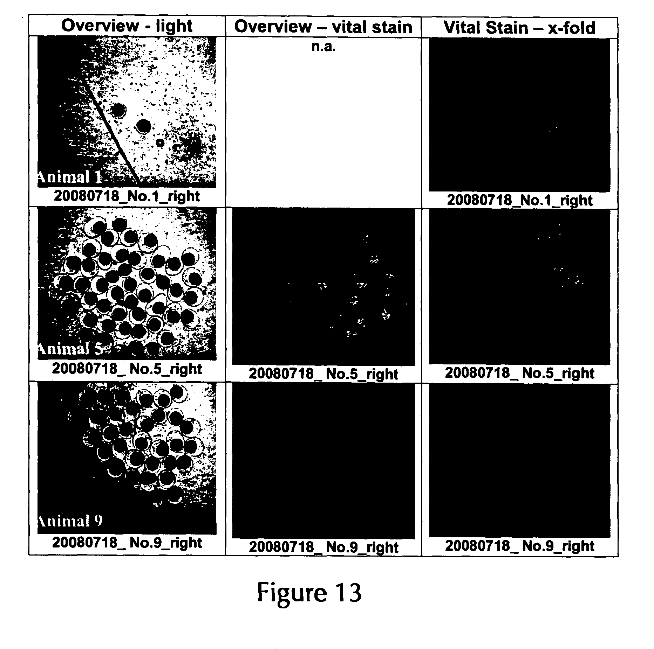

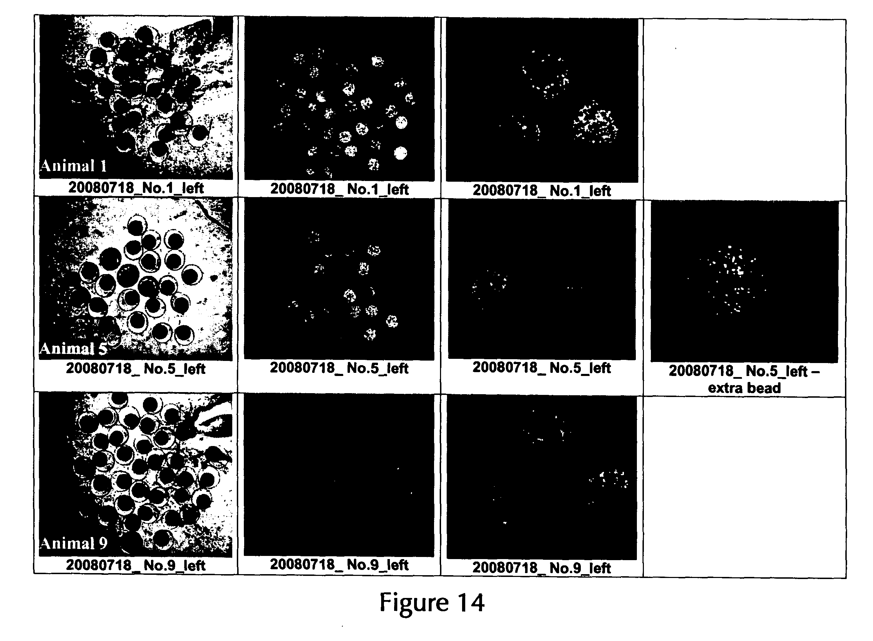

Both types of CellBeads were homogeneously vital in all three animals tested. The results are summarised for GLP-1 in Figure 13 and for Endostatin in Figure 14.

### 3.3.2. Explantation after 14 days

Date of explantation: 29.07.08

| Animal No. | No. of Beads: Right eye (CB-087 GLP-1 CellBeads) | No. of Beads: Left eye (CB-102 Endostatin CellBeads) |
|---|---|---|
| 2 | 40 | 72 |
| 7 | 39 | 56 |
| 8 | 43 | 60 |

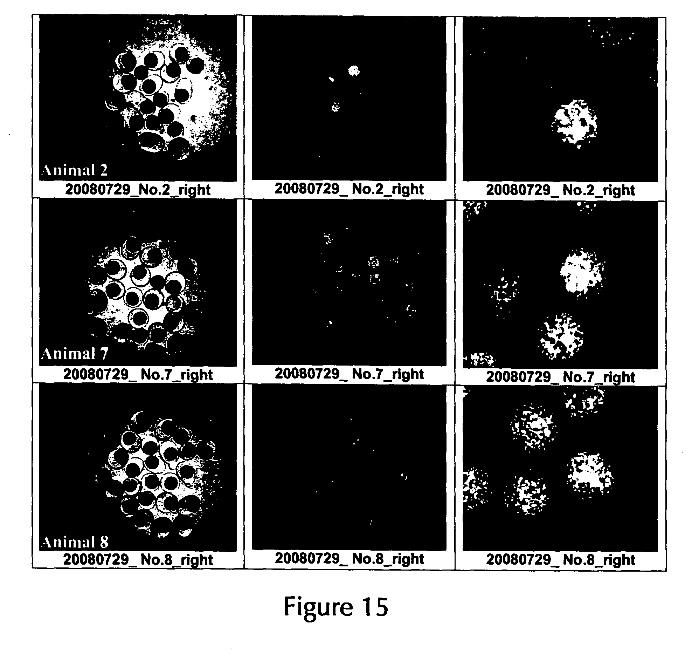

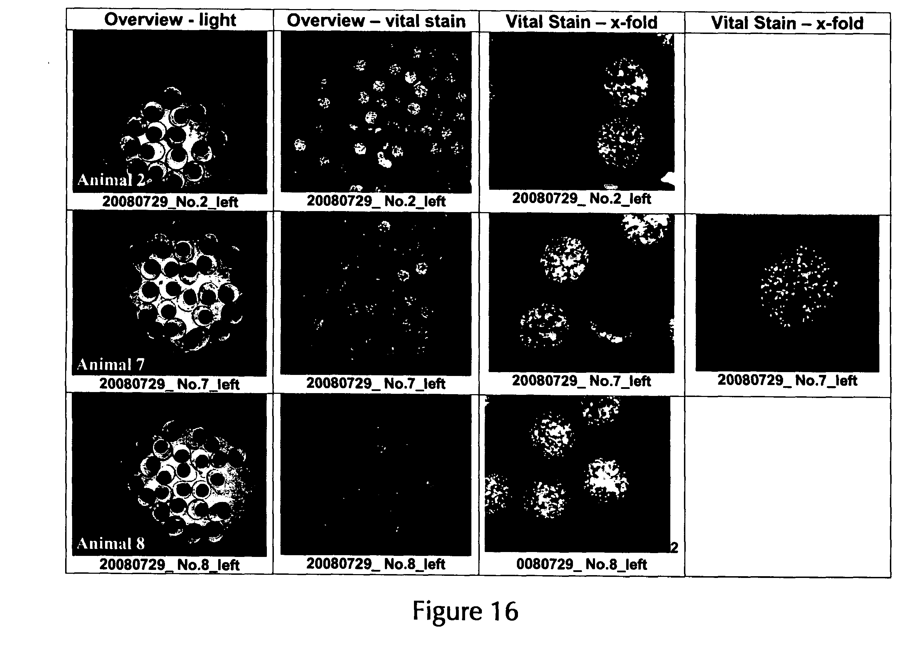

Both types of CellBeads were homogeneously vital in animal 7 and 8. The CellBeads of animal 2 were more heterogeneous in vitality as up to 30% of the Endostatin CellBeads contained predominantly dead cells and up to 90% of GLP-1 CellBeads contained predominantly dead cells. The recovery rate of the GLP-1 CellBeads was lower compared to the Endostatin CellBeads. The results are summarised for GLP-1 in Figure 15 and for Endostatin in Figure 16.

### 3.3.3. Explantation after 56 days

Date of explantation: 09.09.08

| Animal No. | No. of Beads: Right eye (CB-087 GLP-1 CellBeads) | No. of Beads: Left eye (CB-102 Endostatin CellBeads) |
|---|---|---|
| 3 | 0 | 80 |
| 4 | 8 | 50 |
| 6 | 3 | 95 |

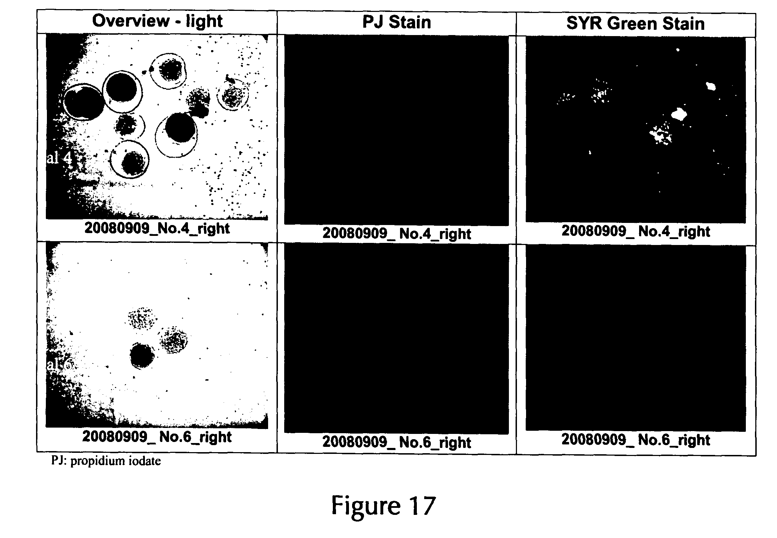

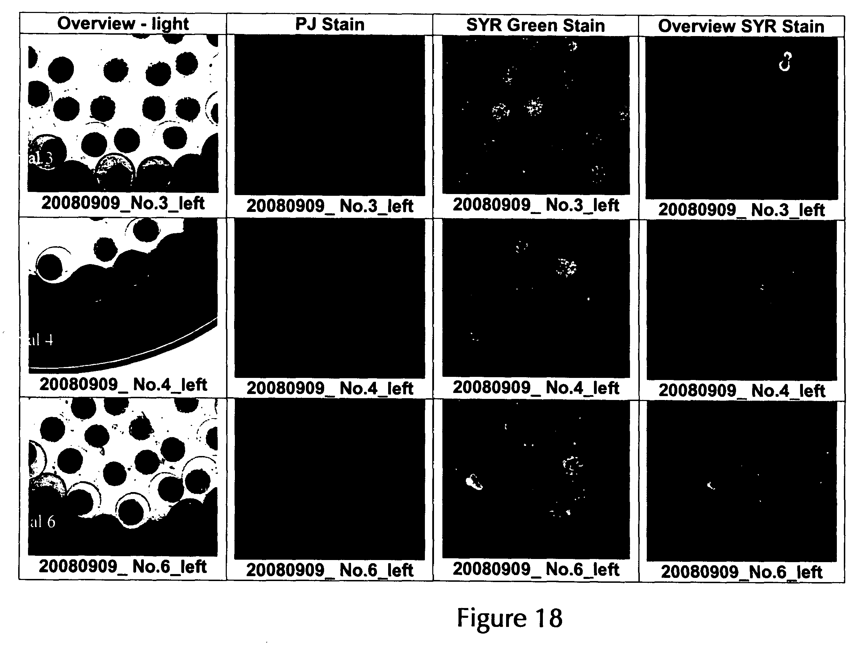

The recovery rate of GLP-1 CellBeads was smaller than on days 3 and 14. However, at day 56 GLP-1 CellBeads still could be found. The Endostatin beads were recovered at high frequency. The vitality was heterogeneous between the three animals with animal 3 showing an overall moderate to good vitality. The beads from animal 4 and 6 stained only weakly and/or dispersed for SBYR green indicating lower than normal vitality with single beads of more intense colouring. The results are summarised for GLP-1 in Figure 17 and for Endostatin in Figure 18.

### 3.3.4. Conclusions:

There was a time and CellBead type dependent course of viability observed in the Experiment, whereas the Endostatin beads retained higher vitality at the end of the study. The GLP-1 CellBeads appeared to be comparable to the Endostatin beads up to Day 14 and with declining viability/vitality thereafter as seen on Day 56. The recovery of Endostatin CellBeads was generally higher compared to the GLP-1 CellBeads irrespective that comparable amounts were successfully implanted. The fate of the lost GLP-1 CellBeads from Day 14 to Day 56 remains unexplained and may be resolved in a further study.

### 3.4. Expression levels in aqueous and vitreous humour

### 3.4.1. GLP-1 Level

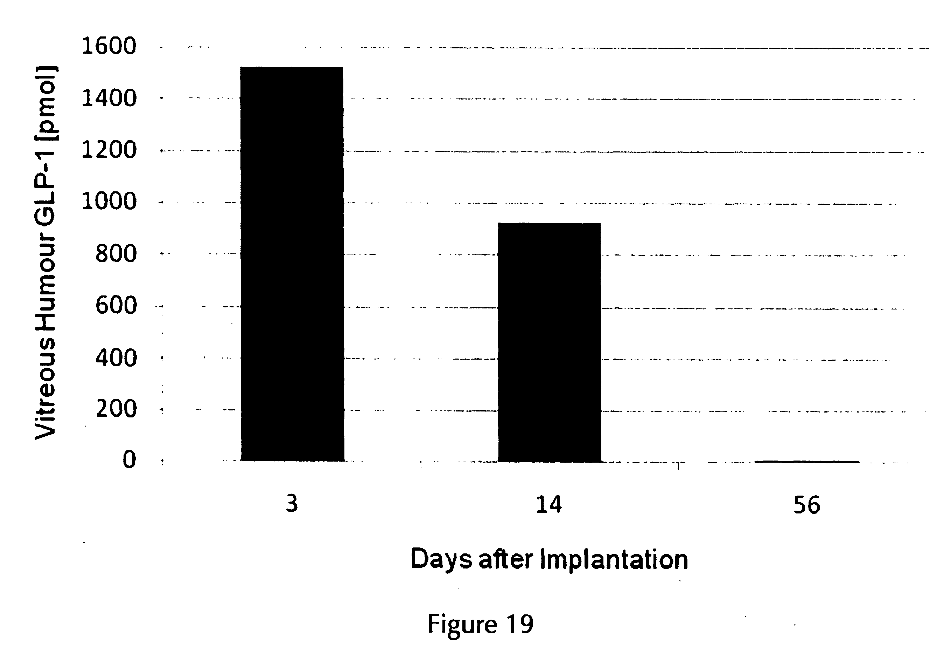

Vitreous humour was collected at each sacrifice along with the recovery of the CellBeads. The GLP-1 content was significantly increased compared to the respective other eye implanted with Endostatin CellBeads (below the detection level at each time point). Highest levels were obtained shortly after implantation which decreased by 40% from Day 3 to Day 14. At Day 56 the GLP-1 concentration found was below the detection level of the ELISA system used. The latter finding corresponds to the fact that at this time point no or only dead CellBeads were found. The absolute values may were even slightly higher but were limited by the effective range of the analytical system. The results are summarised in Figure 19, showing the GLP-1 concentrations in Vitreous Humour at Days 3, 14 and 56 (The samples were collected at each sacrifice, n=3 per time point.).

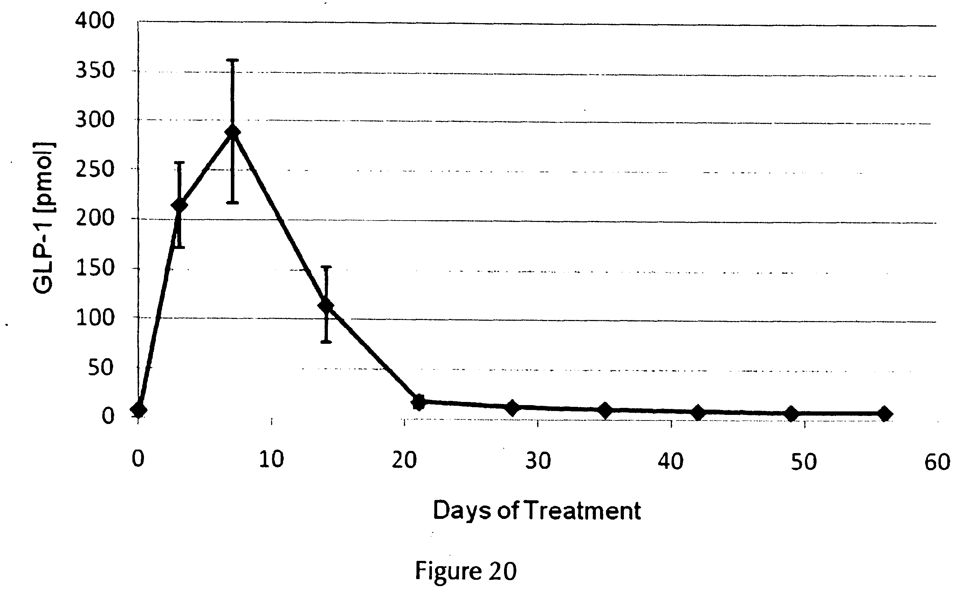

Aqueous humour was sampled once on Day 3 and in weekly intervals from all animals starting on Day 7. There was a strong increase in GLP-1 observed following the implantation of the CellBeads. Concentration peaked on Day 7 with 288 pmol and decreased thereafter. On Day 14 the GLP-1 level was about half the maximal concentration (114 pmol) while on Day 21 the concentration was decreased to slightly above the baseline level/effective range of the assay system. (see Figure 20). The samples were n=9 on Days 0, 3 and 7, n=6 on Day 14, and n=3 on Day 21 to 56. All control samples had GLP-1 levels below the detection limit of the assay system. The values are normalised for the initial number of CellBeads successfully implanted.

### 3.4.2. Endostatin Level

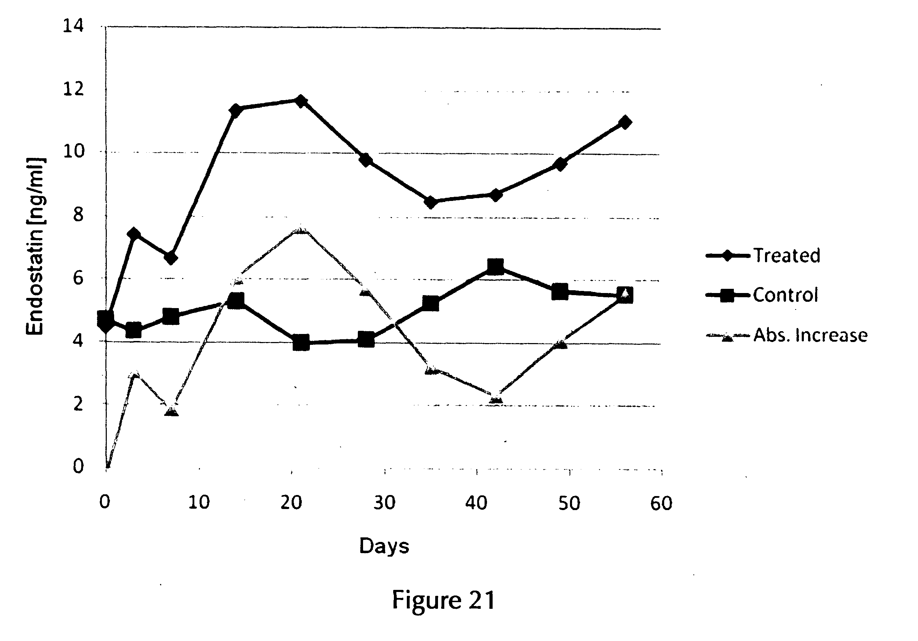

Implantation of Endostatin-CellBeads into rabbit eyes resulted in increased endostatin concentration in the aqueous (anterior eye chamber) and vitreous (posterior eye chamber) humour beginning with the first sampling time point at Day 3. Endostatin is naturally present in the rabbit eye as indicated by an average level of 4 to 6 ng/ml Endostatin found in the eye implanted with the GLP-1 CellBeads which served as control. The time courses of the endostatin concentrations in aqueous humour of treated and control eyes are given in Figure 21. There was a gradual increase in the endostatin concentration observed reaching the peak concentration on treatment days 14 to 21 whereas a transient decrease was noted thereafter with a final concentration on Day 56 in the range of the peak level. The data are presented in Figure 21 and Table 3.

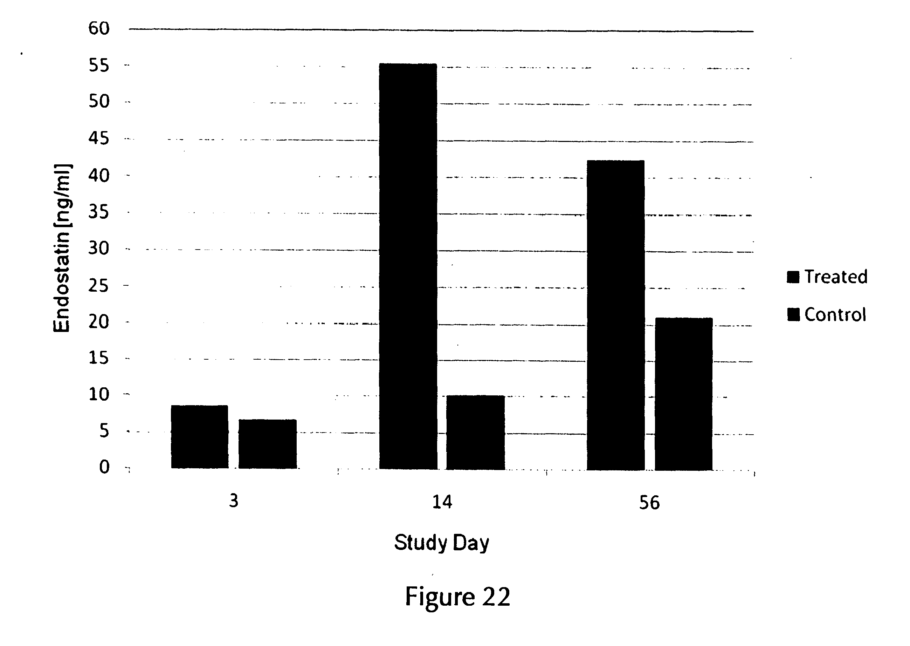

Based on the fact that the CellBeads were implanted into the posterior chamber of the eye the endostatin levels measured in vitreous humour were clearly higher compared to the samples taken from the anterior chamber. Concentrations measured were marginally higher compared to the control samples on Day 3. Highest relative and absolute endostatin levels were found on Day 14. After 56 days the absolute endostatin concentration was slightly below that of Day 14 but the relative levels (with the control value subtracted, [ ] in Table 3) was considerably lower as the concentration in the control samples was higher compared to Day 14. The results are summarised in Figure 22 and Table 4.

**Table 3: Summary of Data Endostatin in Aqueous Humour**

| **Treatment Days** | **0** | **3** | **7** | **14** | **21** | **28** | **35** | **42** | **49** | **56** |
|---|---|---|---|---|---|---|---|---|---|---|
| Mean Treated | 4,50 | 7,43 | 6,67 | 11,36 | 11,67 | 9,81 | 8,48 | 8,72 | 9,71 | 11,05 |
| SE Treated | 0,29 | 1,34 | 0,77 | 1,80 | 3,62 | 1,70 | 0,90 | 1,58 | 1,50 | 2,25 |
| Mean Control | 4,71 | 4,36 | 4,80 | 5,31 | 3,98 | 4,09 | 5,25 | 6,42 | 5,64 | 5,53 |
| SE Control | 0,33 | 0,47 | 0,39 | 0,82 | 0,55 | 0,45 | 0,62 | 1,18 | 0,82 | 0,70 |
| Treated/Control | -0,21 | 3,07 | 1,87 | 6,05 | 7,69 | 5,72 | 3,23 | 2,30 | 4,07 | 5,52 |
| p (2-sided T-test) | 0,664 | 0,047 | 0,061 | 0,013 | 0,105 | 0,033 | 0,051 | 0,342 | 0,087 | 0,084 |
| n (per group) | 9 | 9 | 6 | 6 | 3 | 3 | 3 | 3 | 3 | 3 |

| | | | | | | | | | | |
|---|---|---|---|---|---|---|---|---|---|---|
| Concentrations are given in [pmol]. SE: Standard error. | | | | | | | | | | |

**Table 4: Summary of Data Endostatin in Vitreous Humour**

| | | | |
|---|---|---|---|
| **Treatment Days** | **3** | **14** | **56** |
| Mean Treated | 11.38 | 55.46 | 42.34 |
| SE Treated | 2.13 | 4.93 | 6.35 |
| Mean Control | 8.82 | 10.03 | 20.90 |
| SE Control | 2.70 | 2.60 | 7.72 |
| Treated/Control | 2.55 | 45.43 | 21.44 |
| p (2-sided T-test) | 0.577 | 0.0026 | 0.154 |
| n (per group) | 3 | 3 | 3 |

| | | | |
|---|---|---|---|
| Concentrations are given in ng/ml. SE: Standard error. | | | |

### 3.5. TUNEL stain of the retinas

The retinas of the treated animals were analysed for apoptotic and anti-apoptotic effect, respectively, with the TUNEL stain. Using the TUNEL stain an apoptotic effect would become visible as stained nuclei while a reduction of these nuclei relative to untreated control animals may indicate an anti-apoptotic effect of the treatment with GLP-1 and/or Endostatin CellBeads.

The staining procedure of the TUNEL stain was conducted according to MET080-01 und micrographs were taken at identical conditions with no further corrections regarding e.g. brightness or contrast.

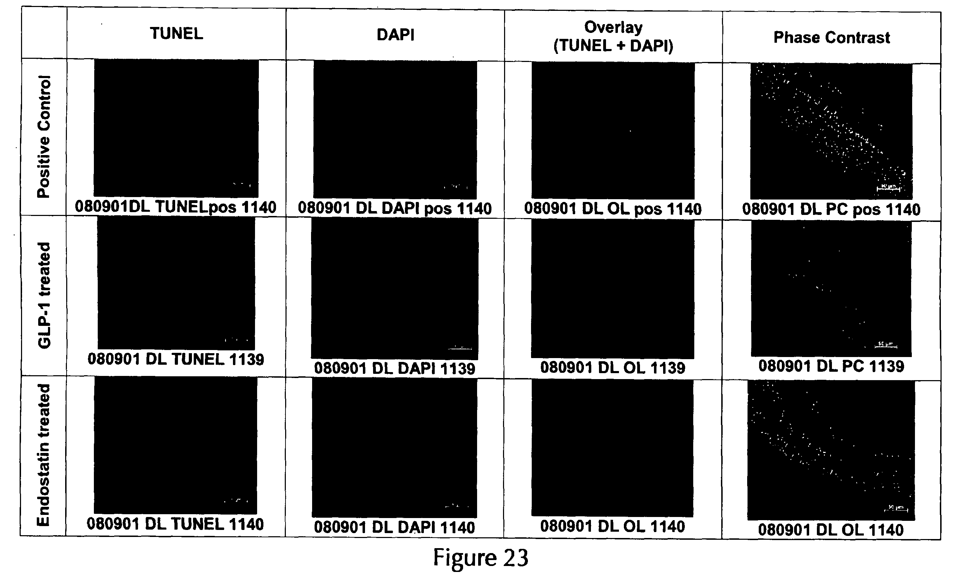

In Figure 23 the results of a TUNEL stain are given. The positive control (section pretreated with DNA digestive enzyme I) showed distinct positive nuclei at inner retina border. This finding was not observed in GLP-1 CellBead treated animals. In Endostatin CellBeads treated animals only weakly stained structures were noted but these may be considered to be artifacts as Endostatin is not known to be an inducer of apoptosis. The outer retina border showed an intense presumably unspecific staining as no distinct structures were labelled. Therefore the examination of further sections of these retinas was conducted to ensure validity of the above findings.

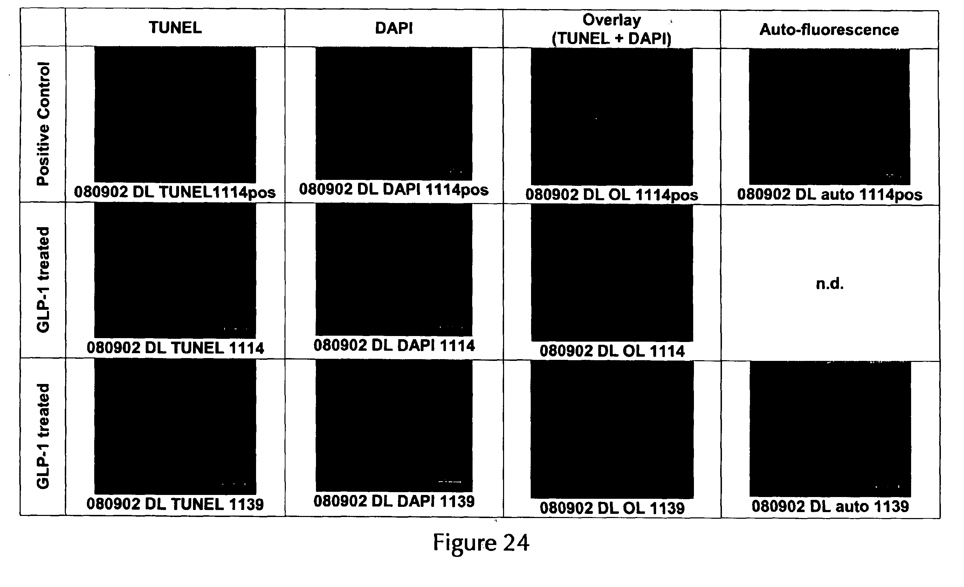

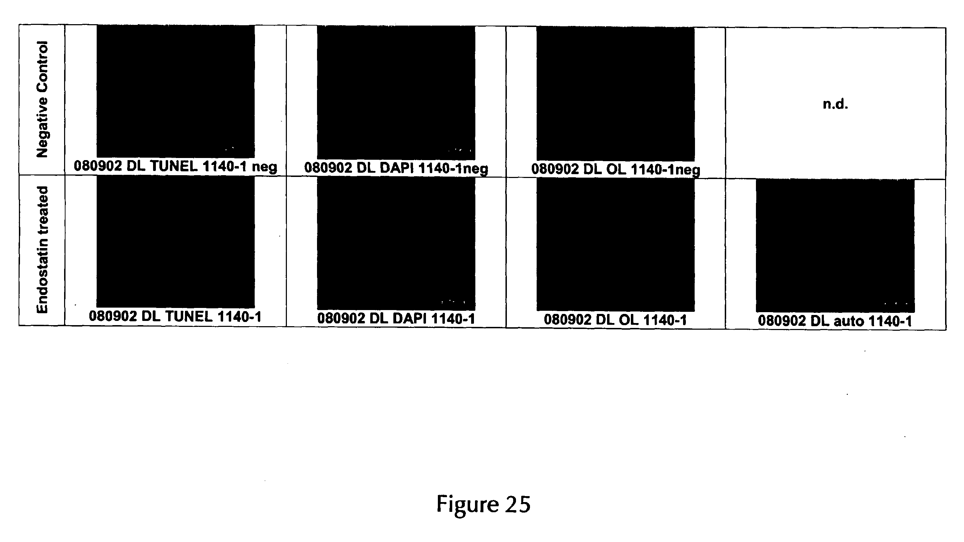

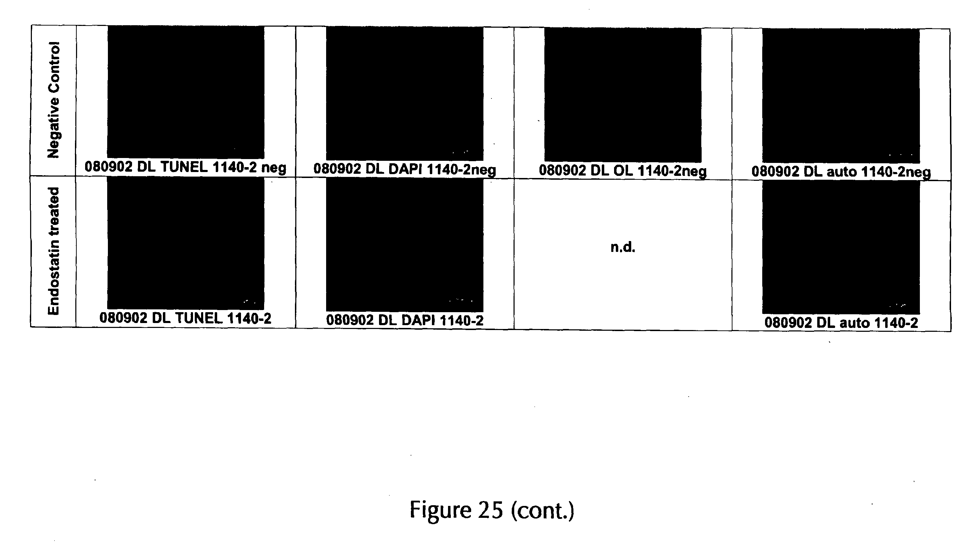

The results of the second TUNEL stain are shown in Figure 24 and Figure 25. The positive control gave distinct positive structures for the TUNEL stain in the lower segment of the retina. The GLP-1 treated animal showed no such findings indicating no apoptotic processes being induced by GLP-1. The retina derived from the Endostatin treated animal showed again some weakly/faintly stained nuclei in the lower region of the retina but the intensity was clearly lower compared to the unspecific signal seen in the outer layer of the retina. In the negative control, section treated with the same procedure but without enzyme, the lower layer of the retina showed also some positive signal but distinct structures were not identified. It was concluded that these differences may result from different/variable thickness of the sections analysed and therefore the nuclei could not have been identified in the thicker slices of the negative control. Furthermore, these findings were also seen when the auto-fluorescent mode (red light channel) of the microscope was used and thereby substantiating the earlier assumption (reference: KM09_08 DL).

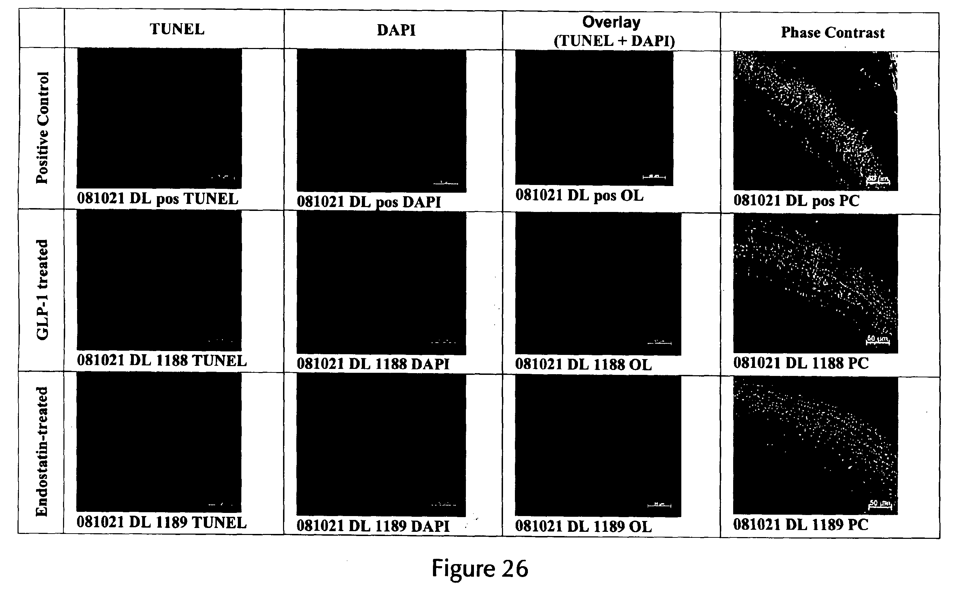

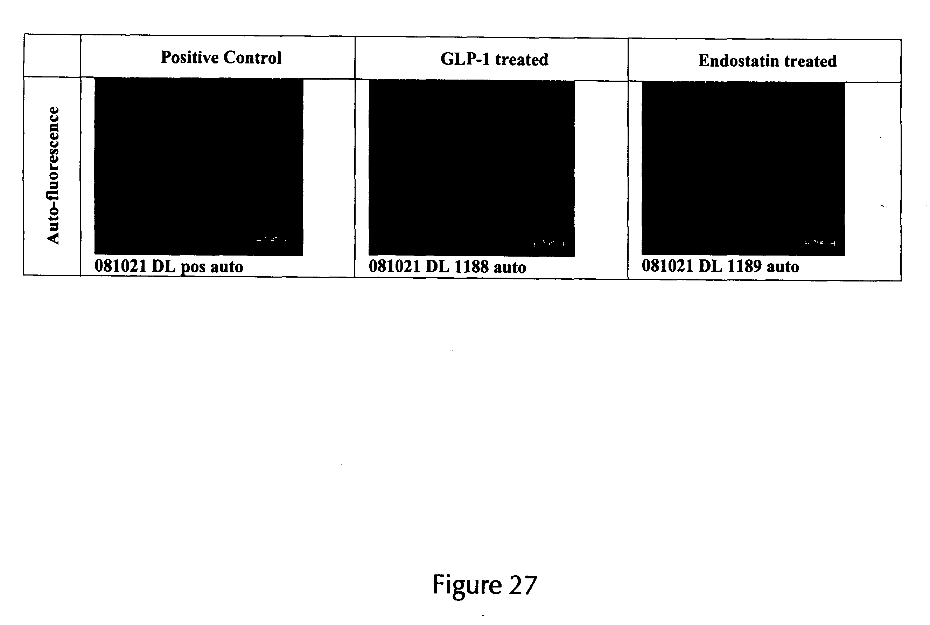

A replicate analysis of the retinas treated for two month with CellBeads with new sections being prepared by the same technician allowed for a more precise investigation by TUNEL stains. Representative results are given in Figure 26 and Figure 27.

The results showed that the outer receptor layer had, again, a fairly strong unspecific staining which was also seen in the auto-fluorescence mode of the microscope. There were no specific TUNEL positive cells seen in the retinas of either the GLP-1 or Endostatin treated animals. The validity of the staining procedure was confirmed by the positive control where apoptotic cells/nuclei were noted at high frequency.

In conclusion, both GLP-1 and Endostatin treated rabbit retinas were TUNEL negative after 2 month treatment. Thus, no negative effect of both proteins on the vitality of retinal cells was observed.

### 4. Discussion and Conclusions

The above experiments established positive results in the intravetreal implantation of CellBeads into the rabbit eye by an indwelling catheter. The success rate was variable due to the use of standard equipment with Luer connectors that exhibited a non-linear injection channel resulting in loss of beads at the nozzle. The CellBeads were well tolerated as only some minor irritations such as reddening and swelling were observed after administration. The viability of both GLP-1 and Endostatin CellBeads was good for up to 14 days. At the end of the study (Day 56) the GLP-1 CellBeads were recovered to a minor extent whereas the Endostatin CellBeads showed a satisfactory recovery and viability rate.

The implantation of the CellBeads significantly increased the GLP-1 and Endostatin levels found in the aqueous and vitreous humour significantly whereas the effect was much more pronounced in the vitreous humour (implantation site). Peak concentrations for GLP-1 were approx. 15-fold and 100-fold above baseline in aqueous and vitreous humour, respectively, at Day 7 and 3. For Endostatin peak concentrations were noted on Days 21 and 14 for aqueous and vitreous humour, respectively, where approx. 3-fold and 5.5-fold increases above baseline were measured.

TUNEL stains of the rabbit retinas following to the 56 days exposure to GLP-1 and endostatin showed no increase in apoptotic cells. Thus, it can be excluded that the CellBeads treatment had adverse effects on the eyes.

The present experiment was conducted to evaluate the efficacy and safety of GLP-1 and endostatin CellBeads as defined herein for the delivery of therapeutic proteins/peptides secreted from genetically modified mesenchymal stem cells.

Technical experience in implanting CellBeads into the vitreous cavity of the eye was established in laboratory rabbits. A major challenge was to monitor the number of beads actually implanted into the eye with standard injection equipment with Luer connectors complicated the transfer of the beads as they tend to accumulate at the nozzle of the syringe. This finding may (or most likely) rely on the fact that the amount of fluid made available for the process is limited to avoid to put pressure on the eye ball by injecting too much fluid along with the CellBeads. Overall, the method used in the experiment showed satisfactory results.

A key feature of the experimens was to analyse the viability of GLP-1 and Endostatin CellBeads in the vitreous fluid over time. The GLP-1 CellBeads showed satisfactory viability at least up to Day 14. The recovery and viability of Endostatin CellBeads was good at any time point until the end of the experiment at Day 56 and the Endostatin CellBeads survived in a reasonable number for at least 56 days.

The viability of the CellBeads is reflected by the concentration levels obtained for the therapeutic peptides in vitreous and aqueous humour. The GLP-1 peptide concentration peaked on Day 7 after implantation and declined thereafter. On Day 14 about half the maximal concentration was noted while on Day 21 the level was slightly above the baseline concentration. From Day 28 onward no significant GLP-1 concentrations were detected in the aqueous and vitreous humour (Day 56). The concentrations were consistently higher in the vitreous humour at the respective time points but were expected as the beads were implanted in the posterior cavity. The substantially improved viability of the Endostatin CellBeads resulted in significantly increased endostatin levels in the aqueous and vitreous humour up to Day 56. There were some fluctuations observed with advancing duration of the study but may refer to the low number of samples/animals at Days 15 to 56. Overall, at the end of the study the endostatin levels in the aqueous humour were as high as the peak concentration on Days 14 and 21 even though the viability of the Endostatin CellBeads was slightly compromised compared to the earlier time points.

The safety of the therapeutic peptides regarding the vitality of the retina was checked by TUNEL stains to detect increased cell apoptosis. Due to initial technical difficulties the assay was repeated but in any case retina sections from the GLP-1 and Endostatin CellBeads treated eyes were negative for apoptotic cells.

In conclusion, both types of CellBeads investigated in the present experiment were well tolerated by the rabbit eyes as the irritations resulting from the implantation procedures disappeared generally in due course and no opacity of the lenses was observed at the long term. Furthermore, the vitality of the retinal cells was not affected by GLP-1 and Endostatin as no induction of apoptosis was observed in histological sections.

## Claims

1. Use of cells, engineered to encode and secrete an anti-angiogenic factor, a neuroprotective factor and/or a fragment or variant thereof, for the preparation of a medicament for the (intraocular) treatment of eye diseases or disorders, wherein the cells, engineered to encode and secrete the anti-angiogenic factor, a neuroprotective factor and/or a fragment or variant thereof, are encapsulated in a (spherical) microcapsule.

2. Use according to claim 1, wherein the anti-angiogenic factor or the neuroprotective factor are selected from a GLP-1 peptide, including a GLP-1 fusion peptide, Endostatin.

3. Use according to any of claims 1 or 2, wherein the spherical microcapsule preferably comprises a spherical core and at least one surface coating layer,
wherein the spherical core comprises or consists of a mixture of cross-linked polymers and cells, encoding and secreting the anti-angiogenic factor, the neuroprotective factor and/or a fragment or variant thereof; and
wherein the at least one surface coating layer comprises or consists of cross-linked polymers.

4. Use according to any of claims 1 to 3, wherein eye diseases and disorders include eye diseases and disorders caused by a degeneration of the retina, including retinal diseases, selected from retinitis pigmentosa (RP), macular degeneration (MD), age-related macular degeneration (AMD or ARMD), retinopathy, diabetic retinopathy, macula edema, abnormalities of the vascular endothelium and pericytes, retinal vessel occlusions, glaucomas, including the trabecular meshwork, and optic neuropathies, including alterations of the trabecular meshwork, and abnormalities of the corneal endothelium, or any disease or disorder associated thereto.

5. Use according to any of claims 1 to 4, wherein the spherical microcapsule has a total diameter of about 120 µm to about 800 µm, a total diameter of about 120 µm to about 700 µm, a total diameter of about 150 µm to about 650 µm, or a total diameter of about 165 µm to about 600 µm, or even a total diameter of about 120 µm to about 300 µm, a total diameter of about 150 µm to about 250 µm, a total diameter of about 165 µm to about 225 µm, or a total diameter of about 180 µm to about 200 µm, including a total diameter of about 180, 185, 190 or 200 µm.

6. Use according to any of claims 1 to 5, wherein the cells are mesenchymal stem cells, mesenchymal stromal cells, human mesenchymal stem cells, differentiated cells derived from human mesenchymal stem cells, allogenic cells or autologous cells encoding and secreting the anti-angiogenic, the neuroprotective factor and/or a fragment or variant thereof.

7. Use according to any of claims 3 to 6, wherein the cross-linked polymer of the core and/or the at least one surface coating layer comprise a chemically identical polymer in identical or differing concentrations, wherein the polymers further may have different molecular weights and/or may be cross-linked differently.

8. Use according to any of claims 3 to 7, wherein the cross-linked polymer is selected from the group comprising biopolymers and alginates.

9. Use according to any of claims 3 to 9, wherein the spherical microcapsule comprises 1, 2, 3, 4, 5, 5-10 or more surface coating layers.

10. Use according to any of claims 3 to 9, wherein the spherical microcapsule comprises an additional external surface coating layer consisting of polycations.

11. Use according to any of claims 1 to 10, wherein the anti-angiogenic, the neuroprotective factor and/or a fragment or variant thereof is a GLP-1 peptide selected from the group consisting of:
a) a peptide comprising aa 7 - 35 of GLP-1; or
b) a peptide comprising aa 7 - 36 of GLP-1 or GLP-1 (7-36)amide; or
c) a peptide comprising aa 7 - 37 of GLP-1; or
d) a peptide comprising the sequence according to formula II:
Xaa7-Xaa8-Glu-Gly-Thr-Phe-Thr-Ser-Asp-Xaa16-Ser-Xaa18-Xaa19-Xaa20-Glu-Xaa22-Xaa23-Ala-Xaa25-Xaa26-Xaa27-Phe-Ile-Xaa30-Trp-Leu-Xaa33-Xaa34-Xaa35-Xaa36-Xaa37,
wherein Xaa7 is L-histidine; Xaa8 is Ala, Gly, Val, Leu, Ile or Lys; Xaa16 is Val or Leu; Xaa18 is Ser, Lys or Arg; Xaa19 is Tyr or Gln; Xaa20 is Leu or Met; Xaa22 is Gly or Glu; Xaa23 is Gln, Glu, Lys or Arg; Xaa25 is Ala or Val; Xaa26 is Lys, Glu or Arg; Xaa27 is Glu or Leu; Xaa30 is Ala, Glu or Arg; Xaa33 is Val or Lys; Xaa34 is Lys, Glu, Asn or Arg; Xaa35 is Gly; Xaa36 is Arg, Gly or Lys or amide or absent; Xaa37 is Gly, Ala, Glu, Pro, Lys, amide or is absent; or
e) a peptide comprising the sequence according to formula III:
Xaa7-XaaB-Glu-Gly-Thr-Phe-Thr-Ser-Asp-Val-Ser-Xaa18-Tyr-Leu-Glu-Xaa22-Xaa23-Ala-Ala-Xaa26-Glu-Phe-Ile-Xaa30-Trp-Leu-Val-Xaa34-Xaa35-Xaa36-Xaa37,
wherein Xaa7 is L-histidine; Xaa8 is Ala, Gly, Val, Leu, Ile or Lys; Xaal8 is Ser, Lys or Arg; Xaa22 is Gly or Glu; Xaa23 is Gln, Glu, Lys or Arg; Xaa26 is Lys, Glu or Arg; Xaa30 is Ala, Glu or Arg; Xaa34 is Lys, Glu or Arg; Xaa35 is Gly; Xaa36 is Arg or Lys, amide or is absent; Xaa37 is Gly, Ala, Glu or Lys, amide or is absent, or
f) a peptide showing an identity of at least 80 % with any of the above peptides according to a) to e).

12. Use according to any of claims 1 to 11, wherein the anti-angiogenic, the neuroprotective factor and/or a fragment or variant thereof is a GLP-1 fusion peptide or a fragment or variant thereof comprising components (I) and (II),
wherein component (I) N-terminally is selected from the group of peptides consisting of or comprising the sequence of
a) a GLP-1 (7-35, 7-36 or 7-37) sequence, or
b) a sequence according to SEQ ID NO: 1; or
c) a peptide comprising or consisting of the sequence according to formula II:
Xaa7-XaaB-Glu-Gly-Thr-Phe-Thr-Ser-Asp-Xaa16-Ser-Xaa18-Xaa19-Xaa20-Glu-Xaa22-Xaa23-Ala-Xaa25-Xaa26-Xaa27-Phe-Ile-Xaa30-Trp-Leu-Xaa33-Xaa34-Xaa35-Xaa36-Xaa37,
wherein Xaa7 is L-histidine; Xaa8 is Ala, Gly, Val, Leu, Ile or Lys; Xaa16 is Val or Leu; Xaa18 is Ser, Lys or Arg; Xaa19 is Tyr or Gln; Xaa20 is Leu or Met; Xaa22 is Gly or Glu; Xaa23 is Gln, Glu, Lys or Arg; Xaa25 is Ala or Val; Xaa26 is Lys, Glu or Arg; Xaa27 is Glu or Leu; Xaa30 is Ala, Glu or Arg; Xaa33 is Val or Lys; Xaa34 is Lys, Glu, Asn or Arg; Xaa35 is Gly; Xaa36 is Arg, Gly or Lys or amide or absent; Xaa37 is Gly, Ala, Glu, Pro, Lys, amide or is absent; or
d) a peptide comprising or consisting of the sequence according to formula III:
Xaa7-Xaa8-Glu-Gly-Thr-Phe-Thr-Ser-Asp-Val-Ser-Xaal 8-Tyr-Leu-Glu-Xaa22-Xaa23-Ala-Ala-Xaa26-Glu-Phe-Ile-Xaa30-Trp-Leu-Val-Xaa34-Xaa35-Xaa36-Xaa37,
wherein Xaa7 is L-histidine; Xaa8 is Ala, Gly, Val, Leu, Ile or Lys; Xaa18 is Ser, Lys or Arg; Xaa22 is Gly or Glu; Xaa23 is Gln, Glu, Lys or Arg; Xaa26 is Lys, Glu or Arg; Xaa30 is Ala, Glu or Arg; Xaa34 is Lys, Glu or Arg; Xaa35 is Gly; Xaa36 is Arg or Lys, amide or is absent; Xaa37 is Gly, Ala, Glu or Lys, amide or is absent; or
e) or a sequence having at least 80 % sequence identity with a sequence of any of sequence according to a) to d); and
component (II) C-terminally of component (II) is selected from a peptide sequence of at least 9 amino acids or a functional fragment or variant thereof.

13. Use according to claim 12, wherein component (II) of the GLP-1 fusion peptide, is selected from:
a) a peptide sequence containing a sequence according to SEQ ID NO: 22 (RRDFPEEVAI), SEQ ID NO: 27 (DFPEEVAI), SEQ ID NO: 28 (RDFPEEVA), or SEQ ID NO: 29 (RRDFPEEV), SEQ ID NO: 30 (AADFPEEVAI), SEQ ID NO: 31 (ADFPEEVA), or SEQ ID NO: 32 (AADFPEEV), or a sequence having at least 80% sequence identity with SEQ ID NO: 22, 27, 28, 29, 30, 31 or 32; or
b) a peptide sequence containing a sequence according to SEQ ID NO: 23 (RRDFPEEVAIVEEL) or SEQ ID NO: 24 (RRDFPEEVAIAEEL), or SEQ ID NO: 33 (AADFPEEVAIVEEL) or SEQ ID NO: 34 (AADFPEEVAIAEEL), or a sequence having at least 80% sequence identity with SEQ ID NOs: 23, 24, 33 or 34; or
c) a peptide sequence containing a sequence according to SEQ ID NO: 2 (RRDFPEEVAIVEELG), SEQ ID NO: 3 (RRDFPEEVAIAEELG), SEQ ID NO: 35 (AADFPEEVAIVEELG), or SEQ ID NO: 36 (AADFPEEVAIAEELG), or a sequence having at least 80% sequence identity with SEQ ID NOs: 2, 3, 35 or 36.

14. Use according to any of claims 12 or 13, wherein component (I) and component (II) of the GLP-1 fusion peptide are directly linked or linked *via* a linker sequence.

15. Use according to any of claims 12 to 14, wherein the GLP-1 fusion peptide contains alternatively or additionally to components (I) and (II) a component (III), wherein component (III) may be linked to the C-terminus of component (I) and/or to the N-terminus of component (I), if components (I) and (III) are present in the fusion protein, or wherein component (III) may be linked to the C-terminus of component (II) and/or to the N-terminus of component (I), if components (I), (II) and (III) are present in the fusion protein.

16. Use according to any claim 15,
wherein component (III) comprises at least four amino acid residues, preferably at least 10 additional amino acid residues, more preferably at least 20, or most preferably at least 30, or
wherein component (III) comprises at least 4, preferably at least 10, more preferably at least 20 additional amino acid residues of
a) the N-terminal sequence of GLP-2 as in proglucagon, or
b) a GLP-1 (5-37, 6-37, or 7-37) sequence, or
c) a peptide comprising the sequence according to formula II:
Xaa7-Xaa8-Glu-Gly-Thr-Phe-Thr-Ser-Asp-Xaa16-Ser-Xaa18-Xaa19-Xaa20-Glu-Xaa22-Xaa23-Ala-Xaa25-Xaa26-Xaa27-Phe-Ile-Xaa30-Trp-Leu-Xaa33-Xaa34-Xaa35-Xaa36-Xaa37,
wherein Xaa7 is L-histidine; Xaa8 is Ala, Gly, Val, Leu, Ile or Lys; Xaa16 is Val or Leu; Xaa18 is Ser, Lys or Arg; Xaa19 is Tyr or Gln; Xaa20 is Leu or Met; Xaa22 is Gly or Glu; Xaa23 is Gln, Glu, Lys or Arg; Xaa25 is Ala or Val; Xaa26 is Lys, Glu or Arg; Xaa27 is Glu or Leu; Xaa30 is Ala, Glu or Arg; Xaa33 is Val or Lys; Xaa34 is Lys, Glu, Asn or Arg; Xaa35 is Gly; Xaa36 is Arg, Gly or Lys or amide or absent; Xaa37 is Gly, Ala, Glu, Pro, Lys, amide or is absent; or
d) a peptide comprising a sequence according to formula III:
Xaa7-XaaB-Glu-Gly-Thr-Phe-Thr-Ser-Asp-Val-Ser-Xaa18-Tyr-Leu-Glu-Xaa22-Xaa23-Ala-Ala-Xaa26-Glu-Phe-Ile-Xaa3O-Trp-Leu-Val-Xaa34-Xaa35-Xaa36-Xaa37,
wherein Xaa7 is L-histidine; Xaa8 is Ala, Gly, Val, Leu, Ile or Lys; Xaa18 is Ser, Lys or Arg; Xaa22 is Gly or Glu; Xaa23 is Gln, Glu, Lys or Arg; Xaa26 is Lys, Glu or Arg; Xaa30 is Ala, Glu or Arg; Xaa34 is Lys, Glu or Arg; Xaa35 is Gly; Xaa36 is Arg or Lys, amide or is absent; Xaa37 is Gly, Ala, Glu or Lys, amide or is absent.
e) or a sequence having at least 80 % sequence identity with a sequence of any of sequence according to a) to d), or
wherein component (III) contains or comprises the sequence of SEQ ID NOs: 4 or 5 or a sequence having at least 80% sequence identity with SEQ ID NOs: 4 or 5.

17. Use according to any of claims 2 to 16, wherein the GLP-1 fusion peptide additionally contains or comprises a carrier protein, in particular transferrin or albumin, as component (IV).

18. Use according to any of claims 2 to 17, wherein the GLP-1 fusion peptide comprises or consists of a peptide sequence selected from the sequence of: SEQ ID NO: 6, SEQ ID NO: 7, SEQ ID NO: 8, SEQ ID NO: 10, SEQ ID NO: 11, SEQ ID NO: 12, SEQ ID NO: 26, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, or SEQ ID NO: 48, or a sequence having at least 80% sequence identity with SEQ ID NOs: 6, 7, 8, 10, 11, 12, 26, or 37 to 48.

19. Use according to any of claims 1 to 18, wherein the cells in the core of the spherical microcapsules are engineered to additionally secrete a factor selected from the group consisting of anti-apoptotic factors, growth factors, VEGF, erythropoietin (EPO), anti-platelet drugs, anti-coagulant drugs, and anti-thrombotic drugs, and/or secrete endogenous proteins or peptides as paracrine factors that are released through the capsule in therapeutic levels selected from VEGF, IL6, IL8, GDNF, NT3, and MCP1.

20. Use according to any of claims 1 to 19, wherein the spherical microcapsules are implanted or injected into an administration site selected from the eye of a patient to be treated, including a tissue or an area of the eye, including the affected area or tissue of the eye of a patient to be treated including the retina, the iris, an area or a region 2-3 mm apart the iris, including an area or a region 2-3 mm apart the iris after the conjunctiva and sclera has been slightly disarranged, the vitreous cavity of the eye, any surface of the eye or the retina, the iris or its tissues or surrounding areas, the anterior chamber, the lens capsular bag, the subretinal space, the suprachoroidal space, the interior of eye, or any cavity of the eye.
